# EUROPEAN PATENT SPECIFICATION

(11) **EP 2 077 855 B1**
(45) Date of publication and mention of the grant of the patent: **01.02.2012**
(21) Application number: 07822132.2
(22) Date of filing: 31.10.2007
(51) Int. Cl.: A61K 38/46, A61P 3/00, A61P 35/00

(54) **THERAPEUTIC USE OF GBA2**
THERAPEUTISCHE VERWENDUNG VON GBA2
UTILISATION THÉRAPEUTIQUE DE GBA2

(30) Priority: 31.10.2006 US 855524 P
(43) Date of publication of application: 15.07.2009
(73) Proprietor: Yildiz, Dr. med., Yildiz, 53105 Bonn (DE); The University of Texas Sothwestern Medical Center, Dallas, TX 75390-9094 (US)
(72) Inventor: MATERN, Heidrun, 52066 Aachen (DE); MATERN, Siegfried, 52066 Aachen (DE); RUSSEL, David W., Dallas, TX 75229-6114 (US); YILDIZ, Yildiz, 53347 Impekoven (DE)
(74) Representative: von Kreisler Selting Werner
(86) International application number: PCT/EP2007/061786
(87) International publication number: WO 2008/053025

(56) References cited:
- US-B2- 7 771 726
- SUGANUMA ET AL: "Alkylated imino sugars, reversible male infertility-inducing agents, do not affect the genetic integrity of male mouse germ cells during short-term treatment despite induction of sperm deformities" BIOLOGY OF REPRODUCTION, vol. 72, 2005, pages 805-813, XP002468046 cited in the application
- OVERKLEEFT ET AL: "Generation of specific deoxynojirimycin-type inhibitors of the non-lysosomal glucosylceramidase" THE JOURNAL OF BIOLOGICAL CHEMISTRY, vol. 273, 1998, pages 26522-26527, XP000934220 cited in the application
- YILDIZ ET AL: "Mutation of beta-glucosidase 2 causes glycolipid storage disease and impaired male fertility" THE JOURNAL OF CLINICAL INVESTIGATION, vol. 116, November 2006 (2006-11), pages 2985-2994, XP002467760
- ROY ET AL: "Shaping the sperm head: an ER enzyme leaves its mark" THE JOURNAL OF CLINICAL INVESTIGATION, vol. 116, November 2006 (2006-11), pages 2860-2863, XP002467988
- BOOT ET AL: "Identification of the non-lysosomal glucosylceramidase as beta-glucosidase 2" THE JOURNAL OF BIOLOGICAL CHEMISTRY, vol. 282, 12 January 2007 (2007-01-12), pages 1305-1312, XP002445181
- WALDEN ET AL: "Accumulation of glycosylceramide in murine testis, caused by inhibition of beta-glucosidase 2: Implications for spermatogenesis" JOURNAL OF BIOLOGICAL CHEMISTRY, 11 September 2007 (2007-09-11), page 1, XP002467759 Retrieved from the Internet: URL:http://www.jbc.org/cgi/content/short/M 702387200v1> [retrieved on 2008-02-06]
- WALDEN ET AL: "Accumulation of glucosylceramide in murine testis, caused by inhibition of beta-glucosidase 2" THE JOURNAL OF BIOLOGICAL CHEMISTRY, vol. 282, 9 November 2007 (2007-11-09), pages 32655-32664, XP002467761
- MAEDA ET AL: "Anti-tumor effect of orally administered spinach glycolipid fraction on implanted cancer cells, colon-26, in mice", LIPIDS, vol. 43, 2008, pages 741-748,
- 'Synthesis of immunopotent alpha glycolipids via glycosyl iodides' UC DAVIS INNOVATION ACCESS, [Online] page 1 Retrieved from the Internet: <URL:www.research.ucdavis.edu/NCD.cfm?ncdid =751>
- 'Gene: GBA2' G2SBC GENES-TO-SYSTEMS BREAST CANCER DATABASE, [Online] page 1 Retrieved from the Internet: <URL:www.itb.cnr.it/breastcancer/php/geneRe port.php?id=57704>
- HAKOMORI: "Aberrant glycosylation in cancer cell membranes as focused on glycolipids: overview and perspectives", CANCER RESEARCH, vol. 45, 1985, pages 2405-2414,

## Description

The present invention provides a medicament or pharmaceutical composition comprising bile acid β-glucosidase 2 (GBA2), said medicament or pharmaceutical composition being, among others, for use in the treatment or prevention of a glycolipid storage disease or of male infertility. The invention furthermore provides specific GBA2 inhibitors for contraception in male subjects or for tumor therapy. Finally, a method for screening for compounds and compositions which may be suitable in treatment or prophylaxis of a glycolipid storage disease or male infertility is provided.

### Background of the Invention

Glycolipids are an abundant and diverse class of lipids in mammalian cells that play important functional roles in membrane structure and signaling (Simons, K. and Toomre, D., Nat. Rev. Mol. Cell. Biol. 1:31-39 (2000); Anderson, R.G. and Jacobson, K., Science 296:1821-1825 (2002)). A particularly complex class of glycolipids are glycosphingolipids, which are composed of a sphingosine backbone linked to a fatty acid and one or more hexose sugars (Menaldino, D.S. et al., Pharmacol. Res. 47:373-381 (2003)). Recent chemical analyses indicate that a typical mammalian cell may contain as many as ten thousand different glycosphingolipids (www.sphingomap.org). This structural complexity is generated by an equally elaborate network of biosynthetic and degradative enzymes (Merrill, J.A.H., J. Biol. Chem. 277:825,843-825,846 (2002)), many of which remain to be biochemically identified.

Glycosphingolipids are synthesized in the endoplasmic reticulum (ER) and Golgi compartments of the cell and broken down in lysosomes (Perry, R.J. and Ridgway, N.D., Biochim. Biophys. Acta 1734:220-234 (2005)). The functional consequences of this subcellular organization are evident in human genetic diseases in which glycosphingolipid degradation is affected. Loss of any of a number of catabolic enzymes causes accumulation of glycosphingolipids in the lysosome and a variety of symptoms that worsen with age (Futerman, A.H. and van Meer, G., Nat. Rev. Mol. Cell Biol. 5:554-565 (2004)).

Several lipid storage diseases are caused by a diminished capacity to degrade glycosphingolipids, inter alia Tay-Sachs-disease, Niemann-Pick-disease, Fabry's disease and Sandhoff disease. The most common lipid storage disease and most common inherited defect in glycosphingolipid breakdown is Gaucher's disease, an autosomal recessive disorder arising from glucocerebrosidase deficiency. Said deficiency is usually caused by mutations in the gene encoding the lysosomal acid β-glucosidase GBA1 (Beutler, E. and Grabowski, G.A., Gaucher Disease. In The Metabolic & Molecular Bases of Inherited Disease. C.R. Scriver et al., editors. New York: McGraw-Hill. 3,635-3,668 (2001)), a glucocerebrosidase. GBA1 (glucosidase, beta, acid 1) is a membrane-associated enzyme that cleaves the glucosesphingosine linkage present in glucosylceramides and other glycosphingolipids. The enzyme is abundant in macrophages, which normally degrade large amounts of cellular membrane lipids acquired by phagocytosis. When GBA1 is absent or impaired, glycosphingolipids accumulate within the macrophage lysosome, and the engorged cells in turn are deposited in the liver, spleen, and lung causing organ enlargement and progressive dysfunction.

There are three types of Gaucher's disease. In all three types, the enzyme deficiency results in a buildup of the glycolipid glucocerebroside in the bone marrow, liver, and spleen, resulting in anemia and other blood disorders, bone pain and pathologic fractures, and enlarged liver and spleen. In Type II, the central nervous system is also affected. Patients are severely mentally retarded and have difficulty controlling their muscles. The disease progresses quickly from birth and usually is fatal by the age of two. In Type III disease, the course is chronic and central nervous system involvement begins later on. The symptoms are the same as those of Type II. Type I disease occurs most often in Ashkenazi Jews of Eastern European origin, Type III in people of Swedish origin. Type I is also known as non-neuronopathic form, Types II and III as neuronopathic forms of Gaucher's disease.

In all, there are about 80 known mutations of GBA1 which have been connected to Gaucher's disease. The most prominent ones are:
Type I (N370S homozygote, the most common, also called the "non-neuropathic" type): occurs mainly (100x the general populace) in Ashkenazi Jews. It is mainly diagnosed in late childhood or early adulthood. Life expectancy is mildly decreased. There are no neurological symptoms;
Type II (1 or 2 alleles L444P): is characterized by neurological problems in small children. The enzyme is hardly released into the lysosomes. Prognosis is dismal: most die before reaching the third birthday;
Type III (also 1-2 copies of L444P, possibly delayed by protective polymorphisms): occurs in Swedish patients from the Norrbotten region. This group develops the disease somewhat later, but most die before their 30th birthday.

All three types of Gaucher's disease are inherited in an autosomal recessive fashion.

Current treatments for Gaucher's disease include enzyme replacement therapy with recombinant GBA1 (Cerezyme^{®}) and substrate-reduction therapy with N-butyldeoxynojirimycin (Zavesca^{®}). The most common treatment involves enzyme replacement therapy with recombinant GBA1 (Cerezyme^{®}), which reduces the accumulation of glucosylceramides in most peripheral tissues (Beutler, E., Baillieres Clin. Haematol. 10:751-763 (1997)). For type I and most type III patients, enzyme replacement treatment with mannose-terminated recombinant glucocerebrosidase, 60 Units/kg, given intravenously every two weeks can dramatically decrease liver and spleen size, reduce skeletal abnormalities, and reverse other manifestations. There is currently no effective treatment for the severe brain damage that may occur in patients with types II and III. Thus, intravenous enzyme replacement with GBA1 can decrease the severity of or reverse many of the complications of type I Gauchers disease, including liver/spleen enlargement, anemia, neurologic problems and bone abnormalities. Severe brain damage cannot be reversed, however.

A newer treatment, termed substrate-reduction therapy, involves administration of N-butyldeoxynojirimycin (Zavesca^{®}), which decreases the biosynthesis of glucosylceramides and thereby reduces accumulation (Futerman, A.H. et al., Trends Pharmacol. Sci. 25:147-151 (2004)). *N*-butyldeoxynojirimycin is thought to act by inhibiting glucosylceramide synthetase, the first enzyme in the glycosphingolipid biosynthetic pathway. Because *N*-butyldeoxynojirimycin causes globozoospermia when administered to rodents (Hamra, F.K. et al., Proc. Natl. Acad. Sci. U. S. A. 99:14931-14936 (2002); van der Spoel, A.C. et al., Proc. Natl. Acad. Sci. U. S. A. 99:17,173-17,178 (2002)), male Gaucher's patients who receive the drug are advised to take appropriate birth control measures. Male Gaucher patients not receiving the drug are fertile.

A drawback of GBA1 enzyme replacement therapy is that the recombinant production of functional GBA1 is restricted to the use of mammalian cells, especially CHO cells. This is due to the fact that only the production in mammalian cells like CHO cells leads to correct (mammalian) glycosylation of the recombinant GBA1. Insect cells do overglycosylate the GBA1 translation product, which leads to inactive GBA1. Bacterial cells like *E. coli* do not glycosylate the GBA1 translation product, which also leads to inactive GBA1. As a result of the restriction to mammalian cells and especially to CHO, production of recombinant GBA1 is costly and laborious. The involvement of glycolipids in mammalian spermatogenesis has been discussed in (Hamra, F.K. et al., Proc. Natl. Acad. Sci. U. S. A. 99:14931-14936 (2002)) and (van der Spoel, A.C. et al., Proc. Natl. Acad. Sci. U. S. A. 99:17,173-17,178 (2002)). The capacity of iminosugars like N-butyldeoxynojirimycin (NB-DNJ) and N-butyldeoxygalactonojirimycin (NB-DGJ) to impair spermatogenesis was associated with their potential to attenuate the biosynthesis of glucosylceramide-based sphingolipids by inhibition of ceramide-specific glucosyltransferase or an unspecified nonlysosomal glucosylceramidase activity. The latter activity is mentioned in (van der Spoel, A.C. et al., Proc. Natl. Acad. Sci. U. S. A. 99:17,173-17,178 (2002)), but not annotated to a specific protein, i.e. the source of said activity remained unknown.

NB-DGJ is not a GBA1 inhibitor, but a transferase inhibitor. As outlined above, NB-DNJ (Zavesca^{®}) is an iminosugar used in substrate reduction therapy of Gaucher's disease. It is only a weak GBA1 inhibitor *in vitro* and does not show inhibitory activity *in vivo* (US 2005/0032841).

GBA1 inhibitors are not used as male contraceptives. It is to be noted that the use of GBA1 inhibitors for male contraception would be hampered by the fact that this treatment would lead to Gaucher symptoms in the treated subjects. Thus, the provision of a specific inhibitor for glucosylceramide-based sphingolipid biosynthesis which attenuates neither GBA1 activity nor ceramide-specific glucosyltransferase activity is desirable as active agent for male contraception.

GBA1 is classified as an *O*-glycosyl hydrolase, a widely distributed group of enzymes that cleave *O*-glycosyl linkages between carbohydrates or between carbohydrates and lipids (Henrissat, B. and Bairoch, A., Biochem. J. 316:695-696 (1996)). GBA1 is distinguished from other *O*-glycosyl hydrolases by an acidic pH optimum and a preference for glycolipids. A second hydrolase with these general properties is GBA2, a membrane-bound enzyme of the ER that is active in both the cleavage and formation of bile acid glucosides and is inhibited by alkyldeoxynojirimycin derivatives (Matern, H. et al., FEBS Lett. 314:183-186 (1992); Gartung, C. et al., J. Hepatol. 20:32-40 (1994); Matern, H. et al., J. Biol. Chem. 272:11,261-11,267 (1997); Matern, H. et al., J. Biol. Chem. 276:37929-37933 (2001). Bile acid glucosides, in which glucose is attached in β-linkage to carbon 3 of the bile acid (Marschall, H.U. et al., J. Lipid Res. 35:1,599-1,610 (1994)), are normally excreted in the urine (Marschall, H.U. et al., FEBS Lett. 213:411-414 (1987)). Their synthesis by the liver is thought to assist in the disposal of hepatotoxic bile acids (Matern, H. et al., Proc. Natl. Acad. Sci. U. S. A. 81:7036-7040 (1984); Wietholtz, H. et al., Hepatology 13:656-662 (1991)). Although GBA1 and GBA2 are both glycolipid hydrolases, the two enzymes apparently do not share sequence identity, and are expressed in different tissues and subcellular compartments. Up to now, no activity of GBA2 on glucocerebrosides has been demonstrated. On the contrary, GBA2 did not show any glucocerebrosidase activity when tested with glucocerebrosides (Matern, H. et al., J. Biol. Chem. 272:11,261-11,267 (1997); Matern, H. et al., Proc. Natl. Acad. Sci. U. S. A. 81:7036-7040 (1984)).

Thus, the problem underlying present invention is the provision of medical application fields for GBA2, especially fields wherein GBA2 can replace GBA1.

### Summary of Invention

To gain insight into the metabolism of bile acid glucosides, a line of knockout mice with a disruption in *Gba2* was produced. Unexpectedly, bile acid metabolism was normal in the mutant mice but glucosylceramides were found to accumulate in multiple tissues. Accumulation in the testis led to decreased fertility and the formation of abnormal sperm. These data reveal GBA2 to be a second glucosylceramidase that is located in the ER rather than the lysosome and that plays an important role in preventing glycolipid accumulation.

The present invention provides
(1) a medicament or pharmaceutical composition comprising bile acid β-glucosidase 2 (GBA2);
(2) the medicament or pharmaceutical composition of embodiment (1) for use in the treatment or prevention of a glycolipid storage disease;
(3) the medicament or pharmaceutical composition of embodiment (1) for use in the treatment of male infertility, or for use in the prophylaxis or treatment of male infertility caused by therapeutic treatment with GBA1 inhibitors;
(4) the use of GBA2 inhibitors selected from carboxynonyl-deoxynojirimycin and dodecyldeoxynojirimycin for manufacturing a medicament for the treatment or prophylaxis of a glycolipid storage disease or male infertility;
(5) a method for screening for compounds and compositions which may be suitable in treatment or prophylaxis of a glycolipid storage disease or male infertility, comprising the administration of a test compound or test composition to the non-human transgenic animal with a (+/-) or (-/-) GBA2 genotype or a transgenic cell comprising a knockout construct for the knockout of GAB2 translation, preferably to the non-human transgenic animal with the GBA2 (-/-) genotype;
(6) the use of a GBA2 inhibitor for manufacturing a medicament for contraception in male subjects or for tumor therapy, wherein the GBA2 inhibitor is selected from carboxynonyl-deoxynojirimycin and dodecyldeoxynojirimycin;
(7) a GBA2 inhibitor or a pharmaceutical composition comprising a GBA2 inhibitor for use in contraception in male subjects or for use in tumor therapy, wherein the GBA2 inhibitor is selected from carboxynonyl-deoxynojirimycin and dodecyldeoxynojirimycin.

### Short Description of the Figures

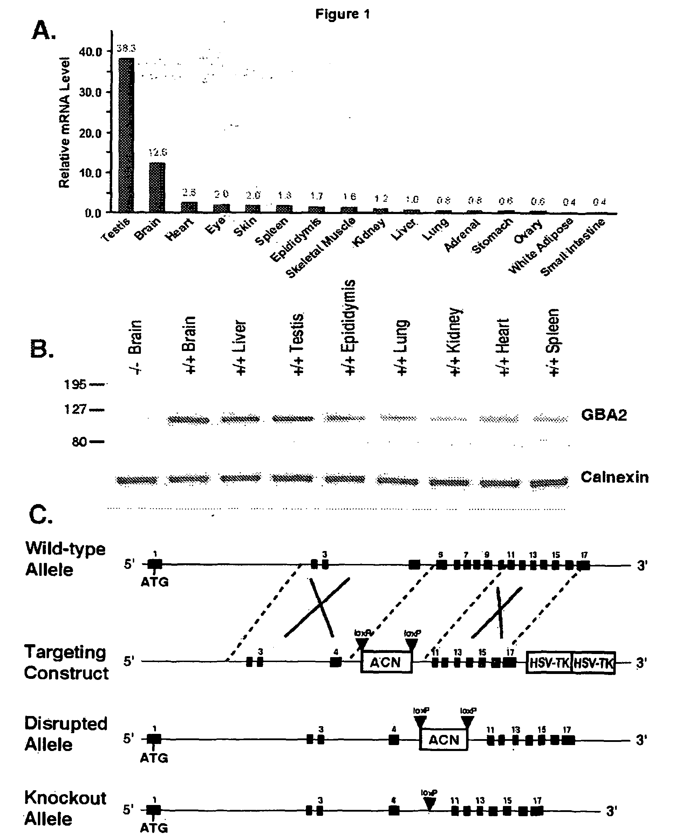

Figure 1: Tissue distribution of GBA2 mRNA, protein, and strategy to knock out *Gba2*.
   (A) The relative levels of GBA2 mRNA in the indicated pooled tissues of male mice (n = 6) were determined by real time RT-PCR. The data were normalized to the threshold value determined in the liver (CT = 26 to 28). (B) Microsomal membranes were prepared and pooled from the indicated tissues isolated from male mice (n = 6). Aliquots of protein (30 µg) were resolved by SDS-PAGE, transferred to nitrocellulose, and then blotted with an antibody raised against amino acids 1 to 508 of the GBA2 protein (upper panel). To ensure equal amounts of protein were examined in each lane, bound GBA2 antibody complexes were removed and the filter was probed a second time with an antiserum raised against calnexin (lower panel). (C) Schematics of the *Gba2* wild-type allele, the targeting construct used to induce a deletion mutation by homologous recombination in ES cells, the predicted disrupted allele, and the final knockout allele are shown. Exons are numbered black' boxes and ATG represents the initiation codon specified within exon 1. Black triangles indicate positions of LoxP sites recognized and cleaved by Cre recombinase.

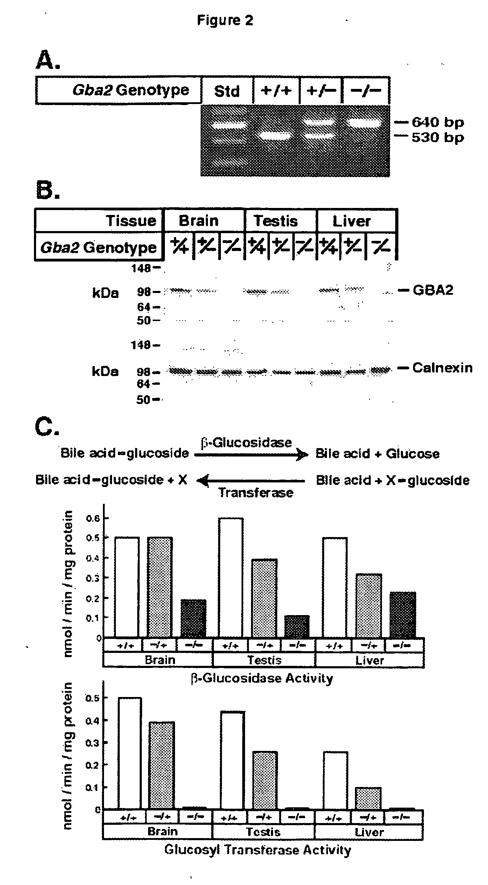

Figure 2: Characterization of *Gba2*-/- mice. (A) PCR genotyping of *Gba2* wild-type (+/+), heterozygous (+/-), and homozygous (-/-) genomic DNA. A 640 bp product is generated from the mutant allele while a 530 bp product is produced from the wild-type allele. (B) Immunoblotting of GBA2 protein levels in pooled brain, testis, and liver from *Gba2* wild-type, heterozygous, and homozygous mice (n = 6 per genotype). The filter of the upper panel was stripped of antibody-antigen complexes and re-probed with an antiserum recognizing calnexin to serve as a loading control. (C) Bile acid β-glucosidase and transferase enzyme activities were determined in the brain, testis, and liver of *Gba2* wild-type, heterozygous, and homozygous mice. "X" in the reaction shown is either dolichyl phosphoglucose or octyl β-glucoside.

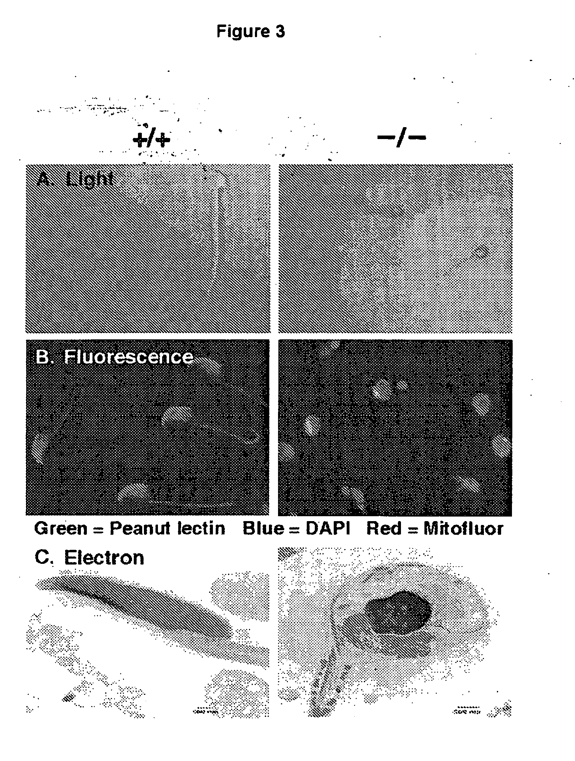

Figure 3: Sperm morphology in *Gba2* wild-type (+/+) and knockout (-/-) mice. (A) Photomicrographs taken on light microscope showing normal sickle-headed sperm in wild-type mice and round-headed sperm in knockout mice. (B) Fluorescent staining for cell nuclei with DAPI (blue), immunofluorescent staining of acrosomes with peanut lectin (green), and fluorescent staining of mitochondria (red). (C) Electron microscopic analyses of sperm showing abnormal head shape, nuclear organization, and distribution of mitochondria in knockout sperm.

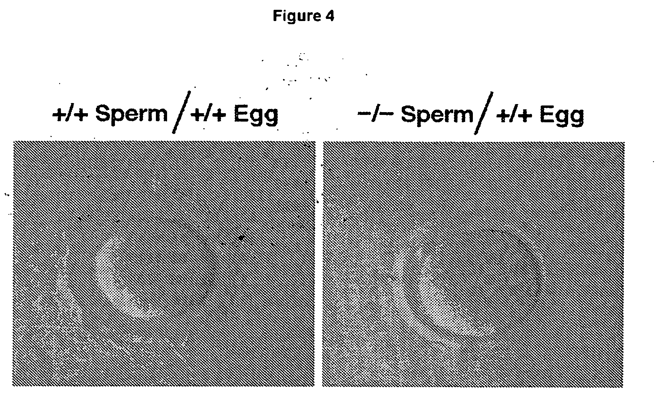

Figure 4: Failure of sperm from *Gba2*-/- mice to bind the zona pellucida. Cumulus enclosed oocytes from *Gba2*+/+ mice were incubated for 5 h with either *Gba2*+/+ sperm (left panel) or *Gba2*-/- sperm (right panel) and then examined by light microscopy. Sperm from knockout mice were deficient in zona pellucida binding.

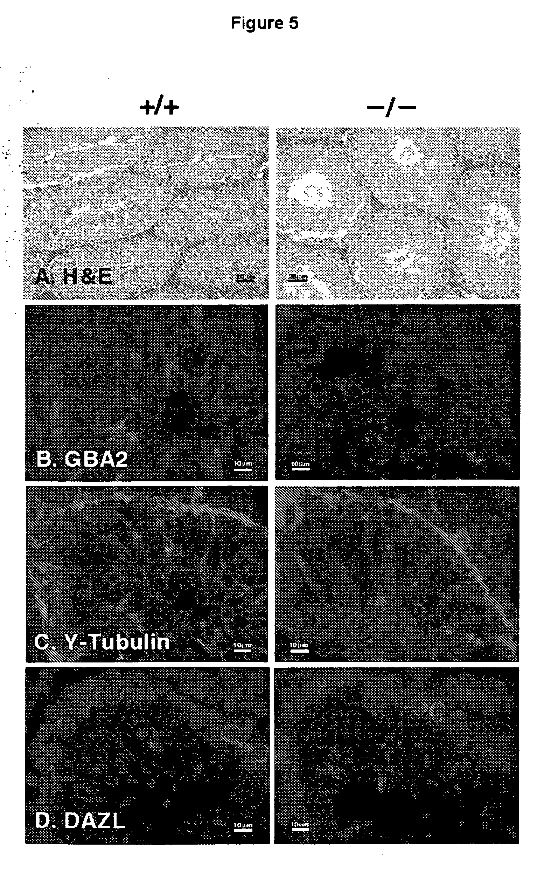

Figure 5: Cell type specific expression patterns of GBA2, tyrosine-tubulin, and DAZL (Deleted in Azoospermia-Like) in testis from *Gba2* wild-type (+/+) and knockout (-/-) mice. (A) H & E (hematoxylin-eosin) staining of testis showing near normal cellular organization of the gonads. (B) Immunofluorescent staining of GBA2 in Sertoli cells of the organ. (C) Immunofluorescent staining with antibodies that recognize tyrosine-tubulin, a Sertoli cell marker protein. (D) Immunofluorescent staining with antibodies that recognize DAZL, a germ cell marker protein.

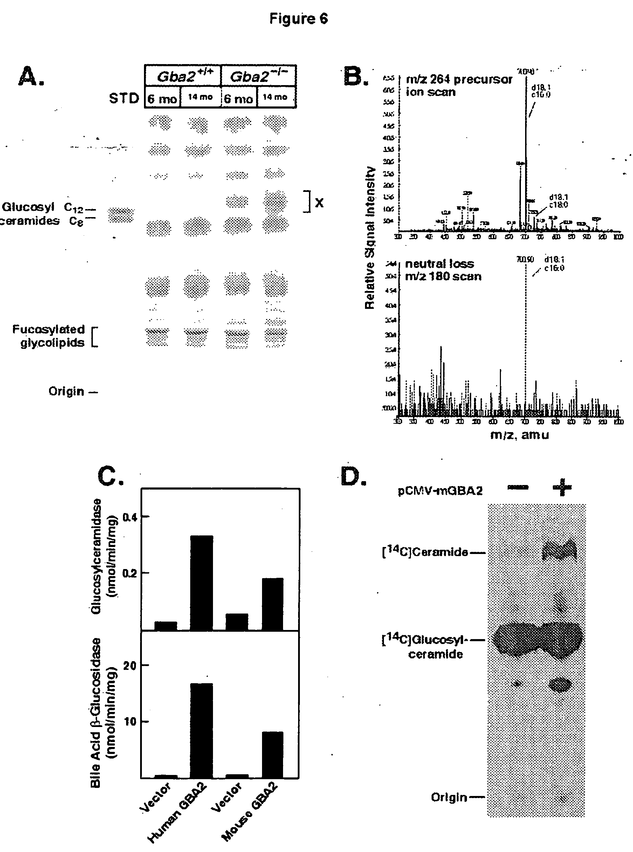

Figure 6: Analysis of glycolipids in *Gba2* wild-type (+/+) and knockout (-/-) mice. (A) Glycolipids were extracted from testes of 6- and 14-month old mice, resolved by onedimensional thin layer chromatography (TLC), and visualized by staining with orcinol. The positions of the origin and glucosylceramide standards are indicated on the left. A glycolipid that accumulates with age in knockout tissue is marked "X" on the right. The positions to which several fucosylated glycolipids migrated to are indicated on the left. (B) Structural analysis of glycolipid "X". Glycolipids were extracted from the testes of *Gba2*-/- mice, resolved by one-dimensional TLC, and glycolipid "X" was analyzed by mass spectrometry. An *m*/*z* 264 precursor ion scan (upper panel) revealed compounds of molecular masses (700.9 and 728.3) characteristic of monohexosylceramides containing sphingosine (d18:1) and either 16:0 or 18:0 fatty acyl groups. A neutral loss scan for *m*/*z* 180 diagnostic for glucose- or galactose-containing monohexosylceramides revealed a major compound of mass 700.9 (lower panel). (C) Expression of mouse and human GBA2 in COS cells and measurement of glucosylceramidase and bile acid β-glucosidase enzyme activities. Cells were transfected with the indicated plasmid DNA and lysates assayed for enzyme activity using fluorescently-labeled glucosylceramide (upper panel) or bile acid glucoside (lower panel) substrates. (D) Expression of mouse GBA2 in human embryonic kidney 293 cells and measurement of glucosylceramidase activity. Cells were transfected and assayed for enzyme activity using [¹⁴C]glucosylceramide. The positions of the origin and to which authentic glucosylceramide and ceramide standards migrated to are indicated on the left.

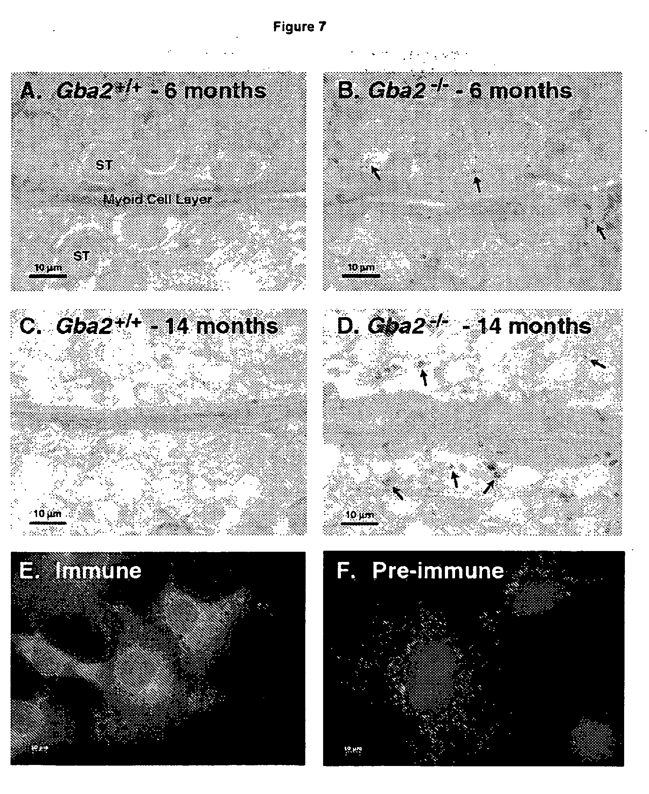

Figure 7: Glycolipid accumulation in *Gba2*-/- mice and subcellular localization of GBA2. Glycolipids were visualized in the testis of 6- and 14-month-old wild-type (A,C) and knockout (B,D) mice by periodic acid Schiff staining of 8 µm sections. Arrows mark small intensely stained deposits of glycolipids that accumulate with age in the knockout mice. ST, seminiferous tubule. (E) The subcellular localization of GBA2 (green signal) was determined by immunofluorescent staining of simian COS cells. Lysosomes (red signal) were identified by staining with LysoTracker^{®}. (F) COS cells stained with a preimmune serum and LysoTracker^{®}. Scale bars are indicated in individual panels.

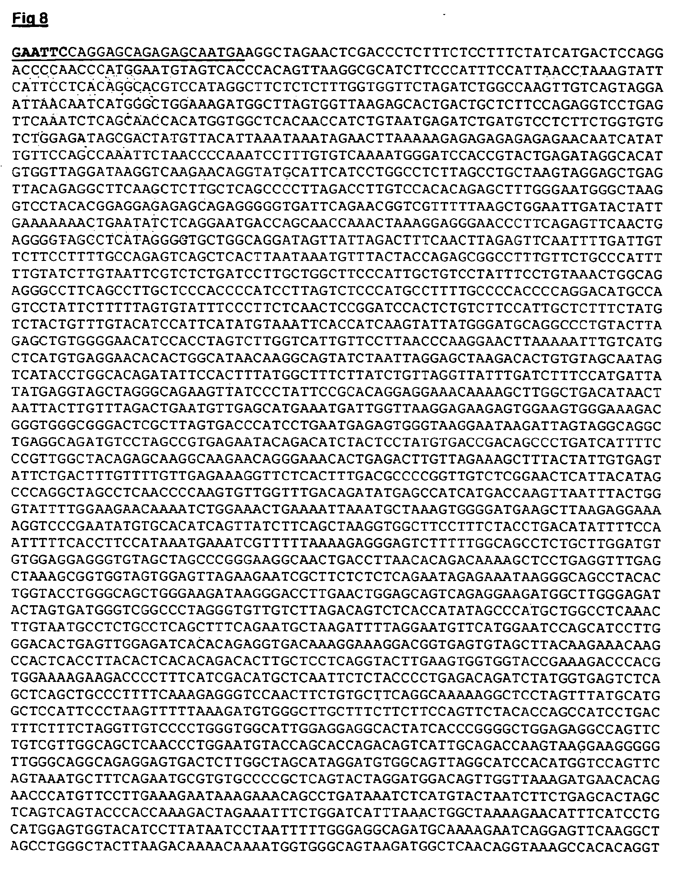

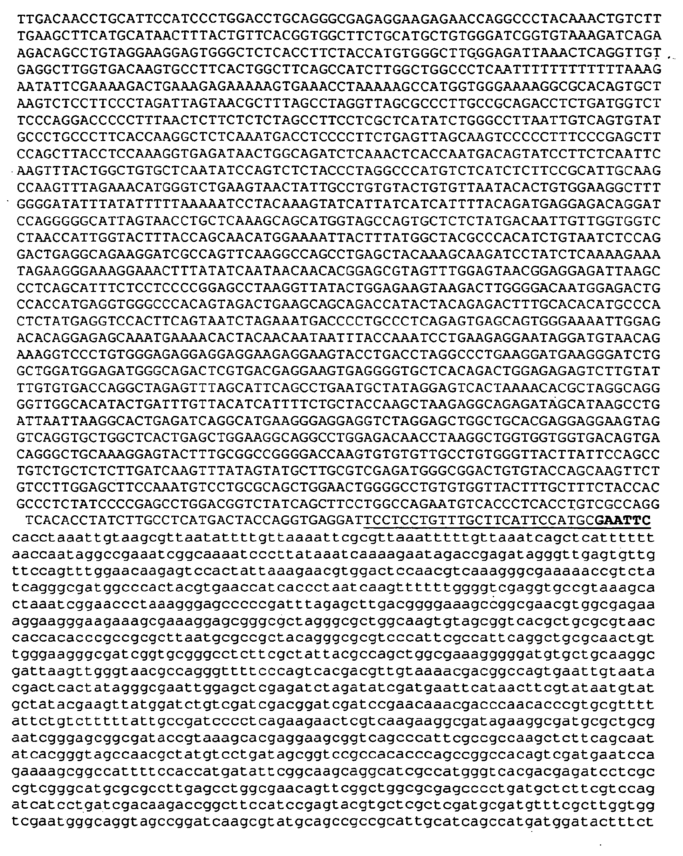

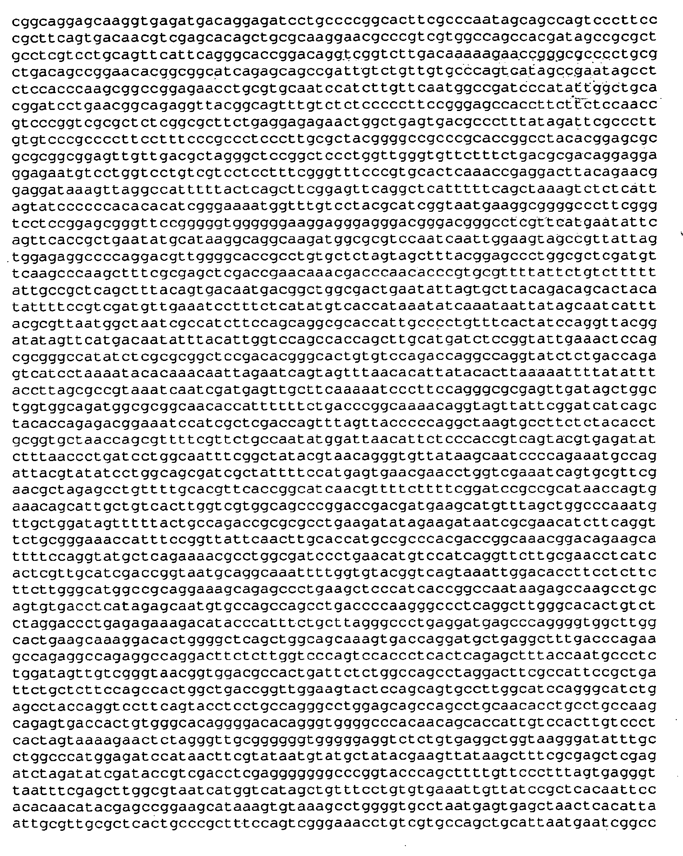

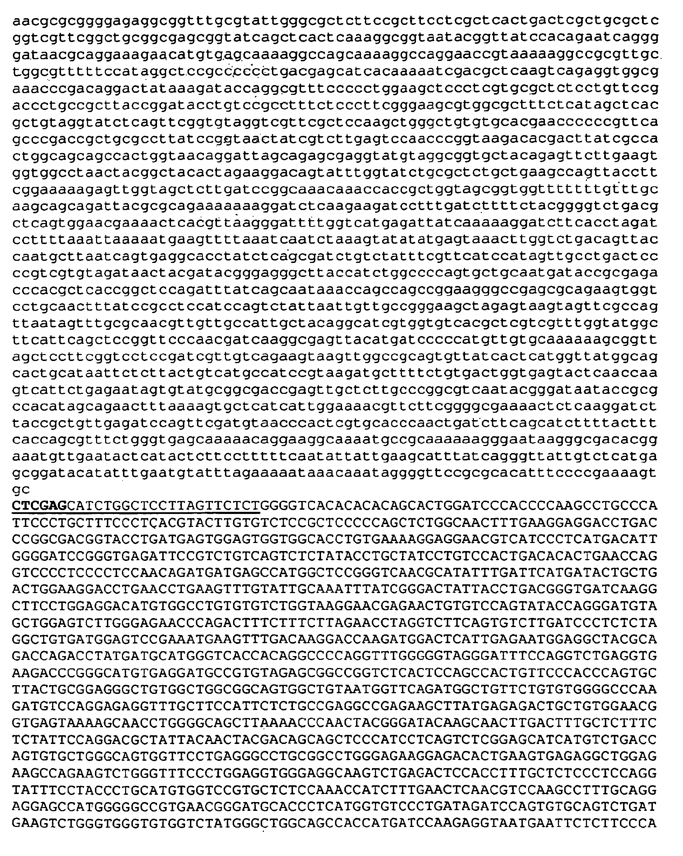

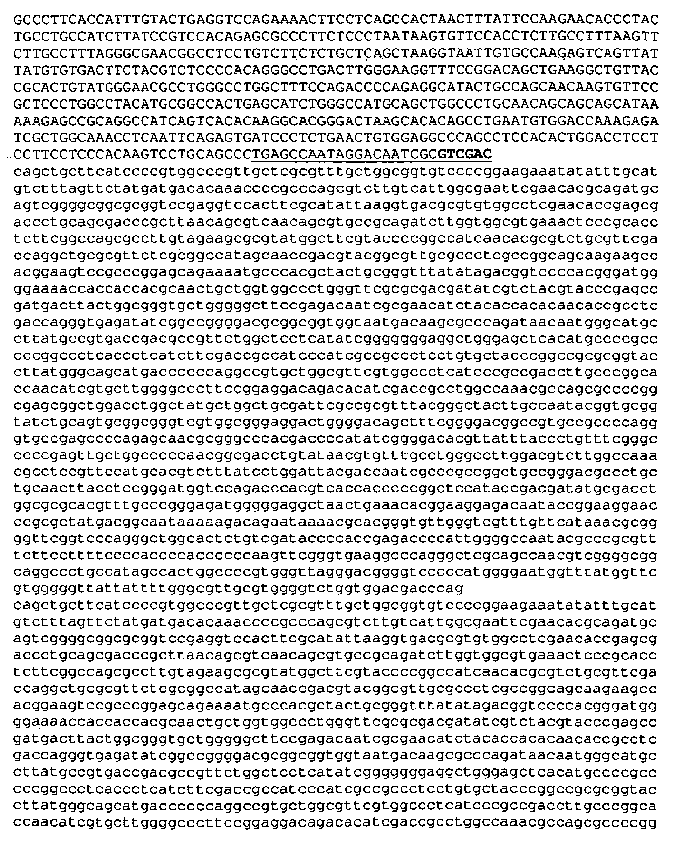

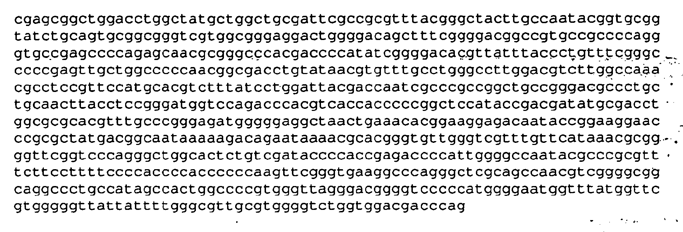

Figure 8: DNA sequence of vector construct for preparing the GBA2-deficient mouse (SEQ ID NO:18). The genomic sequences of the homologous arms are given in capital letters, the selection cassette is in lower case letters, the primer sequences utilized for obtaining the homologous arms are underlined and the restriction enzyme recognition sites are in bold letters.

### Sequence Listing, Free Text

| SEQ ID NO: | Description |
|---|---|
| 1 | human *Gba2* cDNA; NM_020944 |
| 2 | human GBA2 |
| 3 | mouse *Gba2* cDNA; NM_172692 |
| 4 | mouse GBA2 |
| 5 to 8 | Primers |
| 9 | *HSV-thymidin kinase* |
| 10 | plasmid containing ACN cassette; AF_169416 |
| 11 | pBluescript II KS(+) |
| 12 to 14 | genotyping primers |
| 15, 16 | RT-PCR detection primers |
| 17 | mouse *Gba2* gene; NC_000070, gi:94471531 |
| 18 | Gba2 knock-out vector |

### Detailed Description of Invention

The following abbreviations and terms are used:
GBA1, lysosomal acid β-glucosidase 1; GBA2, bile acid β-glucosidase 2; ER, endoplasmatic reticulum.

A glycosphingolipid is a glycoside of ceramide. In most cases, the glycoside part is glucose or galactose. Glycosphingolipids are the most abundant and structurally diverse type of glycolipids in animals (including humans).

A glucosylceramide is a glycolipid containing a fatty acid, glucose, and sphingosine. It is also called glucocerebroside.

A glucocerebrosidase is an enzyme which hydrolyzes glucocerebrosides into glucose and ceramide. Glucocerebrosidase deficiency causes Gaucher's disease.

The present disclosure provides several fields of application for GBA2 and GBA2 inhibitors, as well as methods for recombinant GBA2 production and GBA2 knockout mice.

The medicament of embodiment (1) comprises animal (mammalian) or human GBA2 or a mutein derivative thereof, preferably GBA2 from recombinant GBA2 production. Muteins of GBA2 include deletion muteins, addition muteins or substitution muteins having 1 to 10, preferably 1 to 5 single or consecutive amino acid residues deleted, added or substituted, respectively. Muteins also include fragments having up to 100, preferably up to 50 amino acid residues deleted at the C-terminal and/or at the N-terminal. Particularly preferred are the native GBA2 proteins or muteins having conservative substitutions only. Derivatives include chemical modifications of amino acid residues of the GBA (C-terminal PEGylation, amidation) and fusion proteins containing GBA2 as a first domain and a second domain containing a further functional protein or peptide moiety.

The medicament of embodiment (1) is suitable for treatment and prevention of glycolipid storage diseases, male infertility, tumors and as complementary therapy to chemotherapy in order to enhance receptor sensitivity. In addition, it is suitable for wound healing, tissue regeneration, and enhancement of blood formation. Furthermore, it is suitable for treatment of Alzheimer's disease and Parkinson's disease. The effect of the medicament comprising GBA2 is founded in the known properties of glycosphingolipids (reviewed in: Merrill, A. H. et al., toxicology and applied pharmacology 142:208-225 (1997); Zhang, X. and Kiechle, F.L., Ann. Clin. Lab. Sci. 34(1):3-13 (2004); Bektas, M. and Spiegel, S. Glycoconj. J. 20(1):39-47 (2004)) and in the experimental findings in Gba2 (-/-) mice discussed below.

In embodiment (2) - a preferred aspect of embodiment (1) - the medicament comprising GBA2 is suitable for the treatment or prevention of a glycolipid storage disease.

In a preferred aspect of embodiment (2), said glycolipid storage disease is caused by a defect in glycosphingolipid breakdown including Tay-Sachs-disease, Niemann-Pick-disease, Fabry's disease, Sandhoff disease and Gaucher's disease. Of these defects, Gaucher's disease is most preferred, especially Type II or III of Gauche's disease.

In a further preferred aspect of embodiment (2), the glycolipid storage disease is caused by a glucosylceramide storage defect and/or a defect in glucosylceramide breakdown. Gaucher's disease is one of the diseases falling under this definition. GBA2, preferably recombinant GBA2, is especially suitable in enzyme replacement therapy of Gaucher's disease. Enzyme replacement with GBA2 can decrease the severity of or reverse many of the complications of type I Gaucher's disease, including liver/spleen enlargement, anemia, neurologic problems and bone abnormalities. Even more important, it can decrease the severity of the neuronopathic Types II and III of Gaucher's disease. This is especially the case for Type III, as the glycolipid accumulation does not take place in the lysosomes in this Type (Kamoshita, S. et al., Adv. Exp. Med. Biol. 68:63 (1976)).

In embodiment (3) - a further preferred aspect of embodiment (1) - the medicament comprising GBA2 is suitable for the treatment of male infertility. It is moreover suitable for prophylaxis or treatment of male infertility caused by therapeutic treatment of subjects with GBA1 inhibitors (e.g. because they suffer from Gaucher's disease).

The medicament or pharmaceutical composition of any of embodiments (1) to (3) is in one preferred aspect of present invention suitable for enzyme replacement therapy. I.e., the active ingredient GBA2 of the medicament/pharmaceutical composition is able to replace an enzyme or enzymatic activity lacking in the subject in need of the therapy. It is most preferred that the medicament or pharmaceutical composition is suitable for replacement of GBA1. In this last aspect, GBA2 provides the enzymatic activity associated with GBA1.

In embodiments (1) to (3), the GBA2 is preferably recombinant GBA2, i.e. it is produced by transgenic cells. In contrast to Cerezyme^{®} (GBA1), recombinant GBA2 can be produced easily in its active form, especially by insect cells (compare Example 24), COS-7 or HEK293 cells (compare Fig. 6). Preferably, the recombinant GBA2 is produced by COS-7 or HEK293 cells.

In a further preferred aspect of embodiments (1) to (3), the GBA2 is human GBA2, preferably human GBA2 with SEQ ID NO:2 or a derivative thereof which differs from SEQ ID NO:2 only by conservative amino acid exchanges. Furthermore, the human GBA2 must be suitable for application to humans. This includes that the GBA2 is glycosylated in a manner which leads to an enzyme whose activity is similar to the activity of the wild type human GBA2. In a special aspect, this is achieved by recombinant expression of human GBA2 in insect cells, COS-7 cells or HEK-293 cells as outlined above. In a further special aspect, the glycosyl chains on GBA2 are shortened *in vitro* to terminal mannoses in order to ensure lysosomal targeting of the enzyme after its application to a subject. This shortening to terminal mannoses is performed as described for GBA1 (US 2006/0204487).

In one aspect of embodiment (4) of present invention, specific inhibitors of GBA2 are used for manufacturing a medicament for contraception in male subjects. The specific inhibitors are selected from the group consisting of carboxynonyldeoxynojirimycin and dodecyldeoxynojirimycin (compare Example 23).

It is to be noted that this use of GBA2 inhibitors as a male contraceptive is surprising because glucocerebrosidase activity of GBA2 had been denied in the literature (Matern, H. et al., J. Biol. Chem. 272:11,261-11,267 (1997)).

In contrast to GBA2 inhibitors, GBA1 inhibitors cannot be used as a male contraceptive. This is due to the fact that GBA1 is scarcely expressed in testis, whilst GBA2 expression in testis is high. Thus, male contraception is only achievable using GBA2 inhibitors. The use of GBA1 inhibitors for male contraception would moreover be hampered by the fact that this treatment would lead to Gaucher symptoms in the treated subjects. Thus, the provision of a specific inhibitor for glucosylceramide-based sphingolipid biosynthesis which is not attenuating GBA1 activity is desirable. In conclusion, GBA2 inhibitors which are specific for GBA2 and which are especially not GBA1 inhibitors are most preferred in the "male contraception" aspect of embodiment (4). This is the case for Carboxynonyldeoxynojirimycin and Dodecyldeoxynojirimycin, whose considerable inhibitory effect on β-glucosidase and transferase activity (especially on GBA2) is reported herein for the first time (see Example 22).

In a further aspect of embodiment (4), such specific inhibitors of GBA2 are useful for the treatment of cancer, preferably of hepatocellular carcinoma and leukemia. This finding is supported by the fact that GBA2 (-/-) mice show an increased apoptotic cell death and by the role of glycosphingolipids in cell death (Bektas, M. and Spiegel, S. Glycoconj. J. 20(1):39-47 (2004)). Furthermore, GBA2 expression *in vitro* is high during cell division. It is also high in tissue lesions, especially in liver tissue lesions. The specific inhibitors are selected from the group consisting of carboxynonyldeoxynojirimycin and dodecyldeoxynojirimycin (compare Example 22).

The medicament of embodiments (1), (2), (3) and (4) of the invention may be applied in any administration form suitable for enzyme administration to a subject in need thereof, but is preferably applied intravenously. In order to ensure lysosomal targeting of the GBA2 enzyme or its inhibitor, there are several application forms at hand for the skilled artisan, including liposomal formulation, furnishing the recombinant GBA2 with a lysosomal targeting sequence and further lysosmoal targeting methods known in the art (reviewed in: Torchilin, V.P., Annu. Rev. Biomed. Eng. 8:343-75 (2006)). For the enzyme, the application of GBA2 whose glycosyl chains have been shortened to terminal mannoses *in vitro* (US 2006/0204487) is an especially preferred option, as this modification ensures lysosomal targeting of the enzyme.

One focus of the present invention is the use of transgenic cells or non-human animals which express recombinant GBA2 for the search of novel inhibitors of glucocerebroside synthesis.

The cell used in the method of embodiment (5) is preferably an insect or mammalian cell. More preferably, it is a mammalian cell, even more preferably a human or mouse cell, most preferably a COS-7 or a HEK 293 cell (compare Fig. 6). The heterologous GBA2 which is expressed in the cell is preferably human or mouse GBA2, more preferably the human GBA2 of SEQ ID NO:2 or the mouse GBA2 of SEQ ID NO:4. In one specific aspect, the cell is a mouse cell and the heterologous GBA2 expression construct encodes SEQ ID NO:4 and preferably comprises SEQ ID NO: 3.

This method is a screening method for compounds and compositions which may be suitable as GBA2 inhibitors.

A further aspect of the method of embodiment (5) is the use of non-human animals which lack GBA2 (due to a recombinant knockout of their native *Gba2* gene).

The knockout vector or knockout construct for the knockout of the native *Gba2* of a host cell is disclosed in the following. This knock out may be performed by any known knockout method including RNAI and homologous recombination, but homologous recombination is preferred. When homologous recombination is used, the knockout construct preferably lacks one or more exons of the *Gba2* gene, preferably one, more or all of exons 5 to 10, It is furthermore preferred that the construct is flanked by recombination sites like loxP. It may additionally comprise at least one positive selection marker (e.g. an antibiotic resistance gene like the antibiotic resistance gene comprised in the ACN cassette of example 1) and/or one negative selection marker (e.g. HSV thymidin kinase). Furthermore, It may comprise a nucleic acid sequence which encodes Cre. The Cre enzyme may also be provided by the host cell itself or by a further exogenous construct, but the presence of a Cre encoding nucleic acid sequence inside the construct on the vector is preferred.

This knockout vector may be comprised in the cell used in the method of embodiment (5).

The GBA2 (-/-) transgenic animal used in the method of embodiment (8) is suitable as animal model for storage diseases, tissue regeneration defects, tumors (especially hepatocellular tumors and leukemia), Alzheimer's disease and Parkinson's disease. It is preferably a mouse. It exhibits a Gaucher like phenotype, i.e. the accumulated glycolipids are stored in the same organs, but not necessarily in the same cellular compartments as in Gaucher patients lacking GBA1, and malformed spermatozoa. It may be used in screening assays for the search of agents which can supplement the missing GBA2 activity in GBA2 deficient animals and man.

In contrast to the GBA2 (-/-) mouse, GBA1 (-/-) mice are not suitable for such screening. This is due to the fact that they are not available: they die immediately after their birth (Xu, Y.-H. et al., Am. J. Pathol. 163:2,093-092,101 (2003)). Mouse GBA2 of SEQ ID NO:4, which is known in the art, and a nucleic acid sequence encoding mouse GBA2 are used for performing some aspects of present invention. The nucleic acid sequence used is preferably the cDNA sequence represented by SEQ ID NO:3, a corresponding mRNA sequence or a genetic sequence encoding said corresponding mRNA sequence like SEQ ID NO:17. In a less preferred aspect, nucleic acid sequences which are derivable from SEQ ID NO:3 by conservative nucleotide exchange are also considered. Conceptual translation of the cDNA sequence of mouse GBA2 (SEQ ID NO:3) predicted a protein of 918 amino acids with 83% sequence Identity to the previously characterized human enzyme (Matern, H. et al., J. Biol. Chem. 276:37929-37933 (2001)). Database searches with the mouse and human cDNA sequences revealed apparent orthologs of the GBA2 enzyme in species ranging from *Drosophila* (CG33090 gene) to *Arabidopsis* (MUJ8.8 gene) to vertebrates, suggesting that the protein was conserved and thus of functional importance. No overt sequence identity was detected between GBA2 and other proteins in the database.

A cell comprising a vector comprising a nucleic acid sequence encoding mouse GBA2, especially a sequence as characterized in the preceding section, is used in some aspects of the invention as well, especially in the test method of embodiment (10). Said cell is preferably a mammalian cell, more preferably a mouse cell.

The invention is explained in more detail in the following sections, which additionally refer to some specifically preferred aspects of the invention, but are not to be construed as limiting the invention:
Bile acid β-glucosidase (GBA2) is a resident enzyme of the endoplasmic reticulum thought to play a role in the metabolism of bile acid-glucose conjugates. To gain insight into the biological function of this enzyme and its substrates, mice deficient in GBA2 were generated. Surprisingly, these animals had normal bile acid metabolism. Knockout males exhibited impaired fertility. Microscopic examination of sperm revealed large round heads (globozoospermia), abnormal acrosomes, and defective mobility. Glycolipids, identified as glucosylceramides by mass spectrometry, accumulated in the testis, brain, and liver of the knockout mice but did not cause obvious neurological symptoms, organomegaly, or a reduction in lifespan. Recombinant GBA2 hydrolyzed glucosylceramide to glucose and ceramide; the same reaction catalyzed by the acid-β- glucosidase (GBA1) defective in subjects with the Gaucher's form of lysosomal storage disease. Thus, GBA2 is a glucosylceramidase whose loss causes accumulation of glycolipids and an endoplasmic reticulum storage disease.

The present invention shows that the GBA2 enzyme, initially identified as a microsomal bile acid β-glucosidase *in vitro*, functions as a glucocerebrosidase *in vivo*.

The reassignment of the enzyme to glycolipid metabolism is based on two observations in *Gba2*-/- mice. First, enzyme loss does not disrupt cholesterol or bile acid metabolism, and second, tissues from the knockout mice, in which tissues GBA2 is normally expressed in the wild type mice, including the testes, liver, and brain, accumulate glucosylceramides. Consistent with these observations, recombinant GBA2 enzyme exhibits glucosylceramidase activity *in vitro*. The accumulation of glycolipids in the Sertoli cells of the testis gives rise to misshapen sperm and impaired fertility in the knockout mice. In contrast, while these same glycolipids build up in the liver and brain, they do not appear to grossly affect organ function. We conclude that GBA2 is a glucocerebrosidase whose loss leads to glycolipid accumulation and a form of ER storage disease.

The existence of a non-lysosomal glucocerebrosidase activity in various human tissues and cell types was reported in the 1990's by Aerts and colleagues (van Weely, S. et al., Biochim. Biophys. Acta 1181:55-62 (1993); Overkleeft, H.S. et al., J. Biol. Chem. 273:26,522-26,527 (1998)). This activity was shown to be tightly associated with the membrane, selectively inhibited by hydrophobic deoxynojirimycin derivatives and the conjugated bile acid taurocholate, and to be present at normal levels in cells isolated from Gaucher's patients. Although the enzyme responsible for this activity was never identified, the described biochemical properties are similar to those of GBA2 and it is therefore possible that the non-lysosomal glucocerebrosidase detected in these earlier studies was GBA2.

*In vitro*, GBA2 catalyzes the conjugation of glucose to lipid substrates- and the hydrolysis of glycolipid conjugates. The fact that glucosylceramides accumulate in *Gba2*-/- mice suggests that the preferred reaction direction *in vivo*, at least in the testes, liver, and brain, lies in favor of the β-glucosidase activity of the enzyme. The glucosyltransferase reaction may predominate in other tissues or the equilibrium may be regulated depending on the needs of the cell. Along these lines, glycolipids are typically synthesized in the ER and broken down in the lysosome (Perry, R.J. and Ridgway, N.D., Biochim. Biophys. Acta 1734:220-234 (2005)), and it is conceivable that the need to catabolize glucosylceramides in the ER arises under some conditions and is met by the reversible GBA2 enzyme. Similarly, the glucosyltransferase activity may predominate with bile acid substrates in the liver to facilitate their disposal.

Both the GBA2 bile acid β-glucosidase and the GBA1 lysosomal acid β-glucosidase deficient in Gaucher's disease hydrolyze glucosylceramides, and it is instructive to compare the two enzymes and the phenotypes arising from their loss.

GBA2 is a microsomal enzyme that catalyzes the transfer of glucose to multiple lipid substrates as well as the hydrolysis of these conjugates. Thus, GBA2 is suitable for the treatment of other lipid storage diseases besides Gaucher's disease, namely glycolipid storage diseases caused by a defect in glycosphingolipid breakdown including Tay-Sachs-disease, Niemann-Pick-disease, Fabry's disease and Sandhoff disease. In contrast thereto, GBA1 is a lysosomal enzyme that preferentially hydrolyzes glucosylceramides (Pentchev, P.G. et al., J. Biol. Chem. 248:5,256-5,261 (1973)). *Gba2*-/- mice and Gaucher's patients accumulate glucosylceramides in multiple tissues; however, the pattern of deposition and its consequences are different in each. In the knockout mice, glycolipid accumulates in the ER while in Gaucher's subjects, the lipid accumulates in the lysosome (Futerman, A.H. and van Meer, G., Nat. Rev. Mol. Cell Biol. 5:554-565 (2004); Beutler, E. and Grabowski, G.A., Gaucher Disease. In The Metabolic & Molecular Bases of Inherited Disease. C.R. Scriver et al., editors. New York: McGraw-Hill. 3,635-3,668 (2001)). This difference in subcellular accumulation leads to globozoospermia and decreased fertility in GBA2-deficient male mice, whereas human males with Gaucher's disease are fertile (Beutler, E. and Grabowski, G.A., Gaucher Disease. In The Metabolic & Molecular Bases of Inherited Disease. C.R. Scriver et al., editors. New York: McGraw-Hill. 3,635-3,668 (2001)). Although glycolipids build up in the tissues of the mutant mice, they do not cause organomegaly, whereas in Gaucher's subjects these lipids accumulate in resident and infiltrating phagocytes and cause organ enlargement (Beutler, E. and Grabowski, G.A., Gaucher Disease. In The Metabolic & Molecular Bases of Inherited Disease. C.R. Scriver et al., editors. New York: McGraw-Hill. 3,635-3,668 (2001)) GBA2 is no lysosomal protein. Thus, in order to act as a replacement enzyme for GBA1 in lysosomes, it must be targeted to lysosomes. In order to ensure lysosomal targeting of the GBA2 enzyme, there are several application forms at hand for the skilled artisan, including liposomal formulation, combinantion with lysosomal targeting sequences and the lysosomal targeting methods reviewed in Torchilin, V.P., Annu. Rev. Biomed. Eng. 8:343-375 (2006). The application of GBA2 whose glycosyl chains have been shortened to terminal mannoses *in vitro* (US 2006/0204487) is the most preferred option.

Knockout mice lacking GBA1 die shortly after birth (Xu, Y.-H. et al., Am. J. Pathol. 163:2,093-092,101 (2003)), whereas GBA2 deficient mice have normal life spans. The variation in phenotype arising from the loss of GBA1 versus GBA2 is most likely due to the unique tissue-specific expression patterns and subcellular localizations of these isozymes. These properties may also explain why the two enzymes do not appear to compensate for one another. This lack of compensation should nevertheless not influence an enzyme replacement therapy with recombinant GBA2 in Gaucher's patients, as the targeting of the extraneous protein is different from the targeting of GBA2 during GBA2 transcription *in vivo*.

Two treatments are currently used for the management of Gaucher's disease. The most common treatment involves enzyme replacement therapy with recombinant GBA1 (Cerezyme^{®}), which reduces the accumulation of glucosylceramides in most peripheral tissues (Beutler, E., Baillieres Clin. Haematol. 10:751-763 (1997)). A newer treatment, termed substrate-reduction therapy, involves administration of N-butyldeoxynojirimycin (Zavesca^{®}), which decreases the biosynthesis of glucosylceramides and thereby reduces accumulation (Futerman, A.H. et al., Trends Pharmacol. Sci. 25:147-151 (2004)). N-butyldeoxynojirimycin is thought to act by inhibiting glucosylceramide synthetase, the first enzyme in the glycosphingolipid biosynthetic pathway. Because N-butyldeoxynojirimycin causes globozoospermia when administered to rodents (van der Spoel, A.C. et al., Proc. Natl. Acad. Sci. U. S. A. 99:17,173-17,178 (2002)), male Gaucher's patients who receive the drug are advised to take appropriate birth control measures. Given the expression of GBA2 in the Sertoli cells of wild-type mouse testis, the presence of globozoospermia in the GBA2 knockout mice, and the ability of alkylated deoxynojirimycin derivatives to inhibit the GBA2 enzyme (Matern, H. et al., FEBS Lett. 314:183-186 (1992); Matern, H. et al., J. Biol. Chem. 272:11,261-11,267 (1997)), it is possible that inhibition of testicular GBA2 rather than glucosylceramide synthetase underlies the globozoospermia which is a side effect in Gaucher's subjects treated with butyldeoxynojirimycin. If this hypothesis is correct, then more selective inhibitors of GBA1 than butyldeoxynojirimycin may be useful in the treatment of Gaucher's disease while GBA2-specific inhibitors may be useful as male contraceptives.

Why does loss of an enzyme that is selectively expressed in Sertoli cells and that hydrolyzes glucosylceramide give rise to malformed sperm in the *Gba2*-/- mice. Sertoli cells play an essential role in the differentiation and maturation of germ cells (Russell, L.D., Gamete Res. 3:179-202 (1980)), and the current results suggest that catabolism of glycolipids by the GBA2 enzyme is an important aspect of this process. The source of the glycolipids that accumulate in the testis of the knockout mice is not known but by analogy to Gaucher's patients in whom these lipids accumulate in macrophages by phagocytosis (Beutler, E. and Grabowski, G.A., Gaucher Disease. In The Metabolic & Molecular Bases of Inherited Disease. C.R. Scriver et al., editors. New York: McGraw-Hill. 3,635-3,668 (2001)), an interesting possibility is that glucosylceramides are normally transferred from developing germ cells to Sertoli cells for subsequent breakdown. Loss of the GBA2 enzyme causes glucosylceramide accumulation in Sertoli cells, which may in turn further disrupt transport of glycolipid from germ cells leading to the formation of abnormal sperm. There are extended Sertoli-germ cell contacts through which glycolipid transport could take place, including desmosomes, gap junctions, and two anatomically unique contacts referred to as Sertoli ectoplasmic junctional specializations and the tubulobulbar complex (Russell, L.D., Gamete Res. 3:179-202 (1980)). It also is possible that the source of glycolipid is the Sertoli cell itself and that accumulation disrupts normal Sertoli-germ cell interactions leading to malformed sperm. The cellular source of the accumulating glucosylceramide and the mechanism of toxicity may be revealed by further histological and biochemical studies in the *Gba2*-/-mice.

Globozoospermia is an infrequent cause of infertility in human males (Brugh, V.M.R. et al., Urol. Clin. North Am. 30:143-152 (2003)), and while mutations in several mouse genes, including casein kinase II alpha (Xu, X. et al., Nat. Genet. 23:118-121 (1999)), the Golgi associated PDZ- and coiled-coil motif-containing protein (GOPC) (Yao, R. et al., Proc. Natl. Acad. Sci. U. S. A. 99:11,211-11,216 (2002)), and *Gba2,* give rise to round-headed sperm, the genetic basis of the human inherited disease is not known. Mutations in human *GBA2* (Olivier, P. et al., Hum. Reprod. 20:1,314- 1,318 (2005) may be the cause of globozoospermia.

GBA2 (-/-) mice furthermore exhibit retarded tissue regeneration, especially soft tissue regeneration. In mice and men, up to 70% of liver tissue can be removed surgically and will be replaced by subsequent tissue regeneration *in vitro*. This regeneration is retarded in GBA2 (-/-) mice (data not shown). Thus, in one aspect of embodiment (1), the medicament comprising GBA2 is suitable for enhancement of tissue regeneration. This includes tissue regeneration after surgical tissue removal, accidental tissue destruction and disease induced tissue destruction (like cirrhosis of the liver and liver fibrosis).

GBA2 (-/-) mice do also possess lower thrombocyte levels than their wild type counterparts. Thus, in a further aspect of embodiment (1), the medicament comprising GBA2 is suitable for enhancement of blood formation.

Finally, GBA2 (-/-) mice display increased expression of genes which are characteristic for Alzheimer's and Parkinson's disease (cDNA-microarray data, not shown). Thus, in a further aspect of embodiment (1), the medicament comprising GBA2 is suitable for treatment of Alzheimer's and Parkinson's disease.

The present invention is described in more detail by reference to the following examples. It should be understood that these examples are for illustrative purpose only and are not to be construed as limiting the invention.

### Examples

In the following examples, standard techniques of recombinant DNA technology and molecular biology were used that were described in various publications, e.g. Sambrook et al. (2001), Molecular Cloning: A Laboratory Manual, 3rd edition, Cold Spring Harbor Laboratory Press, or Ausubel et al. (1987), Current Protocols in Molecular Biology, Current Protocols in Immunotogy, Current Protocols in Protein Science, and Current Protocols in Cell Biology, edition as of 2002, Wiley Interscience. Unless otherwise indicated, all enzymes, antibodies, cells, reagents, devices and kits were used according to the manufacturers' instructions.

Student's unpaired t test was used to compare data sets, a P value <0.05 was considered significant.

### Example 1: Construction of Gba2 Knockout Mouse.

General knockout strategy (see Fig. 1C): A targeting vector composed of a 5.5 kb long arm (nt 1879 to nt 7394 of SEQ ID NO: 17) of the mouse *Gba2* gene (SEQ ID NO:17), a ca. 2.0 kb short arm (nt 9451 of SE ID NO:17, ending ca. 60 nt downstream of nt 11613 of SEQ ID NO:17 on the mouse chromosome) of the mouse *Gba2* gene (SEQ ID NO:17), two copies of a viral thymidine kinase gene (TK) conferring sensitivity to gancyclovir, and a DNA fragment specifying the ACN cassette (Bunting, M. et al., Genes Dev. 13:1524-1528 (1999)), was assembled. In more detail, the long and short arm were amplified by PCR from genomic DNA of 12956/SvEv mouse embryonic stem cells (ES) (Lund, E.G. et al., J. Biol. Chem. 278:22980-22988 (2003)).

The long arm of 5.5 kb length with EcoRI restriction sites at both ends was amplified using the sense primer 5'-CAGGAGCAGAGAGCAATGA-3' (SEQ ID NO:5) and the antisense primer 5'- GCATGGAATGAAGCAAACAGGAGGA-3' (SEQ ID NO:6). The short arm of about 2.0 kb length with a XhoI restriction site at its 5'end and a SalI restriction site at its 3' end was amplified by PCR using the sense primer 5'-CATCTGGCTCCTTAGTTCTCT-3' (SEQ ID NO:7) and the antisense primer 5'-GCGATTGTCCTATTGGCTCA-3' (SEQ ID NO:8). Thus, exons 5 to 10 of *Gba2* were left out in the amplification (Fig. 1C). The long arm was introduced into the EcoRI cloning site of pBluescript vector II KS+ (SEQ ID NO: 11).

The short arm was introduced into the PpolII-neoPA-HSVTK vector (Mansour, S.L. et al., Nature 336:348-352 (1988)) which comprises two subsequent HSV-TK cassettes (herpes simplex virus thymidine kinase cassette: SEQ ID NO:9, Accession No: V00470). Said vector was construed by Shun Ishibashi, 1991. The TK cassettes are necessary for negative selection using ganciclovir. For introducing the short arm into the vector, the PpolII-neoPA-HSVTK vector was opened with XhoI and the short arm was ligated into the vector 5' (upstream) of the TK cassettes. The SalI site between short arm and TK cassette gets lost during the ligation. Thereafter, a nucleic acid segment comprising the short arm and the TK cassettes was cut from the vector using XhoI and SalI.

The ACN (Angiotensin-Converting-Neomycin) cassette was cut from the vector represented by SEQ ID NO:10 and having the accession number AF169416 (Bunting, M. et al., Genes Dev. 13:1524-1528 (1999)) by restriction digestion using XhoI and BglI. It is approximately 3.7 kb long. ACN is a positive selection marker selecting for resistance against the antibiotic G418. The ACN cassette is completely described in (Bunting, M. et al., Genes Dev. 13:1524-1528 (1999)) and has the following sections: loxP-tACE-Cre-PolII-Neo-loxP. Thus, it contains a PolII promoter which can be specifically activated during spermatogenesis. Due to the flanking loxP sites, the ACN cassette can be removed by the enzymatic activity of Cre during spermatogenesis from the disrupted allele (compare Fig. 1C).

The nucleic acid segment comprising the ACN cassette was ligated downstream of the long arm into the SalI restriction site of the pBluescriptII KS+ comprising the long arm. Downstream of the ACN cassette, the short arm including the two TK cassettes was ligated into the XhoI site of the vector. In the resulting construct, the ACN cassette is positioned between long and short arm, and at the 3'end of the short arm two TK cassettes are positioned (Fig. 1C, targeting construct). The total length of the construct is 12.4 kb. The constructs comprising the inserts in the correct orientation were identified by nucleic acid sequencing, the sequence thereof being shown in SEQ ID NO: 18.

ES cells derived from the 12956/SvEv mouse strain were electroporated, selected, and used to produce chimeric mice as described (Lund, E.G. et al., J. Biol. Chem. 278:22980-22988 (2003)). High percentage male chimeras arising from six different ES cell clones were crossed with female C57BL/6J mice to generate independent lines of animals carrying the disrupted *Gba2* allele. Routine genotyping of mice was performed by PCR on genomic DNA extracted from tails using a direct lysis kit (102-T, Viagen Biotech) with the following oligonucleotides:
primer 1: 5'-GTGCTGGCTCACTGAGCTGGA-3' (SEQ ID NO: 12),
primer 2: 5'-CCAGTCAGCAGTATCATGAATCAA-3' (SEQ ID NO:13),
primer 3: 5'- GCATGTACAACACATA-CGATGTCCA-3' (SEQ ID NO:14).

### Example 2: Cholesterol and bile acid metabolism.

Intestinal cholesterol absorption, bile acid excretion rates, pool size and composition, and tissue and serum lipid levels were measured as described ((Lund, E.G. et al., J. Biol. Chem. 278:22980-22988 (2003); Turley, S.D. et al., J. Lipid. Res. 35:328-339 (1994); Schwarz, M. et al., J. Lipid. Res. 39:1833-1843 (1998)).

### Example 3: Enzyme assays.

Bile acid β-glucosidase and transferase activities were determined as described (Matern, H. et al., FEBS Lett. 314:183-186 (1992); Matern, H. et al., Proc. Natl. Acad. Sci. U. S. A. 81:7036-7040 (1984)). Glucosylceramidase activity was assessed as in (Dinur, T. et al., Anal. Biochem. 136:223-234 (1984)).

### Example 4: Real-time RT-PCR.

Total RNA was prepared from tissues using an RNA STAT-60 kit (Tel-Test). Equal amounts of RNA from four or six mice were pooled and treated with DNAse I (Ambion). First-strand cDNA was synthesized and subjected to real time RT-PCR as described (Cheng, J.B. and Russell, D.W., J. Biol. Chem. 279:37,789-37,797 (2004)). The relative amount of a target mRNA was calculated using the comparative threshold cycle (CT) method. Cyclophilin mRNA was used as an invariant control. Oligonucleotide primer sequences used to detect GBA2 mRNA were:
5'-GGAGACCTTTTCAAGCTAACAACAT-3' (SEQ ID NO:15) and
5'- GGTACCACCACTTCAAGTACCTCAA-3' (SEQ ID NO:16).
A full-length cDNA (SEQ ID NO:3) encoding the mouse GBA2 enzyme (SEQ ID NO:4) was isolated by RT-PCR from liver mRNA.

### Example 5: Recombinant GBA2 protein expression and antibody production.

A 500 bp cDNA fragment encoding amino acids 547 to 716 and a 1500 bp DNA fragment encoding amino acids 1 to 508 of the mouse GBA2 protein (SEQ ID NO:4) were subcloned independently into the prokaryotic expression vector pET-45b(+) (Novagen) following the vector manufacturer's instructions. Recombinant protein was purified from *E. coli* by affinity chromatography on Ni-NTA columns following the vector manufacturer's instructions. Aliquots of each GBA2 protein fragment (500 µg) were mixed with 1 ml Freund's adjuvant (MP Biochemicals) and injected subcutaneously into rabbits. After two weeks, a booster dose of 250 µg GBA2 protein fragment was administered in Freund's incomplete adjuvant. The boosting procedure was repeated four times. For use of the resulting antibodies see Example 12.

### Example 6: Immunoblot analyses of GBA2.

Freshly isolated or frozen liver, testes, and brain of mice were homogenized in two volumes of ice cold sucrose buffer/g tissue (250 mM sucrose, 10 mM Tris-Cl, pH 7.4, 1 mM sodium EDTA) supplemented with Complete Protease Inhibitors (EDTA-free, Roche). Homogenates were centrifuged at 800 x g for 10 min at 4°C. Supernatants from this step were centrifuged for 20 min at 8,000 x g at 4°C and the pellets discarded. Supernatants were again centrifuged at 55,000 x g for 30 min at 4°C in a Beckman TLA 100.2 rotor. The membrane pellets were resuspended in SDS-lysis buffer (1% (w/v) SDS, 10 mM Tris-Cl, pH 6.8, 100 mM NaCl, 1 mM EDTA, 1 mM EGTA), and analyzed by immunoblotting using standard procedures.

### Example 7: Microscopy.

*Gba2*+/+ and *Gba2*-/- mouse testes from 2-month-old animals were fixed for 4 h in 4% (v/v) paraformaldehyde before freezing in OCT cryoprotectant (Tissue-Tek, Sakura Finetek). Cryosections (8-10 µm) were collected on poly-L-lysine-coated slides (Fisher). For immunofluorescent staining, sections were washed 3 x 15 min with PBS, permeabilized with PBS containing 0.2% (v/v) Triton X-100, and blocked for 1 h at room temperature with PBS containing 5% (w/v) bovine serum (Sigma), 0.025% Triton X-100, and 10 % (v/v) donkey serum (Sigma), followed by overnight incubation at 4°C or room temperature with primary antibodies. Primary antibodies were used at 1:1000 to 1:2000 dilutions and included rabbit polyclonal anti-GBA2 and anti-DAZL (Hamra, F.K. et al., Proc. Natl. Acad. Sci. U. S. A. 99:14931-14936 (2002)) antibodies (both protein A-purified), and a monoclonal anti-tyrosine tubulin antibody (TUB-1A2, Sigma).

After 3 x 15 min washes with PBS, the sections were incubated with combinations of either Alexa488-conjugated anti-rabbit secondary antibodies (diluted 1:2000; Invitrogen Molecular Probes) or Alexa594-conjugated anti-mouse antibodies (1:2000; Invitrogen Molecular Probes) for 1 h at room temperature in blocking buffer. Sections were washed 3 x 15 min with PBS and mounted with ProLong® Gold with DAPI (Invitrogen Molecular Probes). Images were acquired on a Zeiss Axioplan 2 fluorescence microscope. Periodic acid Schiff staining to visualize glycolipids was done using reagents from American Master*Tech Scientific. For electron microscopy, caudal epididymal spermatozoa from adult mice were fixed in 3% (v/v) paraformaldehyde, 0.1% (v/v) glutaraldehyde in 0.1% phosphate buffer (pH 7.4), post-fixed in 1% (w/v) osmium tetroxide in the same buffer, and embedded in a plastic resin according to standard procedures. Following sectioning, specimens were examined with a JEM-2100F transmission electron microscope.

### Example 8: Sperm analyses.

Epididymides from sexually mature mice were minced in PBS and incubated for 60 min at 37°C. Debris was removed and sperm counted using a hemocytometer. For velocity measurements, mature sperm were isolated from the cauda of epididymides dissected from 12-16 week-old males. The tissue was placed in a 1 ml culture dish containing 1 ml KSOM medium (Specialty Media) and incisions were made at four sites with scissors. The dish was placed in a 37°C incubator for 15 min and the medium containing the released sperm was decanted. Aliquots of-the sperm suspension (20 µl) were placed in an 80-µm deep chamber and analyzed with the IVOS Sperm Analyzer (Version 12, Hamilton Thorne Biosciences). Cell tracks were captured for 0.5 s at 60 Hz. All procedures were performed at 37°C. A minimum of 300 cells were analyzed from each animal (n = 2 per *Gba2* genotype) to determine percentage of motile sperm, mean velocity (distance in µm traveled over a smoothed average path per sec), linear velocity (the straight-line distance in µm between the initial and final locations of the sperm head divided by the total time in sec), and curvilinear velocity (total distance swum by the sperm head from image to image divided by the total elapsed time).

### Example 9: In vitro fertilization.

Procedures were performed as described (Howlett, S.K. and Bolton, V.N. J. Embryol. Exp. Morphol. 87:175-206 (1985)). In brief, caudal epididymal sperm were collected in M16 medium (Sigma, M7292) supplemented with 30 mg/ml BSA and capacitated for 90 min at 37°C in a 5% CO₂ atmosphere. Approximately 1 million spermatozoa were added to 1-ml drop cultures of fertilization medium containing unfertilized, cumulus enclosed eggs collected at ca. 13 h post-human chorionic gonadotropin treatment. Eggs were left with spermatozoa for ca. 5 h, washed, placed in microdrop cultures of KSOM medium (Specialty Media), and incubated at 37°C in a 5% CO₂ atmosphere. Blastocyst formation was assessed by light microscopy.

### Example 10: Glycolipid analyses.

Extraction and analyses of glycolipids were performed as described (Bodennec, J. et al., J. Lipid Res. 44:218-226 (2003); Sandhoff, R. et al., J. Biol. Chem. 280:27,310-27,318 (2005)). Structural analyses were carried out on an Applied Biosystems QTRAP4000 mass spectrometer with an electrospray source (Merrill, A.H.J. et al., Methods 36:207-224 (2005)). Briefly, samples were resuspended in 0.5 ml 9:1 methanol:H₂0 (v/v) and analyzed by direct infusion at a rate of 5 µl/min into the mass spectrometer. Scan types consisted of precursor ion scans for 264.4 *m*/*z* representing sphingosine-based glycolipids and 266.4 *m*/*z* representing sphinganine-based glycolipids. Neutral loss scans for 162 Da and 180 Da masses representing those of O-linked hexose moieties were also performed. To determine if the observed monohexosylceramides were glucosylceramides or galactosylceramides, samples from *Gba2-*/*-* mice were separated by normal phase liquid chromatography followed by mass spectrometry-mass spectrometry to distinguish these species (Schwarz, M. et al., J. Lipid. Res. 39:1833-1843 (1998)).

### Example 11: Tissue distribution of mouse GBA2.

Real time RT-PCR was used to determine the tissue distribution of the mouse GBA2 mRNA. Whereas the human GBA2 mRNA is abundant in brain, heart, skeletal muscle, and kidney (Matern, H. et al., J. Biol. Chem. 276:37929-37933 (2001)), the mouse mRNA was most abundant in the testis and brain with lesser amounts present in 14 other tissues (Fig. 1A).

Two fragments of the mouse GBA2 protein spanning amino acids 1 to 508 and 547 to 716 were expressed in *E. coli*, purified, and used to raise polyclonal antisera in rabbits (see Example 5). The antiserum generated against the longer recombinant antigen recognized a GBA2 protein of the expected molecular weight (~110,000) that was absent in GBA2 knockout mice (see below) and most abundant in the brain, liver, and testis (Fig. 1B).

The amount of GBA2 mRNA was proportional to the level of protein in the tissues examined with the exception of the liver where the protein was more abundant than would be expected from the level of mRNA (compare Figure 1A to 1B). Similar immunoblotting results were obtained with the antiserum raised against the shorter recombinant GBA2 fragment (data not shown).

### Example 12: Knockout of mouse Gba2 gene.

Comparison of the GBA2 cDNA and genome sequences indicated that mouse *Gba2* was located on chromosome 4B1 and contained 17 exons (Fig. 1C). Neighbouring genes were located 0.4 kb 5' (1110029E03Riken) and 0.1 kb 3' (*Creb3*) of the *Gba2* locus and for this reason a knockout strategy was designed to ensure that the introduced mutation would not affect expression of these closely linked genes. A targeting construct was assembled to delete exons 5 through 10 of *Gba2* and to introduce the ACN cassette (see Example 1) followed by homologous recombination in ES cells. The ACN cassette contained a selectable marker which conferred neomycin resistance, and a *Cre* recombinase gene linked to a testis-specific promoter (Bunting, M. et al., Genes Dev. 13:1524-1528 (1999)). Following transmission of the disrupted allele through the germ line of male mice, the *Cre* recombinase (which was activated during spermatogenesis due to its testis specific promoter) removed the selectable marker leaving behind a LoxP site at the position of the introduced deletion in the knockout allele (Fig. 1C). Subsequent real time RT-PCR experiments showed that expression of the two immediate flanking genes was not altered in *Gba2-*/*-* mice (data not shown).

### Example 13: Characterization of Gba2-/- mice.

A PCR-based genotyping assay was developed to follow inheritance of the deleted *Gba2* allele (Fig. 2A), and revealed the expected proportions of heterozygous *(Gba2+*/*-)* and homozygous (*Gba2-*/*-*) offspring. Immunoblotting experiments showed that the level of GBA2 protein expressed in the brain, testis, and livers of *Gba2+*/*-* mice was half that of wild-type controls, and that *Gba2-*/*-* mice expressed no detectable protein (Fig. 2B). The GBA2 enzyme catalyzes both the cleavage and formation of bile acid-glucose conjugates (Matern, H. et al., FEBS Lett. 314:183-186 (1992); Matern, H. et al., Proc. Natl. Acad. Sci. U. S. A. 81:7036-7040 (1984)) (Fig. 2C). Of these two reactions, bile acid glucosidase activity in the brain was unchanged in *Gba2+*/*-* mice and was reduced by ∼50% in this tissue from *Gba2-*/*-* mice. The activity was decreased by ∼30% in the testis and liver of *Gba2+*/*-* animals, and by ∼50-85% in these tissues from *Gba2-*/*-* mice. In contrast, bile acid glucosyltransferase activity was decreased by 20 to 50% in the tissues of *Gba2+*/*-* mice, and reduced to undetectable levels in *Gba2*-/- animals (Fig. 2C). These data indicated that GBA2 was responsible for a variable amount of bile acid glucosidase activity in these three tissues and for most if not all of the measurable bile acid glucosyltransferase activity. The body weights of adult *Gba2-*/*-* mice were normal, and tissues in the mutant mice, including the liver, spleen, and brain weighed the same as those in control animals (Tab. 1). In contrast to these findings, the testes of the knockout mice were significantly smaller (P < 0.003) than those of wild-type mice.

Lipid and bile acid metabolism in Gba2-/- mice: Plasma cholesterol and triglyceride levels were similar in mice of different *Gba2* genotypes (Tab. 1), and analysis of plasma by size-exclusion chromatography revealed a normal distribution and amount of cholesterol present in the various subclasses of lipoprotein particles (data not shown). HPLC analyses revealed a bile acid pool size and composition in *Gba2-*/*-* mice that were not significantly different from wild-type controls (Table 1). Analysis of fecal bile acid excretion rates showed that the synthesis of bile acids was similar in knockout and wildtype mice (Tab. 1). In agreement with these findings, intestinal cholesterol absorption in the mutant mice was not different from that in control mice (Tab. 1). Thus, GBA2 did not play a major role in cholesterol and bile acid metabolism, and it was concluded that the enzyme might act on alternate substrates *in vivo.*

**Table 1: Physical and physiologic characteristics of Gba2+/+ and Gba2-/- mice. Values are mean ± SEM. Weights were derived from 12 male mice, 3-4 months of age, for each genotype. Physiologic measurements were made in n = 6-10 male or female mice in two or more experiments.**

| Parameter | *Gba2*+/+ | *Gba2*-/- |
|---|---|---|
| Body weight (g) | 23.8 ± 1.1 | 25.8 ± 1.9 |
| Tissue weight (%, rel. to body weight (bw.)) | | |
| Heart | 0.59 ± 0.039 | 0.52 ± 0.019 |
| Brain | 1.83 ± 0.068 | 1.66 ± 0.116 |
| Liver | 3.95 ± 0.132 | 3.78 ± 0.187 |
| Spleen | 0.28 ± 0.04 | 0.2 ± 0.015 |
| Kidney | 1.44 ± 0.025 | 1.42 ± 0.023 |
| Thymus | 0.67 ± 0.039 | 0.68 ± 0.035 |
| Lung | 0.60 ± 0.013 | 0.59 ± 0.026 |
| Testes | 0.93 ± 0.045 | 0.72 ± 0.03^{A} |
| Plasma triglyceride (mg/dl) | 126.1 ± 20.0 | 96.7 ± 15.6 |
| Plasma cholesterol (mg/dl) | 103.2 ± 4.2 | 96.4 ± 5.8 |
| Bile acid pool size (µmol/100g bw.) | 93.2 ± 7.8 | 92.6 ± 6.5 |
| Ratio Cholic/muricholic acid in bile acid pool | 1.6 | 1.0 |
| Fecal bile acid excretion rate (µmol/d/100g bw.) | 23.5 ± 2.7 | 19.2 ± 1.6 |
| Intestinal cholesterol absorption (%) | 66.2 ± 3.9 | 59.0 ± 3.1 |

| | | |
|---|---|---|
| ^{A}Significantly different from value in *Gba2*+/+ group (P<0.0029) | | |

### Example 14: Fecundity defect in Gba2-/- mice.

Crosses between male and female *Gba2*+/+ mice produced an average litter size of 8.1 pups, and crosses between *Gba2*+/- mice yielded a similar litter size of 7.5 pups (Table 2). In contrast, mating of *Gba2*-/- mice produced an average litter size of only 2.8 offspring. When *Gba2*-/- females were crossed with wildtype males, the litters were of normal size (7.4 pups); however, crosses between *Gba2*-/- males and wild-type females produced small litters (3.0 pups). These data indicated that loss of the GBA2 enzyme in male mice caused a reduction in fecundity but had no effects on reproductive fitness in female mice.

**Table 2: Fecundity defect in Gba2 -/- mice. Data derived from matings between animals of age <6 months.**

| *Gba2* Genotype | | No. of Matings | No. of Pregnancies | Litter Size^{A} |
|---|---|---|---|---|
| Male | Female | | | |
| +/+ | +/+ | 31 | 31 | 8.1 |
| +/- | -/+ | 35 | 35 | 7.5 |
| +/+ | -/- | 10 | 10 | 8.4 |
| -/- | +/+ | 9 | 3 | 3.0 |
| -/- | -/- | 64 | 40 | 2.8 |

| | | | | |
|---|---|---|---|---|
| ^{A}Mean litter size (number of live pups born/number of litters). | | | | |

### Example 15: Sperm morphology in Gba2-/- mice.

Fecundity defects in males can arise from a failure of spermiogenesis or fertilization. To distinguish between these possibilities, we first examined the structure of the sperm produced by *Gba2*-/- mice. Whereas sperm from wild-type mice had a normal sickle-headed morphology when examined by light microscopy, *Gba2*-/- sperm had abnormally large round heads (globozoospermia, Fig. 3A). Immunofluorescent staining of wild-type sperm with a lectin that recognizes carbohydrates in the acrosome, a DNA binding dye (DAPI) that labels nuclei, and a dye that selectively binds mitochondria (MitoFluor™ Red 589) revealed the classic outline of mammalian sperm (Fig. 3B). In comparison, *Gba2*-/- sperm exhibited diffuse lectin positive staining in round heads, indicating that an acrosome-like structure was present but disordered in these cells, and the presence of mitochondria in both the head and sheath of the sperm. Under the electron microscope, the heads of wild-type sperm showed ordered mitochondria in the sheath, well defined acrosomes at the tips, and large dense nuclei in the heads (Fig. 3C). In contrast, *Gba2*-/- sperm had distended round heads that lacked an obvious acrosome, disordered mitochondria in the sheaths and heads, and dense pyknotic nuclei.

### Example 16: Sperm parameters in Gba2-/- mice.

To determine sperm number, epididymides were dissected from *Gba2* wild-type and knockout mice and sperm removed by mincing the organ. Sperm counts were significantly lower (P < 0.02) in the mutant mice (Table 3). The percentage of mature motile sperm in the caudal epididymis was similarly reduced. Motion studies indicated that mature sperm from knockout mice exhibited slower path, linear, and track velocities compared to sperm of wild-type mice (Table 3).

**Table 3: Sperm count, motility, and swim speeds. Values are mean ± SEM derived from six male mice, 3-4 months of age for each genotype.**

| Parameter | *Gba2*^{+/+} | *Gba2*^{-/-} |
|---|---|---|
| Sperm count (no./ml) | 1.2 x 10⁷ ± 1 x 10⁶ | 7.1 x 106 ± 1.4 x 10^{6 B} |
| Sperm Motility (%) | 56 ± 1.3 | 33 ± 3.1 |
| Sperm velocity | | |
| (µm/S)^{A} | 126 ± 2.8 | 104 ± 7.0^{B} |
| Mean | 96 ± 4.6 | 70 ± 5.28 ^{B} |
| Linear | 257 ± 4.0 | 203 ± 17^{B} |
| Curvilinear | | |

| | | |
|---|---|---|
| ^{A} Values from n = 300 sperm isolated from n = 4 animals of each genotype. ^{B}Significantly different from value in *Gba2*+/+ group (P<0.03). | | |

### Example 17: Defective in vitro fertilization.

Mature sperm were collected from cauda epididymides of male *Gba2*+/+ and *Gba2-*/- animals, incubated in fertilization medium for 90 min, and then 1 million sperm/ml were incubated for five h with oocytes in cumulus masses isolated from wild-type female mice. Proceedings see Example 9. Examination by light microscopy showed that sperm from *Gba2*+/+ mice bound in large numbers to the zona pellucida of the egg while those from *Gba2*-/- mice did not (Fig. 4). The efficiency of blastocyst formation when wild-type sperm were incubated with wild-type oocytes was 31% (20 blastocysts developed from 64 eggs), and was 43% (19 blastocysts from 44 eggs) when sperm from *Gba2*+/- males were incubated with wild-type oocytes. In contrast, no blastocysts were formed when *Gba2*-/- sperm were co-cultured with wild-type eggs (0 blastocysts from 63 eggs).

### Example 18: GBA2 expression in testis.

During spermatogenesis, individual germ cells are surrounded by the lamellae of Sertoli cells within the testis. Interactions between the two cell types are required for proper cytological differentiation of sperm, their movement along the seminiferous tubules, and for release into the rete testis (Russell, L.D., Gamete Res. 3:179-202 (1980)). This symbiotic relationship raised the question of whether the anatomical and functional defects observed in *Gba2*-/- sperm were due to loss of GBA2 expression in germ cells or Sertoli cells. Histological analyses of *Gba2*-/- testis showed slightly distended lumens in individual seminiferous tubules and increased numbers of pyknotic germ cells (Fig. 5A). Consistent with the latter observation, TUNEL staining revealed a significantly increased (P < 5.5 x 10-6) number of apoptotic cells in the testis of *Gba2*-/- mice (199 ± 15 expiring cells) versus *Gba2*+/+ mice (57 ± 7 cells). Immunofluorescent staining for GBA2 revealed expression in Sertoli cells that overlapped that of tyrosine-tubulin (Fig. 5B,C), a posttranslationally modified form of tubulin selectively expressed in this testicular cell type (Oke, B.O. and Suarez-Quian, C.A. Biol. Reprod. 48:621-631 (1993)). The GBA2 stain was distinct from that of DAZL (Figure 5D), a germ cell marker protein (Hamra, F.K. et al., Proc. Natl. Acad. Sci. U. S. A. 99:14931-14936 (2002)).

### Example 19: Normal protein glycosylation in Gba2-/- testes.

The bile acid glucosidase activity of GBA2 is inhibited by the imino sugar 1-deoxynojirimycin (Matern, H. et al., FEBS Lett. 314:183-186 (1992); Matern, H. et al., J. Biol. Chem. 272:11,261-11,267 (1997)), and administration of this compound to mice causes male infertility and globozoospermia (van der Spoel, A.C. et al., Proc. Natl. Acad. Sci. U. S. A. 99:17,173-17,178 (2002); Suganuma, R. et al., Biol. Reprod. 72:805-813 (2005); Warden, C.M. et al., Hum. Reprod. 21:1309-1315 (2006)). 1-Deoxynojirimycin and its alkylated derivatives inhibit glucosidases involved in the processing of asparagine-linked carbohydrates attached to proteins as well as those involved in the biosynthesis of glycolipids (Elbein, A.D., Annu. Rev. Biochem. 56:497-534 (1987); Tifft, C.J. and Proia, R.L., Glycobiology 10:1249-1258 (2000)). These. observations suggested that the accumulation of improperly glycosylated proteins or glycolipids within the testis might underlie the decreased fertility observed in the *Gba2-*/*-* mice. To distinguish between these two possibilities, we first carried out a series of lectin blotting experiments to determine whether protein glycosylation was aberrant in the mutant mice. Membrane proteins were isolated from the testis of wild-type and knockout mice and separated by SDS-PAGE. Following transfer to membranes, glycosylated proteins were visualized by probing with eight different lectin-horse radish peroxidase complexes. Multiple different proteins were detected by each lectin; however, the patterns of proteins observed in mice of different *Gba2* genotypes were similar (data not shown). These results suggested that protein glycosylation was normal in the *Gba2*-/- mice and that GBA2 did not utilize glycoproteins as substrates.

### Example 20: Accumulation of glycolipids in Gba2-/- tissues.

Glycolipid accumulation in the mutant mice was monitored by extraction and TLC using two different methods (Bodennec, J. et al., J. Lipid Res. 44:218-226 (2003); Sandhoff, R. et al., J. Biol. Chem. 280:27,310-27,318 (2005)). Comparison of testicular glycolipids separated by one-dimensional TLC revealed normal levels of several fucosylated lipids known to be required for male fertility (Sandhoff, R. et al., J. Biol. Chem. 280:27,310-27,318 (2005)) in the knockout tissue, and the gradual accumulation of a glycolipid species that migrated to a position on the silica plate similar to those of two glucosylceramide standards (Fig. 6A). The accumulation of a similar glycolipid species in the brains of male and female *Gba2-*/- mice was observed after extraction and separation by a two-dimensional TLC method ((Bodennec, J. et al., J. Lipid Res. 44:218-226 (2003)), data not shown). The identity of the glycolipid that accumulated in the testis of the mutant mice was determined by electrospray ionization-mass spectrometry.

Precursor ion and neutral loss scans indicated that the accumulated glycolipid observed on the orcinol-stained TLC plate consisted of a mixture of d18:1/C16:0 and d18:1/C18:0 monohexosylceramides (Fig. 6B). Analysis of the accumulated lipid by normal phase liquid chromatography and mass spectrometry-mass spectrometry indicated that the hexose sugar was glucose. Similar structural results were obtained when glycolipids extracted from the liver and brain were analyzed (data not shown).

To determine whether GBA2 utilized glucosylceramide as a substrate, cDNAs encoding the mouse and human enzymes were introduced into cultured simian COS-7 cells, and the ability of the transfected cells to catalyze cleavage of a fluorescently-derivatized glucosylceramide was monitored by spectrophotometry (Dinur, T. et al., Anal. Biochem. 136:223-234 (1984)). Glucosylceramidase activity in mock transfected cells was 0.024 nmol product formed per min per mg protein assayed, whereas this activity increased to 0.33 nmol/min/mg in cells transfected with the human GBA2 cDNA, and to 0.18 nmol/min/mg in cells transfected with the mouse GBA2 cDNA (Fig. 6C). For comparison purposes, cell extracts were assayed for bile acid glucosidase activity using lithocholic acid glucoside as substrate. This activity was increased in transfected cells relative to mock-transfected cells and was 40-50-times higher than that measured with the glucosylceramide substrate (Fig. 6C). To show that GBA2 cleaved authentic glucosylceramide, transfected human embryonic kidney 293 cells were incubated with [¹⁴C]glucosylceramide- and radiolabeled lipids were examined after resolution by TLC. As shown in Fig. 6D, cells transfected with the mouse GBA2 expression vector produced large amounts of ceramide whereas mock-transfected cells did not.

### Example 21: Subcellular accumulation of glycolipids and localization of GBA2

Periodic acid Schiff staining of testis from 6- and 14-month-old *Gba2*-/- mice revealed the gradual accumulation of small glycolipid-containing vesicles within Sertoli cells that were not present in age-matched *Gba2*+/+ testis (Fig. 7A-D). Immunofluorescent staining of COS cells with an antibody directed against GBA2 revealed a reticular staining pattern characteristic of a resident protein of the ER that did not overlap with that of lysosomes (Fig. 7E,F).

### Example 22: Screening for GBA2-Antagonists.

The effect of various compounds on bile acid β-glucosidase and bile acid glucosyltransferase activity of GBA2 was tested with isolated recombinant GBA2 (Table 4, b and c) and with microsomal enzyme from wild type and Gba2 (-/-) mice (Table 4, a and d) using the method described in Matern et al., J. Biol. Chem. 272:17, 11261-11267 (Matern, H. et al., J. Biol. Chem. 272:11,261-11,267 (1997)). Except for butyl-dNM (Sigma), N-alkylderivatives of deoxynojirimycin were a gift of Prof. Dr. G. Legler, Institut für Biochemie, Universitat Köln, Germany. For dissolution of these derivatives, see H. Matern et al. J. Biol. Chem. 272, 11261-11267, 1997. Conduritol B epoxide is a known β-glucosidase inhibitor.

**Table 4: Effect of various compounds on bile acid β-glucosidase and bile acid glucosyltransferase activity of GBA2.**

| A) liver microsomal enzyme from wild type mouse | | | | |
|---|---|---|---|---|
| Addition | β-Glucosidase activity | | Transferase activity | |
| | µM | % of control | µM | % of control |
| Conduritol B epoxide 1-Deoxynojirimycin Butyl-dNM Carboxynonyl-dNM Dodecyl-dNM | 50 200 1 0.2 0.001 | 46.9 52.3 52.4 48.9 51.9 | 2000 200 2 0.05 0.0006 | 60.0 47.8 49.3 47.1 49.4 |

| B) human enzyme expressed in COS-7-cells | | | | |
|---|---|---|---|---|
| Addition | β-Glucosidase activity | | Transferase activity | |
| | µM | % of control | µM | % of control |
| Conduritol B epoxide 1-Deoxynojirimycin Butyl-d NM Carboxynonyl-dNM Dodecyl-dNM | 1000 10 0.2 0.05 0.0002 | 55.7 50.8 51.7 46.5 49.3 | 2000 200 2 0.2 0.002 | 64.6 52.7 45.5 46.9 50.7 |

| C) murine enzyme expressed in COS-7-cells | | | | |
|---|---|---|---|---|
| Addition | β-Glucosidase activity | | Transferase activity | |
| | µM | % of control | µM | % of control |
| Conduritol B epoxide 1-Deoxynojirimycin Butyl-d NM Ca rboxynonyl-d N M Dodecyl-dNM | 1000 50 0.4 0.02 0.0001 | 44.9 47.2 45.6 50.9 59.5 | 2000 200 1.5 0.05 0.001 | 48.6 45.3 50.5 57.3 47.3 |

| D) liver microsomal β-glucosidase activity from GBA2 (-/-) mice; Transferase activity was not detectable in these microsomes due to GBA2 knockout. | | | | |
|---|---|---|---|---|
| Addition | β-Glucosid ase activity | | | |
| | µM | % of control | | |
| Conduritol B epoxide | 20 | 58.9 | | |
| 1-Deoxynojirimycin | 500 | 42.8 | | |
| Butyl-dNM | 500 | 52.8 | | |
| Carboxynonyl-dNM | 20 | 41.9 | | |
| Dodecyl-dNM | 0.2 | 57.8 | | |

### Example 23: Activity comparison of recombinant GBA2 produced in insect cells and wild type GBA2.

Following the method for measurement of glucosidase activity described in ((Matern, H. et al., J. Biol. Chem. 272:11,261-11,267 (1997), the effect of several test compounds (like bivalent cations and detergents) on the glucosidase activity of recombinant GBA2 produced in insect cells (His-tagged isolated enzyme) and of human liver microsomes was compared. The glucosidase activity of human liver was tested in a microsomal preparation. The tested compounds were: cations including nickel, zinc, cobalt, manganese, calcium, magnesium and barium; detergents including Chaps, Triton X100, taurocholate, Brij58; and phospholipids including lyso-phosphatidylcholine, sphingomyelin, phosphatidylcholine, phosphatidyl-serine, phosphatidylethanolamine and phosphatidylinositol. The enzyme activity results for the recombinant GBA2 were similar or even better than the results for human liver microsomal GBA2 activity. Representative results are summarized in Table 5; the data for the remaining test compounds is not shown. Result: Expression of GBA2 in insect cells leads to recombinant GBA2 whose glucosidase activity is similar to or even higher than the glucosidase activity of human liver microsomes.

**Table 5: Glucosidase activity of microsomal human liver and GBA2 from insect cells upon addition of sphingomyelin (A, B) and cobalt (C, D). The microsomal preparations were diluted 1:8 with Chaps before use. Measurements were performed in duplicate or triplicate.**

| A) insect cell produced recombinant GBA2 | | | | | |
|---|---|---|---|---|---|
| test compound concentration/ sample No | incubation time | counts | mean value | enzyme, activity | % of control |
| blank value minus GBA2 1 | 15 | 118,75 | 116,65 | | |
| blank value minus GBA2 2 | | 114,54 | | | |
| control minus test compound 1 | 15 | 753,36 | 651,75 | 2,4734 | 100 |
| control minus test compound 2 | | 550,14 | | | |
| 0,15mum 1 | 15 | 579,66 | 591,95 | 2,1969 | 89 |
| 0,15mM 2 | | 604,24 | | | |
| 0,3mM 1 | 15 | 675,57 | 674,56 | 2,5788 | 104 |
| 0,3mM 2 | | 673,54 | | | |
| 0,75mM 1 | 15 | 541,76 | 537,54 | 1,9454 | 79 |
| 0,75mM 2 | | 533,32 | | | |
| 1,5mM 1 | 15 | 630,13 | 643,44 | 2,4349 | 98 |
| 1,5mM 2 | | 656,75 | | | |
| 3mM 1 | 15 | 699,42 | 652,37 | 2,4762 | 100 |
| 3mM 2 | | 605,31 | | | |
| 6mM 1 | 15 | 473,49 | 493,49 | 1,7418 | 70 |
| 6mM 2 | | 513,48 | | | |
| 12mM 1 | 15 | 489,65 | 510,93 | 1,8224 | 74 |
| 12mM 2 | | 532,21 | | | |

| B) human liver microsmes | | | | | |
|---|---|---|---|---|---|
| test compound concentration/ sample No. | incubation time | counts | mean value | enzyme activity | % of control |
| blank value minus microsomes 1 | 15 | 112,42 | 119,10 | | |
| blank value minus microsomes 2 | | 125,77 | | | |
| control minus test compound 1 | 15 | 220,71 | 230,79 | 2,0650 | 100 |
| control minus test compound 2 | | 240,87 | | | |
| 0,15mM 1 | 15 | 215,51 | 206,72 | 1,6200 | 78 |
| 0,15mM 2 | | 197,93 | | | |
| 0,3mM 1 | 15 | 187,68 | 195,30 | 1,4089 | 68 |
| 0,3mM 2 | | 202,92 | | | |
| 0,75mM 1 | 15 | 206,90 | 206,21 | 1,6106 | 78 |
| 0,75mM 2 | | 205,52 | | | |
| 1,5mM 1 | 15 | 207,84 | 201,66 | 1,5264 | 74 |
| 1,5mM 2 | | 195,48 | | | |
| 3mM 1 | 15 | 225,57 | 214,01 | 1,7548 | 85 |
| 3mM 2 | | 202,45 | | | |
| 6mM 1 | 15 | 207,54 | 208,40 | 1,6511 | 80 |
| 6mM 2 | | 209,26 | | | |
| 12mM 1 | 15 | 189,25 | 195,24 | 1,4077 | 68 |
| 12mM 2 | | 201,23 | | | |

| C) insect cell produced recombinant GBA2 | | | | | |
|---|---|---|---|---|---|
| test compound concentration/ sample No. | incubation time | counts | mean value | enzyme activity | % of control |
| blank value minus GBA2 1 | 15 | 119,25 | 126,72 | | |
| blank value minus GBA2 2 | | 134,18 | | | |
| blank value minus GBA2 3 | | | | | |
| control minus test compound 1 | 15 | 123,25 | 159,95 | 0,1392 | 100 |
| control minus test compound 2 | | | | | |
| control minus test compound 3 | | 196,65 | | | |
| 0,01mM 1 | 15 | 157,63 | 157,71 | 0,1288 | 93 |
| 0.01mM 2 | | | | | |
| 0,01mM 3 | | 157,78 | | | |
| 0,05mM 1 | 15 | 167,53 | 164,92 | 0,1622 | 117 |
| 0,05mM 2 | | 152,62 | | | |
| 0,05mM 3 | | 174,62 | | | |
| 0,1mM 1 | 15 | 161,62 | 164,54 | 0,1604 | 115 |
| 0,1mM 2 | | 167,45 | | | |
| 0,1mM 3 | | | | | |
| 0,5mM 1 | 15 | 206,30 | 212,08 | 0,3802 | 273 |
| 0,5mM 2 | | 217,85 | | | |
| 0,5mM 3 | | | | | |
| 1mM 1 | 15 | 237,74 | 238,11 | 0,5005 | 360 |
| 1mM 2 | | 260,51 | | | |
| 1mM 3 | | 216,07 | | | |
| 5mM 1 | 15 | | 264,31 | 0,6216 | 97 |
| 5mM 2 | | 208,68 | | | |
| 5mM 3 | | 319,94 | | | |

| D) human liver microsmes | | | | | |
|---|---|---|---|---|---|
| test compound concentration/ sample No. | incubation time | counts | mean value | enzyme activity | % of control |
| blank value minus microsomes 1 | 15 | 130,96 | 129,01 | | |
| blank value minus microsomes 2 | | 128,58 | | | |
| blank value minus microsomes 3 | | 127,50 | | | |
| control minus test compound 1 | 15 | 195,67 | 197,22 | 1,2611 | 100 |
| control minus test compound 2 | | 201,31 | | | |
| control minus test compound 3 | | 194,67 | | | |
| 0,001mM 1 | 15 | 179,50 | 84,391 | 1,0240 | 81 |
| 0,001mM 2 | | 1187,03 | | | |
| 0,001mM 3 | | 186,65 | | | |
| 0,005mM 1 | 15 | 192,98 | 186,39 | 1,0610 | 84 |
| 0,005mM 2 | | 191,68 | | | |
| 0,005mM 3 | | 174,52 | | | |
| 0,01mM 1 | 15 | 180,77 | 188,30 | 1,0961 | 87 |
| 0,01mM 2 | | 193,04 | | | |
| 0,01mM 3 | | 191,08 | | | |
| 0,05mM 1 | 15 | 223,91 | 224,46 | 1,7647 | 140 |
| 0,05mM 2 | | 223,75 | | | |
| 0,05mM 3 | | 225,71 | | | |
| 1mM 1 | 15 | 234,69 | 234,89 | 1,9576 | 155 |
| 1mM 2 | | 233,72 | | | |
| 1mM 3 | | 236,26 | | | |
| 5mM 1 | 15 | 233,59 | 239,941 | 2,0510 | 163 |
| 5mM 2 | | 235,10 | | | |
| 5mM 3 | | 251,13 | | | |

## Claims

1. Medicament or pharmaceutical composition comprising bile acid β-glucosidase 2 (GBA2) or a mutein of GBA2 having 1-10 amino acid residues deleted, added or substituted, or fragments of GBA2 having up to 100 amino acid residues deleted at the C-terminal and/or N-terminal, or derivatives of GBA2 having C-terminal PEGylation or amidation, or fusion proteins containing GBA2 as a first domain and a second domain containing a functional protein or peptide moiety.

2. The medicament or pharmaceutical composition of claim 1, which is for intravenous application.

3. The medicament or pharmaceutical composition of any one of claims 1 to 2, wherein the GBA2 is human GBA2.

4. The medicament or pharmaceutical composition of claim 3, wherein the GBA2 is GBA2 with SEQ ID NO:2.

5. The medicament or pharmaceutical composition of any one of claims 1 to 4 for use in the treatment or prevention of a glycolipid storage disease.

6. The medicament or pharmaceutical composition of any one of claims 1 to 4 for use in the treatment or prevention of a glycolipid storage disease of claim 5, wherein
(i) the glycolipid storage disease is caused by a defect in glycosphingolipid breakdown, and preferably is Gaucher's disease; and/or
(ii) the glycolipid and glycosphingolipid is a glucosylceramide.

7. The medicament or pharmaceutical composition of any one of claim 1 to 4 for use in the the treatment of male infertility, or for use in the prophylaxis or treatment of male infertility caused by therapeutic treatment with GBA1 inhibitors.

8. The medicament or pharmaceutical composition of any one of claims 1 to 4 for use in
(i) enzyme replacement therapy, preferably for replacement of GBA1, and/or
(ii) the treatment of Alzheimer's and Parkinson's disease.

9. Use of a GBA2 as defined in any one of claims 1 to 4 for preparing a medicament for the treatment or prophylaxis of a glycolipid storage disease or male infertility.

10. A method for screening for compounds and compositions which may be suitable in treatment or prophylaxis of a glycolipid storage disease as defined in claim 6 or male infertility as defined in claim 7, comprising the administration of a test compound or test composition to a transgenic non-human animal with a (+/-) or (-/-) GBA2 genotype or to a cell comprising a knockout construct for the knockout of GBA2 translation.

11. The method of claim 10, which composes administration of the test compound to a mouse or human cell comprising a knock-out construct that lacks exons 5 to 10 of the native GBA2 gene.

12. The method of claim 10, which comprises administration of the test compound to the transgenic non-human animal with the GBA2 (-/-) genotype.

13. The method of claim 10, wherein the transgenic non-human animal is the product of a knockout reaction using the knockout vector as defined in claim 11.

14. Use of a GBA2 inhibitor for manufacturing a medicament for contraception in male subjects or for tumor therapy, wherein the GBA2 inhibitor is selected from carboxynonyl-deoxynojirimycin and dodecyldeoxynojirimycin.

15. A GBA2 inhibitor or a pharmaceutical composition comprising a GBA2 inhibitor for use in contraception in male subjects or for use in tumor therapy, wherein the GBA2 inhibitor is selected from carboxynonyl-deoxynojirimycin and dodecyldeoxynojirimycin.

## Patentansprüche

1. Medikament oder pharmazeutische Zusammensetzung, die Gallensäure-β-Glucosidase 2 (GBA2) oder ein Mutein von GBA2 mit 1-10 deletierten, addierten oder substituierten Aminosäureresten oder Fragmente von GBA2, bei denen bis zu 100 Aminosäurereste am C-Terminus und/oder N-Terminus deletiert sind, oder Derivate von GBA2 mit C-terminaler PEGylierung oder Amidierung oder Fusionsproteine, die GBA2 als erste Domäne und eine zweite Domäne, die eine funktionelle Protein- oder Peptid-Struktureinheit enthält, enthalten, umfasst.

2. Medikament oder pharmazeutische Zusammensetzung gemäß Anspruch 1, die für die intravenöse Verabreichung vorgesehen ist.

3. Medikament oder pharmazeutische Zusammensetzung gemäß einem der Ansprüche 1 bis 2, wobei es sich bei dem GBA2 um humanes GBA2 handelt.

4. Medikament oder pharmazeutische Zusammensetzung gemäß Anspruch 3, wobei es sich bei dem GBA2 um GBA2 mit der SEQ ID Nr. 2 handelt.

5. Medikament oder pharmazeutische Zusammensetzung gemäß einem der Ansprüche 1 bis 4 zur Verwendung bei der Behandlung oder Prävention einer lysosomalen Speicherkrankheit (Glycolipid-Speicherkrankheit).

6. Medikament oder pharmazeutische Zusammensetzung gemäß einem der Ansprüche 1 bis 4 zur Verwendung bei der Behandlung oder Prävention einer lysosomalen Speicherkrankheit gemäß Anspruch 5, wobei
(i) die lysosomale Speicherkrankheit durch einen Defekt im Glycosphingolipid-Abbau verursacht ist und es sich dabei vorzugsweise um Morbus Gaucher handelt; und/oder
(ii) das Glycolipid und Glycosphingolipid ein Glucosylceramid ist.

7. Medikament oder pharmazeutische Zusammensetzung gemäß einem der Ansprüche 1 bis 4 zur Verwendung bei der Behandlung von männlicher Unfruchtbarkeit oder zur Verwendung bei der Prophylaxe oder Behandlung von männlicher Unfruchtbarkeit, die durch therapeutische Behandlung mit GBA1-Inhibitoren verursacht ist.

8. Medikament oder pharmazeutische Zusammensetzung gemäß einem der Ansprüche 1 bis 4 zur Verwendung bei der
(i) Enzymersatztherapie, vorzugsweise zum Ersatz von GBA1, und/oder
(ii) Behandlung von Alzheimer- und Parkinson-Krankheit.

9. Verwendung eines GBA2 gemäß einem der Ansprüche 1 bis 4 zur Herstellung eines Medikaments zur Behandlung oder Prophylaxe einer lysosomalen Speicherkrankheit oder männlicher Unfruchtbarkeit.

10. Verfahren zum Screening nach Verbindungen und Zusammensetzungen, die möglicherweise für die Behandlung oder Prophylaxe einer lysosomalen Speicherkrankheit geeignet sind, wie sie in Anspruch 6 definiert sind, oder von männlicher Unfruchtbarkeit, wie sie in Anspruch 7 definiert sind, umfassend die Verabreichung einer Testverbindung oder Testzusammensetzung an ein transgenes nichthumanes Tier mit einem (+/-)- oder (-/-)-GBA2-Genotyp oder an eine Zelle, die ein Knockout-Konstrukt für den Knockout der GBA2-Translation umfasst.

11. Verfahren gemäß Anspruch 10, das die Verabreichung der Testverbindung an eine Maus- oder humane Zelle, die ein Knockout-Konstrukt umfasst, dem die Exons 5 bis 10 des nativen GBA2-Gens fehlen, umfasst.

12. Verfahren gemäß Anspruch 10, das die Verabreichung der Testverbindung an das transgene nichthumane Tier mit dem GBA2-(-/-)-Genotyp umfasst.

13. Verfahren gemäß Anspruch 10, wobei das transgene nichthumane Tier das Produkt einer Knockout-Reaktion unter Verwendung des Knockout-Vektors, wie er in Anspruch 11 definiert ist, ist.

14. Verwendung eines GBA2-Inhibitors zur Herstellung eines Medikaments zur Empfängnisverhütung bei männlichen Patienten oder für die Tumortherapie, wobei der GBA2-Inhibitor aus Carboxynonyldesoxynojirimycin und Dodecyldesoxynojirimycin ausgewählt ist.

15. GBA2-Inhibitor oder pharmazeutische Zusammensetzung, die einen GBA2-Inhibitor umfasst, zur Verwendung bei der Empfängnisverhütung bei männlichen Patienten oder zur Verwendung bei der Tumortherapie, wobei der GBA2-Inhibitor aus Carboxynonyldesoxynojirimycin und Dodecyldesoxynojirimycin ausgewählt ist.

## Revendications

1. Médicament ou composition pharmaceutique comprenant de la β-glucosidase 2 d'acide biliaire (GBA2) ou une mutéine de GBA2 présentant 1 à 10 résidus acide aminé supprimés, ajoutés ou substitués, ou des fragments de GBA2 présentant jusqu'à 100 résidus acide aminé supprimés en extrémité C et/ou en extrémité N, ou des dérivés de GBA2 présentant une PEGylation ou une amidation C-terminale, ou des protéines de fusion contenant GBA2 en tant que premier domaine et un second domaine contenant une protéine fonctionnelle ou une fraction peptide.

2. Médicament ou composition pharmaceutique selon la revendication 1, destinée à une application par voie intraveineuse.

3. Médicament ou composition pharmaceutique selon l'une des revendications 1 ou 2, dans lequel la GBA2 est une GBA2 humaine.

4. Médicament ou composition pharmaceutique selon la revendication 3, dans lequel la GBA2 est une GBA2 présentant le SEQ. ID n°2.

5. Médicament ou composition pharmaceutique selon l'une quelconque des revendications 1 à 4, pour une utilisation dans le traitement ou la prévention d'une maladie du stockage des glycolipides.

6. Médicament ou composition pharmaceutique selon l'une quelconque des revendications 1 à 4, pour une utilisation dans le traitement ou la prévention d'une maladie du stockage des glycolipides selon la revendication 5, où
(i) la maladie du stockage des glycolipides est provoquée par un défaut de dégradation des glycosphingolipides, et est de préférence la maladie de Gaucher ; et/ou
(ii) le glycolipide et le glycosphingolipide sont un glycosylcéramide.

7. Médicament ou composition pharmaceutique selon l'une quelconque des revendications 1 à 4, pour une utilisation dans le traitement de la stérilité masculine, ou pour une utilisation dans la prophylaxie ou le traitement de la stérilité masculine provoquée par un traitement thérapeutique avec des inhibiteurs de GBA1.

8. Médicament ou composition pharmaceutique selon l'une quelconque des revendications 1 à 4, pour une utilisation dans
(i) une thérapie enzymatique substitutive, de préférence destinée au remplacement du GBA1, et/ou dans
(ii) le traitement de la maladie d'Alzheimer et de la maladie de Parkinson.

9. Utilisation d'une GBA2 telle que définie dans l'une quelconque des revendications 1 à 4, pour préparer un médicament destiné au traitement ou à la prophylaxie d'une maladie du stockage des glycolipides ou de la stérilité masculine.

10. Procédé de sélection de composés et de compositions pouvant être approprié(e)s dans le traitement ou la prophylaxie d'une maladie du stockage des glycolipides selon la revendication 6 ou de la stérilité masculine selon la revendication 7, comprenant l'administration d'un composé test ou d'une composition test à un animal transgénique non humain ayant un génotype GBA2 (+/-) ou (-/-) ou à une cellule comprenant un construct knock-out pour la neutralisation de la traduction de GBA2.

11. Procédé selon la revendication 10, qui comprend l'administration du composé test à une souris ou une cellule humaine comprenant un construct knock-out n'ayant pas les exons 5 à 10 du gène GBA2 natif.

12. Procédé selon la revendication 10, qui comprend l'administration du composé test à un animal transgénique non humain présentant le génotype GBA2 (- / -).

13. Procédé selon la revendication 10, dans lequel l'animal transgénique non humain est le produit d'une réaction knock-out en utilisant le vecteur knock-out tel que défini dans la revendication 11.

14. Utilisation d'un inhibiteur de GBA2 pour fabriquer un médicament destiné à la contraception chez des sujets masculins ou à une thérapie tumorale, dans laquelle l'inhibiteur de GBA2 est sélectionné parmi la carboxynonyl-désoxynojirimycine et la dodécyldésoxynojirimycine.

15. Inhibiteur de GBA2 ou composition pharmaceutique comprenant un inhibiteur de GBA2 destiné(e) à une utilisation dans la contraception chez des sujets masculins ou à une utilisation en thérapie tumorale, dans lequel l'inhibiteur de GBA2 est sélectionné parmi la carboxynonyl-désoxynojirimycine et la dodécyldésoxynojirimycine.


### SEQUENCE LISTING

<110> Rheinisch-Westfaelische Technische Hochschule Aachen
   University of Texas, Southwestern Medical Center
   Yildiz, Yildiz
   Matern, Heidrun
   Matern, Siegfried
   Russell, David W.
<120> THERAPEUTIC USE OF GBA2
<130> 072130wo
<150> US 60/855,524 <151> 2006-10-31
<160> 18
<170> PatentIn version 3.3
<210> 1
   <211> 2784
   <212> DNA
   <213> homo sapiens
<400> 1
   atggggaccc aggatccagg gaacatggga accggcgtcc cagcctcgga gcagataagc      60
   tgtgccaaag aggatccaca agtttattgc cctgaagaga ctggcggcac caaggatgtg     120
   caggttacag actgtaagag tcccgaagac agccgacccc caaaagagac ggactgctgc     180
   aatccggagg actctgggca gctgatggtt tcctatgagg gtaaagctat gggctaccag     240
   gtgcctccct ttggctggcg catctgtctg gctcatgagt ttacagagaa gaggaaaccc     300
   tttcaagcta acaacgtctc cctaagcaac atgataaagc atataggcat gggcttgagg     360
   tacctgcagt ggtggtaccg gaagacccat gtggaaaaga agacaccttt catcgacatg     420
   atcaattctg tacccctaag acagatttat ggttgtccct tgggtggcat cgggggaggc     480
   actattaccc gtggctggag aggccagttc tgtcgttggc agcttaaccc tggaatgtat     540
   cagcaccgga cagtcatcgc tgaccaattc acagtgtgcc tgcgtcggga agggcagact     600
   gtgtaccagc aagtcctgtc cctggagcgc ccaagtgtcc tccgcagctg gaactggggc     660
   ctgtgtgggt actttgcttt ctaccatgcc ctctatcccc gagcctggac tgtctatcag     720
   cttcctggcc agaatgtcac cctcacctgc cgtcagatca cacccatctt gccccatgac     780
   taccaggaca gcagcctgcc tgtaggagtc tttgtgtggg atgtggaaaa tgaaggggac     840
   gaagctctag atgtgtccat catgttctcc atgcggaatg gactgggtgg tggagacgat     900
   gccccagggg gtttgtggaa tgagcccttc tgtctggagc gtagcgggga aactgtccgg     960
   gggctgctcc tgcatcatcc aacccttcca aacccctaca cgatggctgt ggctgcacga    1020
   gtcacggcag ctaccacggt aacccacatc acagcctttg accctgacag cacggggcag    1080
   caggtgtggc aggatctact tcaggatgga cagctggact ctcccactgg ccaaagcacc    1140
   cctacgcaga aaggagtagg cattgctgga gctgtgtgtg tttccagcaa gttgcgacct   1200
   cgaggccagt gccgcctgga gttttcactg gcttgggaca tgcccaggat catgtttgga   1260
   gctaaaggcc aagtccacta caggcggtat acaaggttct ttggccagga tggagatgca   1320
   gcacctgccc tcagccacta tgcactgtgc cgatacgcag agtgggaaga gaggatctca   1380
   gcttggcaga gcccggtatt ggatgacaga tcactgcctg cctggtacaa atctgcgctg   1440
   ttcaatgaac tatacttcct ggctgatgga ggcacagtgt ggctggaagt tcttgaggac   1500
   tccctaccag aggagctggg cagaaacatg tgtcacctcc gccccaccct acgggactac   1560
   ggtcgatttg gctaccttga gggccaggag taccgcatgt acaacacata tgatgtccac   1620
   ttttatgctt cctttgccct catcatgctc tggcccaaac ttgagctcag cctacagtat   1680
   gacatggctc tggccactct cagggaggac ctgacacggc gacggtacct gatgagtggg   1740
   gtgatggcac ctgtgaaaag gaggaacgtc atcccccatg atattgggga cccagatgat   1800
   gaaccatggc tccgcgtcaa tgcatattta atccatgata ctgctgattg gaaggacctg   1860
   aacctgaagt ttgtgctgca ggtttatcgg gactattacc tcacgggtga tcaaaacttc   1920
   ctgaaggaca tgtggcctgt gtgtctagct gtgatggaat ctgaaatgaa gtttgacaag   1980
   gaccatgatg gactcattga aaatggaggc tatgcagacc agacctatga tggatgggtg   2040
   accacaggcc ccagtgctta ctgtggaggg ctgtggctgg cagctgtggc tgtgatggtc   2100
   cagatggctg ctctgtgtgg ggcacaggac atccaggata agttttcttc tatcctcagc   2160
   cggggccaag aagcctatga gagactgctg tggaatggcc gctattacaa ctatgacagc   2220
   agctctcggc ctcagtctcg tagtgttatg tctgaccagt gtgctggaca gtggttcctg   2280
   aaggcctgtg gcctaggaga aggagacact gaggtgtttc ctacccaaca tgtggtccgt   2340
   gctctccaaa ctatctttga gctgaacgtc caggcctttg caggaggggc catgggggct   2400
   gtgaatggga tgcagcccca tggtgtccct gataaatcca gtgtgcagtc tgatgaagtc   2460
   tgggtgggtg tggtctacgg gctggcagct accatgatcc aagagggcct gacttgggag   2520
   ggcttccaga cagctgaagg ctgctaccgt accgtgtggg agcgcctggg tctggccttc   2580
   cagaccccag aggcatactg ccagcagcga gtgttccgct cactggccta catgcggcca   2640
   ctgagcatat gggccatgca gctagccctg caacagcagc agcacaaaaa ggcctcctgg   2700
   ccaaaagtca aacagggcac aggactaagg acagggccta tgtttggacc aaaggaagcc   2760
   atggcaaacc tgagcccaga gtga                                          2784
<210> 2
   <211> 927
   <212> PRT
   <213> homo sapiens
<400> 2
<210> 3
   <211> 2757
   <212> DNA
   <213> mus musculus
<400> 3
   atggtaacct gcgtcccggc ctcagagcag gtcggctgtg ccgaaagaga ttcccaagtt     60
   tattgtgaag atactggcgg cactgaggct gtgcgggtta cagactgcgg gagccccgag    120
   gatagtggac cccaggatga accaagctac tgcaattcag aggactctgg gcagctgatg    180
   gcctcctacg agggtaaagc taggggctac caggtgcctc cttttggctg gcggatctgc    240
   ttggctcacg agtttgcaga gaagaggaga ccttttcaag ctaacaacat ctctctcagt    300
   aatttggtaa agcacctcgg tatgggcttg aggtacttga agtggtggta ccgaaagacc    360
   cacgtggaaa agaagacccc tttcatcgac atgctcaatt ctctacccct gagacagatc 420
   tatggttgtc ccctgggtgg cattggagga ggcactatca cccggggctg gagaggccag 480
   ttctgtcgtt ggcagctcaa ccctggaatg taccagcacc agacagtcat tgcagaccaa 540
   tttatagtat gcttgcgtcg agatgggcgg actgtgtacc agcaagttct gtccttggag 600
   cttccaaatg tcctgcgcag ctggaactgg ggcctgtgtg gttactttgc tttctaccac 660
   gccctctatc cccgagcctg gacggtctat cagcttcctg gccagaatgt caccctcacc 720
   tgtcgccagg tcacacctat cttgcctcat gactaccagg acagcagtct ccctgtagga 780
   gtctttgtgt gggatgtaga aaacgaagga gatgagactc tggacgtttc catcacgttc 840
   tccatgcgga atggactagg aggcgaagat gatgcggccg ggagtttgtg gaatgagccc 900
   ttccgcctgg aacagggcgg gacgactgtt caggggctcc tcttgcatca tccaacccct 960
   ccgaacccct acaccatggc tgtggctgca cgatgcacgg cagataccac ggtaacccac 1020
   accacagcct ttgaccccaa tggcactgga cagcaggtgt ggcaggacct acttcaggat 1080
   ggacagctgg actcccctgc tggccaaagc accccaacac agaaaggaga gggtatcgct 1140
   ggggctgtat gcatctctag caagctgtta cctcgaagcc ggtgctgctt ggagttctca 1200
   ctggcttggg atatgcctaa gatcatgttt ggagctaaaa gccaagtcca ctacaggcgg 1260
   tatacacggt tctttggttc agatggtgat gtggcgcctg ccctgagcca ctatgcactg 1320
   tgccactatg cagactggga ggacagaatc tcagcctggc agaacccagt actggatgac 1380
   agaaccttgc ctgcctggta caaatctgca ctgttcaatg aactgtactt cctggccgat 1440
   ggaggcacag tgtggctgga agttcccgca gactccctac cagaggggct gggagggagt 1500
   atgcgtcagc ttcgctctac tctgcaggac tatgggcgat ttggctatct tgaaggccag 1560
   gagtaccgca tgtacaacac atacgatgtc cacttttatg cgtcctttgc cctcgtcatg 1620
   ctgtggccca aacttgagct cagtcttcag tatgatatgg ctctggcaac tttgaaggag 1680
   gacctgaccc ggcgacggta cctgatgagt ggagtggtgg cacctgtgaa aaggaggaac 1740
   gtcatccctc atgacattgg ggatccggat gatgagccat ggctccgggt caacgcatat 1800
   ttgattcatg atactgctga ctggaaggac ctgaacctga agtttgtatt gcaaatttat 1860
   cgggactatt acctgacggg tgatcaaggc ttcctggagg acatgtggcc tgtgtgtctg 1920
   gctgtgatgg agtccgaaat gaagtttgac aaggaccaag atggactcat tgagaatgga 1980
   ggctacgcag accagaccta tgatgcatgg gtcaccacag gccccagtgc ttactgcgga 2040
   gggctgtggc tggcggcagt ggctgtaatg gttcagatgg ctgttctgtg tggggcccaa 2100
   gatgtccagg agaggtttgc ttccattctc tgccgaggcc gagaagctta tgagagactg 2160
   ctgtggaacg gacgctatta caactacgac agcagctccc atcctcagtc tcggagcatc 2220
   atgtctgacc agtgtgctgg gcagtggttc ctgagggcct gcggcctggg agaaggagac 2280
   actgaagtat ttcctaccct gcatgtggtc cgtgctctcc aaaccatctt tgaactcaac   2340
   gtccaagcct ttgcaggagg agccatgggg gccgtgaacg ggatgcaccc tcatggtgtc   2400
   cctgatagat ccagtgtgca gtctgatgaa gtctgggtgg gtgtggtcta tgggctggca   2460
   gccaccatga tccaagaggg cctgacttgg gaaggtttcc ggacagctga aggctgttac   2520
   cgcactgtat gggaacgcct gggcctggct ttccagaccc cagaggcata ctgccagcaa   2580
   caagtgttcc gctccctggc ctacatgcgg ccactgagca tctgggccat gcagctggcc   2640
   ctgcaacagc agcagcataa aaagagccgc aggccatcag tcacacaagg cacgggacta   2700
   agcacacagc ctgaatgtgg accaaagaga tcgctggcaa acctcaattc agagtga      2757
<210> 4
   <211> 918
   <212> PRT
   <213> mus musculus
<400> 4
   Met Val Thr Cys Val Pro Ala Ser Glu Gln Val Gly Cys Ala Glu Arg
   1               5                   10                  15
   Asp Ser Gln Val Tyr Cys Glu Asp Thr Gly Gly Thr Glu Ala Val Arg
               20                  25                  30
   Val Thr Asp Cys Gly Ser Pro Glu Asp Ser Gly Pro Gln Asp Glu Pro
           35                  40                  45
   Ser Tyr Cys Asn Ser Glu Asp Ser Gly Gln Leu Met Ala Ser Tyr Glu
       50                  55                  60
   Gly Lys Ala Arg Gly Tyr Gln Val Pro Pro Phe Gly Trp Arg Ile Cys
   65                  70                  75                  80
   Leu Ala His Glu Phe Ala Glu Lys Arg Arg Pro Phe Gln Ala Asn Asn
                   85                  90                  95
   Ile Ser Leu Ser Asn Leu Val Lys His Leu Gly Met Gly Leu Arg Tyr
               100                 105                 110
   Leu Lys Trp Trp Tyr Arg Lys Thr His Val Glu Lys Lys Thr Pro Phe
           115                 120                 125
   Ile Asp Met Leu Asn Ser Leu Pro Leu Arg Gln Ile Tyr Gly Cys Pro
       130                 135                 140
   Leu Gly Gly Ile Gly Gly Gly Thr Ile Thr Arg Gly Trp Arg Gly Gln
   145                 150                 155                 160
   Phe Cys Arg Trp Gln Leu Asn Pro Gly Met Tyr Gln His Gln Thr Val
   165 170 175
   Ile Ala Asp Gln Phe Ile Val Cys Leu Arg Arg Asp Gly Arg Thr Val
   180 185 190
   Tyr Gln Gln Val Leu Ser Leu Glu Leu Pro Asn Val Leu Arg Ser Trp
   195 200 205
   Asn Trp Gly Leu Cys Gly Tyr Phe Ala Phe Tyr His Ala Leu Tyr Pro
   210 215 220
   Arg Ala Trp Thr Val Tyr Gln Leu Pro Gly Gln Asn Val Thr Leu Thr
   225 230 235 240
   Cys Arg Gln Val Thr Pro Ile Leu Pro His Asp Tyr Gln Asp Ser Ser
   245 250 255
   Leu Pro Val Gly Val Phe Val Trp Asp Val Glu Asn Glu Gly Asp Glu
   260 265 270
   Thr Leu Asp Val Ser Ile Thr Phe Ser Met Arg Asn Gly Leu Gly Gly
   275 280 285
   Glu Asp Asp Ala Ala Gly Ser Leu Trp Asn Glu Pro Phe Arg Leu Glu
   290 295 300
   Gln Gly Gly Thr Thr Val Gln Gly Leu Leu Leu His His Pro Thr Pro
   305 310 315 320
   Pro Asn Pro Tyr Thr Met Ala Val Ala Ala Arg Cys Thr Ala Asp Thr
   325 330 335
   Thr Val Thr His Thr Thr Ala Phe Asp Pro Asn Gly Thr Gly Gln Gln
   340 345 350
   Val Trp Gln Asp Leu Leu Gln Asp Gly Gln Leu Asp Ser Pro Ala Gly
   355 360 365
   Gln Ser Thr Pro Thr Gln Lys Gly Glu Gly Ile Ala Gly Ala Val Cys
   370 375 380
   Ile Ser Ser Lys Leu Leu Pro Arg Ser Arg Cys Cys Leu Glu Phe Ser
   385 390 395 400
   Leu Ala Trp Asp Met Pro Lys Ile Met Phe Gly Ala Lys Ser Gln Val
   405 410 415
   His Tyr Arg Arg Tyr Thr Arg Phe Phe Gly Ser Asp Gly Asp Val Ala
   420 425 430
   Pro Ala Leu Ser His Tyr Ala Leu Cys His Tyr Ala Asp Trp Glu Asp
   435 440 445
   Arg Ile Ser Ala Trp Gln Asn Pro Val Leu Asp Asp Arg Thr Leu Pro
   450 455 460
   Ala Trp Tyr Lys Ser Ala Leu Phe Asn Glu Leu Tyr Phe Leu Ala Asp
   465 470 475 480
   Gly Gly Thr Val Trp Leu Glu Val Pro Ala Asp Ser Leu Pro Glu Gly
   485 490 495
   Leu Gly Gly Ser Met Arg Gln Leu Arg Ser Thr Leu Gln Asp Tyr Gly
   500 505 510
   Arg Phe Gly Tyr Leu Glu Gly Gln Glu Tyr Arg Met Tyr Asn Thr Tyr
   515 520 525
   Asp Val His Phe Tyr Ala Ser Phe Ala Leu Val Met Leu Trp Pro Lys
   530 535 540
   Leu Glu Leu Ser Leu Gln Tyr Asp Met Ala Leu Ala Thr Leu Lys Glu
   545 550 555 560
   Asp Leu Thr Arg Arg Arg Tyr Leu Met Ser Gly Val Val Ala Pro Val
   565 570 575
   Lys Arg Arg Asn Val Ile Pro His Asp Ile Gly Asp Pro Asp Asp Glu
   580 585 590
   Pro Trp Leu Arg Val Asn Ala Tyr Leu Ile His Asp Thr Ala Asp Trp
   595 600 605
   Lys Asp Leu Asn Leu Lys Phe Val Leu Gln Ile Tyr Arg Asp Tyr Tyr
   610 615 620
   Leu Thr Gly Asp Gln Gly Phe Leu Glu Asp Met Trp Pro Val Cys Leu
   625 630 635 640
   Ala Val Met Glu Ser Glu Met Lys Phe Asp Lys Asp Gln Asp Gly Leu
   645 650 655
   Ile Glu Asn Gly Gly Tyr Ala Asp Gln Thr Tyr Asp Ala Trp Val Thr
               660                 665                 670
   Thr Gly Pro Ser Ala Tyr Cys Gly Gly Leu Trp Leu Ala Ala Val Ala
           675                 680                 685
   Val Met Val Gln Met Ala Val Leu Cys Gly Ala Gln Asp Val Gln Glu
       690                 695                 700
   Arg Phe Ala Ser Ile Leu Cys Arg Gly Arg Glu Ala Tyr Glu Arg Leu
   705                 710                 715                 720
   Leu Trp Asn Gly Arg Tyr Tyr Asn Tyr Asp Ser Ser Ser His Pro Gln
                   725                 730                 735
   Ser Arg Ser Ile Met Ser Asp Gln Cys Ala Gly Gln Trp Phe Leu Arg
               740                 745                 750
   Ala Cys Gly Leu Gly Glu Gly Asp Thr Glu Val Phe Pro Thr Leu His
           755                 760                 765
   Val Val Arg Ala Leu Gln Thr Ile Phe Glu Leu Asn Val Gln Ala Phe
       770                 775                 780
   Ala Gly Gly Ala Met Gly Ala Val Asn Gly Met His Pro His Gly Val
   785                 790                 795                 800
   Pro Asp Arg Ser Ser Val Gln Ser Asp Glu Val Trp Val Gly Val Val
                   805                 810                 815
   Tyr Gly Leu Ala Ala Thr Met Ile Gln Glu Gly Leu Thr Trp Glu Gly
               820                 825                 830
   Phe Arg Thr Ala Glu Gly Cys Tyr Arg Thr Val Trp Glu Arg Leu Gly
           835                 840                 845
   Leu Ala Phe Gln Thr Pro Glu Ala Tyr Cys Gln Gln Gln Val Phe Arg
       850                 855                 860
   Ser Leu Ala Tyr Met Arg Pro Leu Ser Ile Trp Ala Met Gln Leu Ala
   865                 870                 875                 880
   Leu Gln Gln Gln Gln His Lys Lys Ser Arg Arg Pro Ser Val Thr Gln
                   885                 890                 895
   Gly Thr Gly Leu Ser Thr Gln Pro Glu Cys Gly Pro Lys Arg Ser Leu
               900                 905                 910
   Ala Asn Leu Asn Ser Glu
   915
<210> 5
   <211> 19
   <212> DNA
   <213> artificial
<220>
   <223> long arm sense primer
<400> 5 19
   caggagcaga gagcaatga 19
<210> 6
   <211> 25
   <212> DNA
   <213> artificial
<220>
   <223> long arm antisense primer
<400> 6
   gcatggaatg aagcaaacag gagga 25
<210> 7
   <211> 21
   <212> DNA
   <213> artificial
<220>
   <223> short arm sense primer
<400> 7
   catctggctc cttagttctc t 21
<210> 8
   <211> 20
   <212> DNA
   <213> artificial
<220>
   <223> short arm antisense primer
<400> 8
   gcgattgtcc tattggctca 20
<210> 9
   <211> 1799
   <212> DNA
   <213> herpes simplex virus
<400> 9
   cagctgcttc atccccgtgg cccgttgctc gcgtttgctg gcggtgtccc cggaagaaat 60
   atatttgcat gtctttagtt ctatgatgac acaaaccccg cccagcgtct tgtcattggc    120
   gaattcgaac acgcagatgc agtcggggcg gcgcggtccg aggtccactt cgcatattaa    180
   ggtgacgcgt gtggcctcga acaccgagcg accctgcagc gacccgctta acagcgtcaa    240
   cagcgtgccg cagatcttgg tggcgtgaaa ctcccgcacc tcttcggcca gcgccttgta    300
   gaagcgcgta tggcttcgta ccccggccat caacacgcgt ctgcgttcga ccaggctgcg    360
   cgttctcgcg gccatagcaa ccgacgtacg gcgttgcgcc ctcgccggca gcaagaagcc    420
   acggaagtcc gcccggagca gaaaatgccc acgctactgc gggtttatat agacggtccc    480
   cacgggatgg ggaaaaccac caccacgcaa ctgctggtgg ccctgggttc gcgcgacgat    540
   atcgtctacg tacccgagcc gatgacttac tggcgggtgc tgggggcttc cgagacaatc    600
   gcgaacatct acaccacaca acaccgcctc gaccagggtg agatatcggc cggggacgcg    660
   gcggtggtaa tgacaagcgc ccagataaca atgggcatgc cttatgccgt gaccgacgcc    720
   gttctggctc ctcatatcgg gggggaggct gggagctcac atgccccgcc cccggccctc    780
   accctcatct tcgaccgcca tcccatcgcc gccctcctgt gctacccggc cgcgcggtac    840
   cttatgggca gcatgacccc ccaggccgtg ctggcgttcg tggccctcat cccgccgacc    900
   ttgcccggca ccaacatcgt gcttggggcc cttccggagg acagacacat cgaccgcctg    960
   gccaaacgcc agcgccccgg cgagcggctg gacctggcta tgctggctgc gattcgccgc   1020
   gtttacgggc tacttgccaa tacggtgcgg tatctgcagt gcggcgggtc gtggcgggag   1080
   gactggggac agctttcggg gacggccgtg ccgccccagg gtgccgagcc ccagagcaac   1140
   gcgggcccac gaccccatat cggggacacg ttatttaccc tgtttcgggc ccccgagttg   1200
   ctggccccca acggcgacct gtataacgtg tttgcctggg ccttggacgt cttggccaaa   1260
   cgcctccgtt ccatgcacgt ctttatcctg gattacgacc aatcgcccgc cggctgccgg   1320
   gacgccctgc tgcaacttac ctccgggatg gtccagaccc acgtcaccac ccccggctcc   1380
   ataccgacga tatgcgacct ggcgcgcacg tttgcccggg agatggggga ggctaactga   1440
   aacacggaag gagacaatac cggaaggaac ccgcgctatg acggcaataa aaagacagaa   1500
   taaaacgcac gggtgttggg tcgtttgttc ataaacgcgg ggttcggtcc cagggctggc   1560
   actctgtcga taccccaccg agaccccatt ggggccaata cgcccgcgtt tcttcctttt   1620
   ccccacccca ccccccaagt tcgggtgaag gcccagggct cgcagccaac gtcggggcgg   1680
   caggccctgc catagccact ggccccgtgg gttagggacg gggtccccca tggggaatgg   1740
   tttatggttc gtgggggtta ttattttggg cgttgcgtgg ggtctggtgg acgacccag    1799
<210> 10
   <211> 6652
   <212> DNA
   <213> artificial
<220>
   <223> plasmid containing ACN cassette
<400> 10
   cacctaaatt gtaagcgtta atattttgtt aaaattcgcg ttaaattttt gttaaatcag     60
   ctcatttttt aaccaatagg ccgaaatcgg caaaatccct tataaatcaa aagaatagac    120
   cgagataggg ttgagtgttg ttccagtttg gaacaagagt ccactattaa agaacgtgga    180
   ctccaacgtc aaagggcgaa aaaccgtcta tcagggcgat ggcccactac gtgaaccatc    240
   accctaatca agttttttgg ggtcgaggtg ccgtaaagca ctaaatcgga accctaaagg    300
   gagcccccga tttagagctt gacggggaaa gccggcgaac gtggcgagaa aggaagggaa    360
   gaaagcgaaa ggagcgggcg ctagggcgct ggcaagtgta gcggtcacgc tgcgcgtaac    420
   caccacaccc gccgcgctta atgcgccgct acagggcgcg tcccattcgc cattcaggct    480
   gcgcaactgt tgggaagggc gatcggtgcg ggcctcttcg ctattacgcc agctggcgaa    540
   agggggatgt gctgcaaggc gattaagttg ggtaacgcca gggttttccc agtcacgacg    600
   ttgtaaaacg acggccagtg aattgtaata cgactcacta tagggcgaat tggagctcga    660
   gatctagata tcgatgaatt cataacttcg tataatgtat gctatacgaa gttatggatc    720
   tgtcgatcga cggatcgatc cgaacaaacg acccaacacc cgtgcgtttt attctgtctt    780
   tttattgccg atcccctcag aagaactcgt caagaaggcg atagaaggcg atgcgctgcg    840
   aatcgggagc ggcgataccg taaagcacga ggaagcggtc agcccattcg ccgccaagct    900
   cttcagcaat atcacgggta gccaacgcta tgtcctgata gcggtccgcc acacccagcc    960
   ggccacagtc gatgaatcca gaaaagcggc cattttccac catgatattc ggcaagcagg   1020
   catcgccatg ggtcacgacg agatcctcgc cgtcgggcat gcgcgccttg agcctggcga   1080
   acagttcggc tggcgcgagc ccctgatgct cttcgtccag atcatcctga tcgacaagac   1140
   cggcttccat ccgagtacgt gctcgctcga tgcgatgttt cgcttggtgg tcgaatgggc   1200
   aggtagccgg atcaagcgta tgcagccgcc gcattgcatc agccatgatg gatactttct   1260
   cggcaggagc aaggtgagat gacaggagat cctgccccgg cacttcgccc aatagcagcc   1320
   agtcccttcc cgcttcagtg acaacgtcga gcacagctgc gcaaggaacg cccgtcgtgg   1380
   ccagccacga tagccgcgct gcctcgtcct gcagttcatt cagggcaccg gacaggtcgg   1440
   tcttgacaaa aagaaccggg cgcccctgcg ctgacagccg gaacacggcg gcatcagagc   1500
   agccgattgt ctgttgtgcc cagtcatagc cgaatagcct ctccacccaa gcggccggag   1560
   aacctgcgtg caatccatct tgttcaatgg ccgatcccat attggctgca cggatcctga   1620
   acggcagagg ttacggcagt ttgtctctcc cccttccggg agccaccttc ttctccaacc   1680
   gtcccggtcg cgctctcggc gcttctgagg agagaactgg ctgagtgacg ccctttatag   1740
   attcgccctt gtgtcccgcc ccttcctttc ccgccctccc ttgcgctacg gggccgcccg 1800
   caccggccta cacggagcgc gcgcggcgga gttgttgacg ctagggctcc ggctccctgg 1860
   ttgggtgttc tttctgacgc gacaggagga ggagaatgtc ctggtcctgt cgtcctcctt 1920
   tcgggtttcc cgtgcactca aaccgaggac ttacagaacg gaggataaag ttaggccatt 1980
   tttactcagc ttcggagttc aggctcattt ttcagctaaa gtctctcatt agtatccccc 2040
   cacacacatc gggaaaatgg tttgtcctac gcatcggtaa tgaaggcggg gcccttcggg 2100
   tcctccggag cgggttccgg gggtgggggg aaggagggag ggacgggacg ggcctcgttc 2160
   atgaatattc agttcaccgc tgaatatgca taaggcaggc aagatggcgc gtccaatcaa 2220
   ttggaagtag ccgttattag tggagaggcc ccaggacgtt ggggcaccgc ctgtgctcta 2280
   gtagctttac ggagccctgg cgctcgatgt tcaagcccaa gctttcgcga gctcgaccga 2340
   acaaacgacc caacacccgt gcgttttatt ctgtcttttt attgccgctc agctttacag 2400
   tgacaatgac ggctggcgac tgaatattag tgcttacaga cagcactaca tattttccgt 2460
   cgatgttgaa atcctttctc atatgtcacc ataaatatca aataattata gcaatcattt 2520
   acgcgttaat ggctaatcgc catcttccag caggcgcacc attgcccctg tttcactatc 2580
   caggttacgg atatagttca tgacaatatt tacattggtc cagccaccag cttgcatgat 2640
   ctccggtatt gaaactccag cgcgggccat atctcgcgcg gctccgacac gggcactgtg 2700
   tccagaccag gccaggtatc tctgaccaga gtcatcctaa aatacacaaa caattagaat 2760
   cagtagttta acacattata cacttaaaaa ttttatattt accttagcgc cgtaaatcaa 2820
   tcgatgagtt gcttcaaaaa tcccttccag ggcgcgagtt gatagctggc tggtggcaga 2880
   tggcgcggca acaccatttt ttctgacccg gcaaaacagg tagttattcg gatcatcagc 2940
   tacaccagag acggaaatcc atcgctcgac cagtttagtt acccccaggc taagtgcctt 3000
   ctctacacct gcggtgctaa ccagcgtttt cgttctgcca atatggatta acattctccc 3060
   accgtcagta cgtgagatat ctttaaccct gatcctggca atttcggcta tacgtaacag 3120
   ggtgttataa gcaatcccca gaaatgccag attacgtata tcctggcagc gatcgctatt 3180
   ttccatgagt gaacgaacct ggtcgaaatc agtgcgttcg aacgctagag cctgttttgc 3240
   acgttcaccg gcatcaacgt tttcttttcg gatccgccgc ataaccagtg aaacagcatt 3300
   gctgtcactt ggtcgtggca gcccggaccg acgatgaagc atgtttagct ggcccaaatg 3360
   ttgctggata gtttttactg ccagaccgcg cgcctgaaga tatagaagat aatcgcgaac 3420
   atcttcaggt tctgcgggaa accatttccg gttattcaac ttgcaccatg ccgcccacga 3480
   ccggcaaacg gacagaagca ttttccaggt atgctcagaa aacgcctggc gatccctgaa 3540
   catgtccatc aggttcttgc gaacctcatc actcgttgca tcgaccggta atgcaggcaa 3600
   attttggtgt acggtcagta aattggacac cttcctcttc ttcttgggca tggccgcagg 3660
   aaagcagagc cctgaagctc ccatcaccgg ccaataagag ccaagcctgc agtgtgacct 3720
   catagagcaa tgtgccagcc agcctgaccc caagggccct caggcttggg cacactgtct 3780
   ctaggaccct gagagaaaga catacccatt tctgcttagg gccctgagga tgagcccagg 3840
   ggtggcttgg cactgaagca aaggacactg gggctcagct ggcagcaaag tgaccaggat 3900
   gctgaggctt tgacccagaa gccagaggcc agaggccagg acttctcttg gtcccagtcc 3960
   accctcactc agagctttac caatgccctc tggatagttg tcgggtaacg gtggacgcca 4020
   ctgattctct ggccagccta ggacttcgcc attccgctga ttctgctctt ccagccactg 4080
   gctgaccggt tggaagtact ccagcagtgc cttggcatcc agggcatctg agcctaccag 4140
   gtccttcagt acctcctgcc agggcctgga gcagccagcc tgcaacacct gcctgccaag 4200
   cagagtgacc actgtgggca caggggacac agggtggggc ccacaacagc accattgtcc 4260
   acttgtccct cactagtaaa agaactctag ggttgcgggg ggtgggggag gtctctgtga 4320
   ggctggtaag ggatatttgc ctggcccatg gagatccata acttcgtata atgtatgcta 4380
   tacgaagtta taagctttcg cgagctcgag atctagatat cgataccgtc gacctcgagg 4440
   gggggcccgg tacccagctt ttgttccctt tagtgagggt taatttcgag cttggcgtaa 4500
   tcatggtcat agctgtttcc tgtgtgaaat tgttatccgc tcacaattcc acacaacata 4560
   cgagccggaa gcataaagtg taaagcctgg ggtgcctaat gagtgagcta actcacatta 4620
   attgcgttgc gctcactgcc cgctttccag tcgggaaacc tgtcgtgcca gctgcattaa 4680
   tgaatcggcc aacgcgcggg gagaggcggt ttgcgtattg ggcgctcttc cgcttcctcg 4740
   ctcactgact cgctgcgctc ggtcgttcgg ctgcggcgag cggtatcagc tcactcaaag 4800
   gcggtaatac ggttatccac agaatcaggg gataacgcag gaaagaacat gtgagcaaaa 4860
   ggccagcaaa aggccaggaa ccgtaaaaag gccgcgttgc tggcgttttt ccataggctc 4920
   cgcccccctg acgagcatca caaaaatcga cgctcaagtc agaggtggcg aaacccgaca 4980
   ggactataaa gataccaggc gtttccccct ggaagctccc tcgtgcgctc tcctgttccg 5040
   accctgccgc ttaccggata cctgtccgcc tttctccctt cgggaagcgt ggcgctttct 5100
   catagctcac gctgtaggta tctcagttcg gtgtaggtcg ttcgctccaa gctgggctgt 5160
   gtgcacgaac cccccgttca gcccgaccgc tgcgccttat ccggtaacta tcgtcttgag 5220
   tccaacccgg taagacacga cttatcgcca ctggcagcag ccactggtaa caggattagc 5280
   agagcgaggt atgtaggcgg tgctacagag ttcttgaagt ggtggcctaa ctacggctac 5340
   actagaagga cagtatttgg tatctgcgct ctgctgaagc cagttacctt cggaaaaaga 5400
   gttggtagct cttgatccgg caaacaaacc accgctggta gcggtggttt ttttgtttgc 5460
   aagcagcaga ttacgcgcag aaaaaaagga tctcaagaag atcctttgat cttttctacg 5520
   gggtctgacg ctcagtggaa cgaaaactca cgttaaggga ttttggtcat gagattatca 5580
   aaaaggatct tcacctagat ccttttaaat taaaaatgaa gttttaaatc aatctaaagt 5640
   atatatgagt aaacttggtc tgacagttac caatgcttaa tcagtgaggc acctatctca 5700
   gcgatctgtc tatttcgttc atccatagtt gcctgactcc ccgtcgtgta gataactacg 5760
   atacgggagg gcttaccatc tggccccagt gctgcaatga taccgcgaga cccacgctca 5820
   ccggctccag atttatcagc aataaaccag ccagccggaa gggccgagcg cagaagtggt 5880
   cctgcaactt tatccgcctc catccagtct attaattgtt gccgggaagc tagagtaagt 5940
   agttcgccag ttaatagttt gcgcaacgtt gttgccattg ctacaggcat cgtggtgtca 6000
   cgctcgtcgt ttggtatggc ttcattcagc tccggttccc aacgatcaag gcgagttaca 6060
   tgatccccca tgttgtgcaa aaaagcggtt agctccttcg gtcctccgat cgttgtcaga 6120
   agtaagttgg ccgcagtgtt atcactcatg gttatggcag cactgcataa ttctcttact 6180
   gtcatgccat ccgtaagatg cttttctgtg actggtgagt actcaaccaa gtcattctga 6240
   gaatagtgta tgcggcgacc gagttgctct tgcccggcgt caatacggga taataccgcg 6300
   ccacatagca gaactttaaa agtgctcatc attggaaaac gttcttcggg gcgaaaactc 6360
   tcaaggatct taccgctgtt gagatccagt tcgatgtaac ccactcgtgc acccaactga 6420
   tcttcagcat cttttacttt caccagcgtt tctgggtgag caaaaacagg aaggcaaaat 6480
   gccgcaaaaa agggaataag ggcgacacgg aaatgttgaa tactcatact cttccttttt 6540
   caatattatt gaagcattta tcagggttat tgtctcatga gcggatacat atttgaatgt 6600
   atttagaaaa ataaacaaat aggggttccg cgcacatttc cccgaaaagt gc 6652
<210> 11
   <211> 2961
   <212> DNA
   <213> artificial
<220>
   <223> pBluescript II KS(+)
<400> 11
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tggagctcca 660
   ccgcggtggc ggccgctcta gaactagtgg atcccccggg ctgcaggaat tcgatatcaa 720
   gcttatcgat accgtcgacc tcgagggggg gcccggtacc cagcttttgt tccctttagt 780
   gagggttaat tgcgcgcttg gcgtaatcat ggtcatagct gtttcctgtg tgaaattgtt 840
   atccgctcac aattccacac aacatacgag ccggaagcat aaagtgtaaa gcctggggtg 900
   cctaatgagt gagctaactc acattaattg cgttgcgctc actgcccgct ttccagtcgg 960
   gaaacctgtc gtgccagctg cattaatgaa tcggccaacg cgcggggaga ggcggtttgc 1020
   gtattgggcg ctcttccgct tcctcgctca ctgactcgct gcgctcggtc gttcggctgc 1080
   ggcgagcggt atcagctcac tcaaaggcgg taatacggtt atccacagaa tcaggggata 1140
   acgcaggaaa gaacatgtga gcaaaaggcc agcaaaaggc caggaaccgt aaaaaggccg 1200
   cgttgctggc gtttttccat aggctccgcc cccctgacga gcatcacaaa aatcgacgct 1260
   caagtcagag gtggcgaaac ccgacaggac tataaagata ccaggcgttt ccccctggaa 1320
   gctccctcgt gcgctctcct gttccgaccc tgccgcttac cggatacctg tccgcctttc 1380
   tcccttcggg aagcgtggcg ctttctcata gctcacgctg taggtatctc agttcggtgt 1440
   aggtcgttcg ctccaagctg ggctgtgtgc acgaaccccc cgttcagccc gaccgctgcg 1500
   ccttatccgg taactatcgt cttgagtcca acccggtaag acacgactta tcgccactgg 1560
   cagcagccac tggtaacagg attagcagag cgaggtatgt aggcggtgct acagagttct 1620
   tgaagtggtg gcctaactac ggctacacta gaaggacagt atttggtatc tgcgctctgc 1680
   tgaagccagt taccttcgga aaaagagttg gtagctcttg atccggcaaa caaaccaccg 1740
   ctggtagcgg tggttttttt gtttgcaagc agcagattac gcgcagaaaa aaaggatctc 1800
   aagaagatcc tttgatcttt tctacggggt ctgacgctca gtggaacgaa aactcacgtt 1860
   aagggatttt ggtcatgaga ttatcaaaaa ggatcttcac ctagatcctt ttaaattaaa 1920
   aatgaagttt taaatcaatc taaagtatat atgagtaaac ttggtctgac agttaccaat 1980
   gcttaatcag tgaggcacct atctcagcga tctgtctatt tcgttcatcc atagttgcct 2040
   gactccccgt cgtgtagata actacgatac gggagggctt accatctggc cccagtgctg 2100
   caatgatacc gcgagaccca cgctcaccgg ctccagattt atcagcaata aaccagccag 2160
   ccggaagggc cgagcgcaga agtggtcctg caactttatc cgcctccatc cagtctatta 2220
   attgttgccg ggaagctaga gtaagtagtt cgccagttaa tagtttgcgc aacgttgttg 2280
   ccattgctac aggcatcgtg gtgtcacgct cgtcgtttgg tatggcttca ttcagctccg 2340
   gttcccaacg atcaaggcga gttacatgat cccccatgtt gtgcaaaaaa gcggttagct   2400
   ccttcggtcc tccgatcgtt gtcagaagta agttggccgc agtgttatca ctcatggtta   2460
   tggcagcact gcataattct cttactgtca tgccatccgt aagatgcttt tctgtgactg   2520
   gtgagtactc aaccaagtca ttctgagaat agtgtatgcg gcgaccgagt tgctcttgcc   2580
   cggcgtcaat acgggataat accgcgccac atagcagaac tttaaaagtg ctcatcattg   2640
   gaaaacgttc ttcggggcga aaactctcaa ggatcttacc gctgttgaga tccagttcga   2700
   tgtaacccac tcgtgcaccc aactgatctt cagcatcttt tactttcacc agcgtttctg   2760
   ggtgagcaaa aacaggaagg caaaatgccg caaaaaaggg aataagggcg acacggaaat   2820
   gttgaatact catactcttc ctttttcaat attattgaag catttatcag ggttattgtc   2880
   tcatgagcgg atacatattt gaatgtattt agaaaaataa acaaataggg gttccgcgca   2940
   catttccccg aaaagtgcca c                                             2961
<210> 12
   <211> 21
   <212> DNA
   <213> artificial
<220>
   <223> genotyping primer 1
<400> 12
   gtgctggctc actgagctgg a 21
<210> 13
   <211> 24
   <212> DNA
   <213> artificial
<220>
   <223> genotyping primer 2
<400> 13
   ccagtcagca gtatcatgaa tcaa 24
<210> 14
   <211> 25
   <212> DNA
   <213> artificial
<220>
   <223> genotyping primer 3
<400> 14
   gcatgtacaa cacatacgat gtcca 25
<210> 15
   <211> 25
   <212> DNA
   <213> artificial
<220>
   <223> RT-PCR detection primer 1
<400> 15
   ggagaccttt tcaagctaac aacat 25
<210> 16
   <211> 25
   <212> DNA
   <213> artificial
<220>
   <223> RT-PCR detection primer 2
<400> 16
   ggtaccacca cttcaagtac ctcaa 25
<210> 17
   <211> 11613
   <212> DNA
   <213> mus musculus
<400> 17
   ggcgactttg ttgctgtagc cgctgctgca gcagcaggag ctctctgact ttacccagat 60
   accaacgtcc cacggctttc cccagcatcg ggccctcccg gactgcacct aactgcaact 120
   ggcatcagcg ttttgaaacg tcagaaacct gtcagaggga accgaggcca ccggggtcgt 180
   gaggacccag gatccagtga acatggtaac ctgcgtcccg gcctcagagc aggtcggctg 240
   tgccgaaaga gattcccaag tttattgtga agatactggc ggcactgagg ctgtgcgggt 300
   tacagactgc gggagccccg aggatagtgg accccaggat gaaccaagct actgcaattc 360
   agaggactct gggcagctga tggcctccta cgagggtaaa gctaggggct accaggtgcc 420
   tccttttggc tggcggatct gcttggctca cgagtttgca gagaagagga gaccttttca 480
   agctaacaac atctctctca gtaatttggt aaagcacctc ggtatgggct tgaggtgagt 540
   ggattccgtt gcctagaacg ccgtggttcc tatgatccca ttcaggctcc ctgcctaggt 600
   tggaaaggtg ggcaagctgc tgggctccta gttctattcc agccaggtat accatatccc 660
   ctgcattctt tagccctgca gtttttgagg gtggaactga agaacatttt gtttttcctt 720
   catccctctt ttcctagcat cttgtcttct cggagcattt agtcctgaag tcagtctttc 780
   ctatttaact gctggtcccc cttcccccca cccccacccc caccatgcac ttcctctttc 840
   ccaggcgtcc ctttgagatt agctataatt tgtaacacgg taggagaacg ccagtctctg 900
   ctgtgcttga tacagtgttt tattttttgg aagaggaggt tctttaggct gcattaccct 960
   ggcagctgac accttctgtg taccttattt aagcgcatgc ttcttcagtc ccagaattct 1020
   tatgtctcta tcctcaagct ctgattcctg gaatagttga tcttggctgt tgtcaaccct 1080
   aaagagaggg tgagaagttc actctgttct gtgacagatg gcttccatac caaaaataaa 1140
   agcgaagttg ggattgggca gagtcctaga gtgccaccta cttgggaggc tgagacagga 1200
   ggataatttg agcctaagag ttcagggcat acttgaacat cacagtgaaa gcatcctccg 1260
   gtaacaagtg gggagagcta gggatgaagc ccggaagtag agagcatgcc ttctaccacg 1320
   gagatgcctc cccagacctc ttcagatgac tttagccagc agtgctttgc tggtcttcca 1380
   gcgtttcctc atatcccaga ggttacaaac actgcagggc aggttctaca gaagcttctg 1440
   gattccccac tgagttcaag gactagtgat gaaggagcat tccgatgaac aggaacaggc 1500
   agacagacag acagagagca tcattctgag tcacttcaca tataaacaca gcactgggga 1560
   gggaagcata aaagtcacca aactcagccc ccaaagcttc taaagtaagg gtcccttctc 1620
   tgccgcggtc ggtgccgtgg agctgtggtg gggaggccac ttacctaagc ctagccctag 1680
   gcagtgaaga gggcctggca ctgcctggac atttggctcc tgcgggtggc ccatctcaga 1740
   gagtcgtccc tccaagtaag ttaggaagct agtggtcaaa gcagacaaaa gtaacttcag 1800
   gaagagtttg tttgaaggta cgacccgtct gtctgtcatg gcagagaagt gatggtggca 1860
   gtcacacggt atcgcagcca ggagcagaga gcaatgaagg ctagaactcg accctctttc 1920
   tcctttctat catgactcca ggaccccaac ccatggaatg tagtcaccca cagttaaggc 1980
   gcatcttccc atttccatta acctaaagta ttcattcctc acaggcacgt ccataggctt 2040
   ctctctttgg tggttctaga tctggccaag ttgtcagtag gaattaacaa tcatgggctg 2100
   gaaagatggc ttagtggtta agagcactga ctgctcttcc agaggtcctg agttcaaatc 2160
   tcagcaacca catggtggct cacaaccatc tgtaatgaga tctgatgtcc tcttctggtg 2220
   tgtctggaga tagcgactat gttacattaa ataaatagaa cttaaaaaga gagagagaga 2280
   gaacaatcat attgttccag ccaaattcta accccaaatc ctttgtgtca aaatgggatc 2340
   caccgtactg agataggcac atgtggttag gataaggtca agaacaggta tgcattcatc 2400
   ctggcctctt agcctgctaa gtaggagctg agttacagag gcttcaagct cttgctcagc 2460
   cccttagacc ttgtccacac agagctttgg gaatgggcta aggtcctaca cggaggagag 2520
   agcagagggg gtgattcaga acggtcgttt ttaagctgga attgatacta ttgaaaaaaa 2580
   ctgaatatct caggaatgac cagcaaccaa actaaaggag ggaacccttc agagttcaac 2640
   tgaggggtag cctcataggg gtgctggcag gatagttatt agactttcaa cttagagttc 2700
   aattttgatt gttcttcctt ttgccagagt cagctcactt aataaatgtt tactaccaga 2760
   gcggcctttg ttctgcccat ttttgtatct tgtaattcgt ctctgatcct tgctggcttc 2820
   ccattgctgt cctatttcct gtaaactggc agagggcctt cagccttgct cccaccccat 2880
   ccttagtctc ccatgccttt tgccccaccc caggacatgc cagtcctatt ctttttagtg 2940
   tatttccctt ctcaactccg gatccactct gtcttccatt gctctttcta tgtctactgt 3000
   ttgtacatcc attcatatgt aaattcacca tcaagtatta tgggatgcag gccctgtact 3060
   tagagctgtg gggaacatcc acctagtctt ggtcattgtt ccttaaccca aggaacttaa 3120
   aaatttgtca tgctcatgtg aggaacacac tggcataaca aggcagtatc taattaggag 3180
   ctaagacact gtgtagcaat agtcatacct ggcacagata ttccacttta tggctttctt 3240
   atctgttagg ttatttgatc tttccatgat tatatgaggt agctagggca gaagttatcc 3300
   ctattccgca caggaggaaa caaaagcttg gctgacataa ctaattactt gtttagactg 3360
   aatgttgagc atgaaatgat tggttaagga gaagagtgga agtgggaaag acgggtgggc 3420
   gggactcgct tagtgaccca tcctgaatga gagtgggtaa ggaataagat tagtaggcag 3480
   gctgaggcag atgtcctagc cgtgagaata cagacatcta ctcctatgtg accgacagcc 3540
   ctgatcattt tcccgttggc tacagagcaa ggcaagaaca gggaaacact gagacttgtt 3600
   agaaagcttt actattgtga gtattctgac tttgttttgt tgagaaaggt tctcactttg 3660
   acgccccggt tgtctcggaa ctcattacat agcccaggct agcctcaacc ccaagtgttg 3720
   gtttgacaga tatgagccat catgaccaag ttaatttact gggtattttg gaagaacaaa 3780
   atctggaaac tgaaaattaa atgctaaagt ggggatgaag cttaagagga aaaggtcccg 3840
   aatatgtgca catcagttat cttcagctaa ggtggcttcc tttctacctg acatattttc 3900
   caatttttca ccttccataa atgaaatcgt ttttaaaaga gggagtcttt ttggcagcct 3960
   ctgcttggat gtgtggagga gggtgtagct agcccgggaa ggcaactgac cttaacacag 4020
   acaaaagctc ctgaggtttg agctaaagcg gtggtagtgg agttagaaga atcgcttctc 4080
   tctcagaata gagaaataag ggcagcctac actggtacct gggcagctgg gaagataagg 4140
   gaccttgaac tggagcagtc agaggaagat ggcttgggag atactagtga tgggtcggcc 4200
   ctagggtgtt gtcttagaca gtctcaccat atagcccatg ctggcctcaa acttgtaatg 4260
   cctctgcctc agctttcaga atgctaagat tttaggaatg ttcatggaat ccagcatcct 4320
   tgggacactg agttggagat cacacagagg tgacaaagga aaggacggtg agtgtagctt 4380
   acaagaaaca agccactcac cttacactca cacagacact tgctcctcag gtacttgaag 4440
   tggtggtacc gaaagaccca cgtggaaaag aagacccctt tcatcgacat gctcaattct 4500
   ctacccctga gacagatcta tggtgagtct cagctcagct gcccttttca aagagggtcc 4560
   aacttctgtg cttcaggcaa aaaggctcct agtttatgca tggctccatt ccctaagttt 4620
   ttaaagatgt gggcttgctt tcttcttcca gttctacacc agccatcctg actttctttc 4680
   taggttgtcc cctgggtggc attggaggag gcactatcac ccggggctgg agaggccagt 4740
   tctgtcgttg gcagctcaac cctggaatgt accagcacca gacagtcatt gcagaccaag 4800
   taaggaaggg ggttgggcag gcagaggagt gactcttggc tagcatagga tgtggcagtt 4860
   aggcatccac atggtccagt tcagtaaatg ctttcagaat gcgtgtgccc cgctcagtac 4920
   taggatggac agttggttaa agatgaacac agaacccatg ttccttgaaa gaataaagaa 4980
   acagcctgat aaatctcatg tactaatctt ctgagcacta gctcagtcag tacccaccaa 5040
   agactagaaa tttctggatc atttaaactg gctaaaagaa catttcatcc tgcatggagt 5100
   ggtacatcct tataatccta atttttggga ggcagatgca aaagaatcag gagttcaagg 5160
   ctagcctggg ctacttaaga caaaacaaaa tggtgggcag taagatggct caacaggtaa 5220
   agccacacag gtttgacaac ctgcattcca tccctggacc tgcagggcga gaggaagaga 5280
   accaggccct acaaactgtc tttgaagctt catgcataac tttactgttc acggtggctt 5340
   ctgcatgctg tgggatcggt gtaaagatca gaagacagcc tgtaggaagg agtgggctct 5400
   caccttctac catgtgggct tgggagatta aactcaggtt gtgaggcttg gtgacaagtg 5460
   ccttcactgg cttcagccat cttggctggc cctcaatttt ttttttttaa agaatattcg 5520
   aaaagactga aagagaaaaa gtgaaaccta aaaagccatg gtgggaaaag gcgcacagtg 5580
   ctaagtctcc ttccctagat tagtaacgct ttagcctagg ttagcgccct tgccgcagac 5640
   ctctgatggt cttcccagga ccccctttaa ctcttctctc tagccttcct cgctcatatc 5700
   tgggccttaa ttgtcagtgt atgccctgcc cttcaccaag gctctcaaat gacctcccct 5760
   tctgagttag caagtccccc tttcccgagc ttccagctta cctccaaagg tgagataact 5820
   ggcagatctc aaactcacca atgacagtat ccttctcaat tcaagtttac tggctgtgct 5880
   caatatccag tctctaccct aggcccatgt ctcatctctt ccgcattgca agccaagttt 5940
   agaaacatgg gtctgaagta actattgcct gtgtactgtg ttaatacact gtggaaggct 6000
   ttggggatat ttatattttt aaaaatccta caaagtatca ttatcatcat tttacagatg 6060
   aggagacagg atccaggggg cattagtaac ctgctcaaag cagcatggta gccagtgctc 6120
   tctatgacaa ttgttggtgg tcctaaccat tggtacttta ccagcaacat ggaaaattac 6180
   tttatggcta cgcccacatc tgtaatctcc aggactgagg cagaaggatc gccagttcaa 6240
   ggccagcctg agctacaaag caagatccta tctcaaaaga aatagaaggg aaaggaaact 6300
   ttatatcaat aacaacacgg agcgtagttt ggagtaacgg aggagattaa gccctcagca 6360
   tttctcctcc ccggagccta aggttatact ggagaagtaa gacttgggga caatggagac 6420
   tgccaccatg aggtgggccc acagtagact gaagcagcag accatactac agagactttg 6480
   cacacatgcc cactctatga ggtccacttc agtaatctag aaatgacccc tgccctcaga 6540
   gtgagcagtg ggaaaattgg agacacagga gagcaaatga aaacactaca acaataattt 6600
   accaaatcct gaagaggaat aggatgtaac agaaaggtcc ctgtgggaga ggaggaggaa 6660
   gaggaagtac ctgacctagg ccctgaagga tgaagggatc tggctggatg gagatgggca 6720
   gactcgtgac gaggaagtga ggggtgctca cagactggag agagtcttgt atttgtgtga 6780
   ccaggctaga gtttagcatt cagcctgaat gctataggag tcactaaaac acgctaggca 6840
   ggggttggca catactgatt tgttacatca ttttctgcta ccaagctaag aggcagagat 6900
   agcataagcc tgattaatta aggcactgag atcaggcatg aagggaggag gtctaggagc 6960
   tggctgcacg aggaggaagt aggtcaggtg ctggctcact gagctggaag gcaggcctgg 7020
   agacaaccta aggctggtgg tggtgacagt gacagggctg caaaggagta ctttgcggcc 7080
   ggggaccaag tgtgtgttgc ctgtgggtta cttattccag cctgtctgct ctcttgatca 7140
   agtttatagt atgcttgcgt cgagatgggc ggactgtgta ccagcaagtt ctgtccttgg 7200
   agcttccaaa tgtcctgcgc agctggaact ggggcctgtg tggttacttt gctttctacc 7260
   acgccctcta tccccgagcc tggacggtct atcagcttcc tggccagaat gtcaccctca 7320
   cctgtcgcca ggtcacacct atcttgcctc atgactacca ggtgaggatt cctcctgttt 7380
   gcttcattcc atgccctccc ccagatccac tgctgctaga aattgtgtaa cctatgaaag 7440
   ggagaggctg gagctgtgaa catcacactg cttccgagac taagtcgtcc tagctgtcta 7500
   gacctcctgg gaggccggtt tcctgggaac atggagatgc acagaagaca gaagggaatg 7560
   tagtggggac aatttgagga ggtactctct ggccactcct aaggatacac tgagactgag 7620
   tcccatctcc catcccagga cagcagtctc cctgtaggag tctttgtgtg ggatgtagaa 7680
   aacgaaggag atgagactct ggacgtttcc atcacgttct ccatgcggaa tggactagga 7740
   ggcgaagatg atgcggccgg gagtttgtgg aatgagccct tccgcctgga acagggcggg 7800
   acgactgttc aggggctcct cttgcatcat ccaacccctc cgaaccccta caccatggct 7860
   gtggctgcac gatgcacggt gagggacaag tctgctctgt acctctcctc gggagcccct 7920
   gccctcatga cctgacccga acctacttta cctctaacgg tcttccattt cgtgaactcc 7980
   catcctcatc acagccctca gcctctagaa cttggaggtg acagtgttta aggagccgaa 8040
   gagctagtca gaatccagtg tgcttggact tgcctttaca gagtctctgg cttcttaaca 8100
   ggcagatacc acggtaaccc acaccacagc ctttgacccc aatggcactg gacagcaggt 8160
   gtggcaggac ctacttcagg atggacagct ggactcccct gctggtgatg gggggtttga 8220
   ctgttgggaa gaaaggctct gtcctgtctt aggaccaggg gtggaagacc tgataatgaa 8280
   tgggccttgt cttcaaatta gaagtccctg gctttggggt tcaagggggt gcctgtgctg 8340
   attcctctgt gctccttagg ccaaagcacc ccaacacaga aaggagaggg tatcgctggg 8400
   gctgtatgcg tctctagcaa gctgttacct cgaagccggt gctgcttgga gttctcactg 8460
   gcttgggata tgcctaagat catgtttgga gctaaaagcc aagtccacta caggtaaatg 8520
   cagggatcca aagaccctgt ggcaggggcg tctagcctag agcatctgtt cttatgcctt 8580
   tctttccatc ccaggcggta tacacggttc tttggttcag atggtgatgt ggcgcctgcc 8640
   ctgagccact atgcactgtg ccactatgca gactgggagg acagaatctc agcctggcag 8700
   aacccagtac tggatgacag gtgccctctt gtcgtttttt cgcaggcact cactccaaat 8760
   atgacttgct ctgcttccct agcagtctgt cgggctgcgc tgggattcac agtgtcccta 8820
   cttcactcta gaatcccctc tatcctggga ggctcataaa gctctatccc ctcctcttgg 8880
   cttcccagaa ccttgcctgc ctggtacaaa tctgcactgt tcaatgaact gtacttcctg 8940
   gccgatggag gcacagtgtg gctggaagtt cccgcagact ccctaccaga ggggctggga 9000
   gggagtatgc gtcagcttcg ctctactctg caggactatg ggcgatttgg ctatcttgaa 9060
   ggtatggcgt gccagtggac tggcatgggt cagagctagg tgtgtcctac ataatgcagg 9120
   atgacagacg ttgcctgtcc tgagatactg acaggataag gggtgaattg ggaaaacgga 9180
   aagatgagtg ggcctacttc cagctttgaa gagctaccga gcagaagccg gcattctggc 9240
   aacagtgtct ctctcttcct ccaggccagg agtaccgcat gtacaacaca tacgatgtcc 9300
   acttttatgc gtcctttgcc ctcgtcatgc tgtggcccaa acttgagctc agtcttcagt 9360
   atgatatggg taacgtctct gctttcccta ctcctgagct cttattgtct ctcagataac 9420
   atgtcctccc cttcctcctg agcatgcccg catctggctc cttagttctc tggggtcaca 9480
   cacacagcac tggatcccac cccaagcctg cccattccct gctttccctc acgtacttgt 9540
   gtctccgctc ccccagctct ggcaactttg aaggaggacc tgacccggcg acggtacctg 9600
   atgagtggag tggtggcacc tgtgaaaagg aggaacgtca tccctcatga cattggggat 9660
   ccgggtgaga ttccgtctgt cagtctctat acctgctatc ctgtccactg acacactgaa 9720
   ccaggtcccc tcccctccaa cagatgatga gccatggctc cgggtcaacg catatttgat 9780
   tcatgatact gctgactgga aggacctgaa cctgaagttt gtattgcaaa tttatcggga 9840
   ctattacctg acgggtgatc aaggcttcct ggaggacatg tggcctgtgt gtctggtaag 9900
   gaacgagaac tgtgtccagt ataccaggga tgtagctgga gtcttgggag aacccagact 9960
   ttctttctta gaacctaggt cttcagtgtc ttgatccctc tctaggctgt gatggagtcc 10020
   gaaatgaagt ttgacaagga ccaagatgga ctcattgaga atggaggcta cgcagaccag 10080
   acctatgatg catgggtcac cacaggcccc aggtttgggg gtagggattt ccaggtctga 10140
   ggtgaagacc cgggcatgtg aggatgccgt gtagagcggc cggtctcact ccagccactg 10200
   ttcccaccca gtgcttactg cggagggctg tggctggcgg cagtggctgt aatggttcag 10260
   atggctgttc tgtgtggggc ccaagatgtc caggagaggt ttgcttccat tctctgccga 10320
   ggccgagaag cttatgagag actgctgtgg aacggtgagt aaaagcaacc tggggcagct 10380
   taaaacccaa ctacgggata caagcaactt gactttgctc tttctctatt ccaggacgct 10440
   attacaacta cgacagcagc tcccatcctc agtctcggag catcatgtct gaccagtgtg 10500
   ctgggcagtg gttcctgagg gcctgcggcc tgggagaagg agacactgaa gtgagaggct 10560
   ggagaagcca gaagtctggg tttccctgga ggtgggaggc aagtctgaga ctccaccttt 10620
   gctctccctc caggtatttc ctaccctgca tgtggtccgt gctctccaaa ccatctttga 10680
   actcaacgtc caagcctttg caggaggagc catgggggcc gtgaacggga tgcaccctca 10740
   tggtgtccct gatagatcca gtgtgcagtc tgatgaagtc tgggtgggtg tggtctatgg 10800
   gctggcagcc accatgatcc aagaggtaat gaattctctt cccagccctt caccatttgt 10860
   actgaggtcc agaaaacttc ctcagccact aactttattc caagaacacc ctactgcctg 10920
   ccatcttatc cgtccacaga gcgcccttct ccctaataag tgttccacct cttgccttta 10980
   agttcttgcc tttagggcga acggcctcct gtcttctctg ctcagctaag gtaattgtgc 11040
   caagagtcag ttattatgtg tgacttctac gtctccccac agggcctgac ttgggaaggt 11100
   ttccggacag ctgaaggctg ttaccgcact gtatgggaac gcctgggcct ggctttccag 11160
   accccagagg catactgcca gcaacaagtg ttccgctccc tggcctacat gcggccactg 11220
   agcatctggg ccatgcagct ggccctgcaa cagcagcagc ataaaaagag ccgcaggcca 11280
   tcagtcacac aaggcacggg actaagcaca cagcctgaat gtggaccaaa gagatcgctg 11340
   gcaaacctca attcagagtg atccctctga actgtggagg cccagcctcc acactggacc 11400
   tcctccttcc tcccacaagt cctgcagccc tgagccaata ggacaatcgc gcccctcttc 11460
   cttcccagct gtgccagtaa aaaggggctg aggaaccact tatttatttc ttaccctacc 11520
   cagtccttcc atggatgaag tattcaaggc cagtaaatgt ctccaaatct attcacgggg 11580
   caacagatac atatgggtat aaataaaata ctc 11613
<210> 18
   <211> 17790
   <212> DNA
   <213> Artificial
<220>
   <223> Gba2 knock-out vector
<400> 18
   gaattccagg agcagagagc aatgaaggct agaactcgac cctctttctc ctttctatca 60
   tgactccagg accccaaccc atggaatgta gtcacccaca gttaaggcgc atcttcccat 120
   ttccattaac ctaaagtatt cattcctcac aggcacgtcc ataggcttct ctctttggtg 180
   gttctagatc tggccaagtt gtcagtagga attaacaatc atgggctgga aagatggctt 240
   agtggttaag agcactgact gctcttccag aggtcctgag ttcaaatctc agcaaccaca 300
   tggtggctca caaccatctg taatgagatc tgatgtcctc ttctggtgtg tctggagata 360
   gcgactatgt tacattaaat aaatagaact taaaaagaga gagagagaga acaatcatat 420
   tgttccagcc aaattctaac cccaaatcct ttgtgtcaaa atgggatcca ccgtactgag 480
   ataggcacat gtggttagga taaggtcaag aacaggtatg cattcatcct ggcctcttag    540
   cctgctaagt aggagctgag ttacagaggc ttcaagctct tgctcagccc cttagacctt    600
   gtccacacag agctttggga atgggctaag gtcctacacg gaggagagag cagagggggt     660
   gattcagaac ggtcgttttt aagctggaat tgatactatt gaaaaaaact gaatatctca    720
   ggaatgacca gcaaccaaac taaaggaggg aacccttcag agttcaactg aggggtagcc    780
   tcataggggt gctggcagga tagttattag actttcaact tagagttcaa ttttgattgt    840
   tcttcctttt gccagagtca gctcacttaa taaatgttta ctaccagagc ggcctttgtt    900
   ctgcccattt ttgtatcttg taattcgtct ctgatccttg ctggcttccc attgctgtcc    960
   tatttcctgt aaactggcag agggccttca gccttgctcc caccccatcc ttagtctccc   1020
   atgccttttg ccccacccca ggacatgcca gtcctattct ttttagtgta tttcccttct    1080
   caactccgga tccactctgt cttccattgc tctttctatg tctactgttt gtacatccat   1140
   tcatatgtaa attcaccatc aagtattatg ggatgcaggc cctgtactta gagctgtggg   1200
   gaacatccac ctagtcttgg tcattgttcc ttaacccaag gaacttaaaa atttgtcatg   1260
   ctcatgtgag gaacacactg gcataacaag gcagtatcta attaggagct aagacactgt   1320
   gtagcaatag tcatacctgg cacagatatt ccactttatg gctttcttat ctgttaggtt   1380
   atttgatctt tccatgatta tatgaggtag ctagggcaga agttatccct attccgcaca   1440
   ggaggaaaca aaagcttggc tgacataact aattacttgt ttagactgaa tgttgagcat   1500
   gaaatgattg gttaaggaga agagtggaag tgggaaagac gggtgggcgg gactcgctta   1560
   gtgacccatc ctgaatgaga gtgggtaagg aataagatta gtaggcaggc tgaggcagat   1620
   gtcctagccg tgagaataca gacatctact cctatgtgac cgacagccct gatcattttc   1680
   ccgttggcta cagagcaagg caagaacagg gaaacactga gacttgttag aaagctttac   1740
   tattgtgagt attctgactt tgttttgttg agaaaggttc tcactttgac gccccggttg   1800
   tctcggaact cattacatag cccaggctag cctcaacccc aagtgttggt ttgacagata   1860
   tgagccatca tgaccaagtt aatttactgg gtattttgga agaacaaaat ctggaaactg   1920
   aaaattaaat gctaaagtgg ggatgaagct taagaggaaa aggtcccgaa tatgtgcaca   1980
   tcagttatct tcagctaagg tggcttcctt tctacctgac atattttcca atttttcacc   2040
   ttccataaat gaaatcgttt ttaaaagagg gagtcttttt ggcagcctct gcttggatgt   2100
   gtggaggagg gtgtagctag cccgggaagg caactgacct taacacagac aaaagctcct   2160
   gaggtttgag ctaaagcggt ggtagtggag ttagaagaat cgcttctctc tcagaataga   2220
   gaaataaggg cagcctacac tggtacctgg gcagctggga agataaggga ccttgaactg   2280
   gagcagtcag aggaagatgg cttgggagat actagtgatg ggtcggccct agggtgttgt   2340
   cttagacagt ctcaccatat agcccatgct ggcctcaaac ttgtaatgcc tctgcctcag   2400
   ctttcagaat gctaagattt taggaatgtt catggaatcc agcatccttg ggacactgag 2460
   ttggagatca cacagaggtg acaaaggaaa ggacggtgag tgtagcttac aagaaacaag 2520
   ccactcacct tacactcaca cagacacttg ctcctcaggt acttgaagtg gtggtaccga 2580
   aagacccacg tggaaaagaa gacccctttc atcgacatgc tcaattctct acccctgaga 2640
   cagatctatg gtgagtctca gctcagctgc ccttttcaaa gagggtccaa cttctgtgct 2700
   tcaggcaaaa aggctcctag tttatgcatg gctccattcc ctaagttttt aaagatgtgg 2760
   gcttgctttc ttcttccagt tctacaccag ccatcctgac tttctttcta ggttgtcccc 2820
   tgggtggcat tggaggaggc actatcaccc ggggctggag aggccagttc tgtcgttggc 2880
   agctcaaccc tggaatgtac cagcaccaga cagtcattgc agaccaagta aggaaggggg 2940
   ttgggcaggc agaggagtga ctcttggcta gcataggatg tggcagttag gcatccacat 3000
   ggtccagttc agtaaatgct ttcagaatgc gtgtgccccg ctcagtacta ggatggacag 3060
   ttggttaaag atgaacacag aacccatgtt ccttgaaaga ataaagaaac agcctgataa 3120
   atctcatgta ctaatcttct gagcactagc tcagtcagta cccaccaaag actagaaatt 3180
   tctggatcat ttaaactggc taaaagaaca tttcatcctg catggagtgg tacatcctta 3240
   taatcctaat ttttgggagg cagatgcaaa agaatcagga gttcaaggct agcctgggct 3300
   acttaagaca aaacaaaatg gtgggcagta agatggctca acaggtaaag ccacacaggt 3360
   ttgacaacct gcattccatc cctggacctg cagggcgaga ggaagagaac caggccctac 3420
   aaactgtctt tgaagcttca tgcataactt tactgttcac ggtggcttct gcatgctgtg 3480
   ggatcggtgt aaagatcaga agacagcctg taggaaggag tgggctctca ccttctacca 3540
   tgtgggcttg ggagattaaa ctcaggttgt gaggcttggt gacaagtgcc ttcactggct 3600
   tcagccatct tggctggccc tcaatttttt ttttttaaag aatattcgaa aagactgaaa 3660
   gagaaaaagt gaaacctaaa aagccatggt gggaaaaggc gcacagtgct aagtctcctt 3720
   ccctagatta gtaacgcttt agcctaggtt agcgcccttg ccgcagacct ctgatggtct 3780
   tcccaggacc ccctttaact cttctctcta gccttcctcg ctcatatctg ggccttaatt 3840
   gtcagtgtat gccctgccct tcaccaaggc tctcaaatga cctccccttc tgagttagca 3900
   agtccccctt tcccgagctt ccagcttacc tccaaaggtg agataactgg cagatctcaa 3960
   actcaccaat gacagtatcc ttctcaattc aagtttactg gctgtgctca atatccagtc 4020
   tctaccctag gcccatgtct catctcttcc gcattgcaag ccaagtttag aaacatgggt 4080
   ctgaagtaac tattgcctgt gtactgtgtt aatacactgt ggaaggcttt ggggatattt 4140
   atatttttaa aaatcctaca aagtatcatt atcatcattt tacagatgag gagacaggat 4200
   ccagggggca ttagtaacct gctcaaagca gcatggtagc cagtgctctc tatgacaatt 4260
   gttggtggtc ctaaccattg gtactttacc agcaacatgg aaaattactt tatggctacg 4320
   cccacatctg taatctccag gactgaggca gaaggatcgc cagttcaagg ccagcctgag 4380
   ctacaaagca agatcctatc tcaaaagaaa tagaagggaa aggaaacttt atatcaataa 4440
   caacacggag cgtagtttgg agtaacggag gagattaagc cctcagcatt tctcctcccc 4500
   ggagcctaag gttatactgg agaagtaaga cttggggaca atggagactg ccaccatgag 4560
   gtgggcccac agtagactga agcagcagac catactacag agactttgca cacatgccca 4620
   ctctatgagg tccacttcag taatctagaa atgacccctg ccctcagagt gagcagtggg 4680
   aaaattggag acacaggaga gcaaatgaaa acactacaac aataatttac caaatcctga 4740
   agaggaatag gatgtaacag aaaggtccct gtgggagagg aggaggaaga ggaagtacct 4800
   gacctaggcc ctgaaggatg aagggatctg gctggatgga gatgggcaga ctcgtgacga 4860
   ggaagtgagg ggtgctcaca gactggagag agtcttgtat ttgtgtgacc aggctagagt 4920
   ttagcattca gcctgaatgc tataggagtc actaaaacac gctaggcagg ggttggcaca 4980
   tactgatttg ttacatcatt ttctgctacc aagctaagag gcagagatag cataagcctg 5040
   attaattaag gcactgagat caggcatgaa gggaggaggt ctaggagctg gctgcacgag 5100
   gaggaagtag gtcaggtgct ggctcactga gctggaaggc aggcctggag acaacctaag 5160
   gctggtggtg gtgacagtga cagggctgca aaggagtact ttgcggccgg ggaccaagtg 5220
   tgtgttgcct gtgggttact tattccagcc tgtctgctct cttgatcaag tttatagtat 5280
   gcttgcgtcg agatgggcgg actgtgtacc agcaagttct gtccttggag cttccaaatg 5340
   tcctgcgcag ctggaactgg ggcctgtgtg gttactttgc tttctaccac gccctctatc 5400
   cccgagcctg gacggtctat cagcttcctg gccagaatgt caccctcacc tgtcgccagg 5460
   tcacacctat cttgcctcat gactaccagg tgaggattcc tcctgtttgc ttcattccat 5520
   gcgaattcca cctaaattgt aagcgttaat attttgttaa aattcgcgtt aaatttttgt 5580
   taaatcagct cattttttaa ccaataggcc gaaatcggca aaatccctta taaatcaaaa 5640
   gaatagaccg agatagggtt gagtgttgtt ccagtttgga acaagagtcc actattaaag 5700
   aacgtggact ccaacgtcaa agggcgaaaa accgtctatc agggcgatgg cccactacgt 5760
   gaaccatcac cctaatcaag ttttttgggg tcgaggtgcc gtaaagcact aaatcggaac 5820
   cctaaaggga gcccccgatt tagagcttga cggggaaagc cggcgaacgt ggcgagaaag 5880
   gaagggaaga aagcgaaagg agcgggcgct agggcgctgg caagtgtagc ggtcacgctg 5940
   cgcgtaacca ccacacccgc cgcgcttaat gcgccgctac agggcgcgtc ccattcgcca 6000
   ttcaggctgc gcaactgttg ggaagggcga tcggtgcggg cctcttcgct attacgccag 6060
   ctggcgaaag ggggatgtgc tgcaaggcga ttaagttggg taacgccagg gttttcccag 6120
   tcacgacgtt gtaaaacgac ggccagtgaa ttgtaatacg actcactata gggcgaattg 6180
   gagctcgaga tctagatatc gatgaattca taacttcgta taatgtatgc tatacgaagt 6240
   tatggatctg tcgatcgacg gatcgatccg aacaaacgac ccaacacccg tgcgttttat 6300
   tctgtctttt tattgccgat cccctcagaa gaactcgtca agaaggcgat agaaggcgat 6360
   gcgctgcgaa tcgggagcgg cgataccgta aagcacgagg aagcggtcag cccattcgcc 6420
   gccaagctct tcagcaatat cacgggtagc caacgctatg tcctgatagc ggtccgccac 6480
   acccagccgg ccacagtcga tgaatccaga aaagcggcca ttttccacca tgatattcgg 6540
   caagcaggca tcgccatggg tcacgacgag atcctcgccg tcgggcatgc gcgccttgag 6600
   cctggcgaac agttcggctg gcgcgagccc ctgatgctct tcgtccagat catcctgatc 6660
   gacaagaccg gcttccatcc gagtacgtgc tcgctcgatg cgatgtttcg cttggtggtc 6720
   gaatgggcag gtagccggat caagcgtatg cagccgccgc attgcatcag ccatgatgga 6780
   tactttctcg gcaggagcaa ggtgagatga caggagatcc tgccccggca cttcgcccaa 6840
   tagcagccag tcccttcccg cttcagtgac aacgtcgagc acagctgcgc aaggaacgcc 6900
   cgtcgtggcc agccacgata gccgcgctgc ctcgtcctgc agttcattca gggcaccgga 6960
   caggtcggtc ttgacaaaaa gaaccgggcg cccctgcgct gacagccgga acacggcggc 7020
   atcagagcag ccgattgtct gttgtgccca gtcatagccg aatagcctct ccacccaagc 7080
   ggccggagaa cctgcgtgca atccatcttg ttcaatggcc gatcccatat tggctgcacg 7140
   gatcctgaac ggcagaggtt acggcagttt gtctctcccc cttccgggag ccaccttctt 7200
   ctccaaccgt cccggtcgcg ctctcggcgc ttctgaggag agaactggct gagtgacgcc 7260
   ctttatagat tcgcccttgt gtcccgcccc ttcctttccc gccctccctt gcgctacggg 7320
   gccgcccgca ccggcctaca cggagcgcgc gcggcggagt tgttgacgct agggctccgg 7380
   ctccctggtt gggtgttctt tctgacgcga caggaggagg agaatgtcct ggtcctgtcg 7440
   tcctcctttc gggtttcccg tgcactcaaa ccgaggactt acagaacgga ggataaagtt 7500
   aggccatttt tactcagctt cggagttcag gctcattttt cagctaaagt ctctcattag 7560
   tatcccccca cacacatcgg gaaaatggtt tgtcctacgc atcggtaatg aaggcggggc 7620
   ccttcgggtc ctccggagcg ggttccgggg gtggggggaa ggagggaggg acgggacggg 7680
   cctcgttcat gaatattcag ttcaccgctg aatatgcata aggcaggcaa gatggcgcgt 7740
   ccaatcaatt ggaagtagcc gttattagtg gagaggcccc aggacgttgg ggcaccgcct 7800
   gtgctctagt agctttacgg agccctggcg ctcgatgttc aagcccaagc tttcgcgagc 7860
   tcgaccgaac aaacgaccca acacccgtgc gttttattct gtctttttat tgccgctcag 7920
   ctttacagtg acaatgacgg ctggcgactg aatattagtg cttacagaca gcactacata 7980
   ttttccgtcg atgttgaaat cctttctcat atgtcaccat aaatatcaaa taattatagc 8040
   aatcatttac gcgttaatgg ctaatcgcca tcttccagca ggcgcaccat tgcccctgtt 8100
   tcactatcca ggttacggat atagttcatg acaatattta cattggtcca gccaccagct 8160
   tgcatgatct ccggtattga aactccagcg cgggccatat ctcgcgcggc tccgacacgg 8220
   gcactgtgtc cagaccaggc caggtatctc tgaccagagt catcctaaaa tacacaaaca 8280
   attagaatca gtagtttaac acattataca cttaaaaatt ttatatttac cttagcgccg 8340
   taaatcaatc gatgagttgc ttcaaaaatc ccttccaggg cgcgagttga tagctggctg 8400
   gtggcagatg gcgcggcaac accatttttt ctgacccggc aaaacaggta gttattcgga 8460
   tcatcagcta caccagagac ggaaatccat cgctcgacca gtttagttac ccccaggcta 8520
   agtgccttct ctacacctgc ggtgctaacc agcgttttcg ttctgccaat atggattaac 8580
   attctcccac cgtcagtacg tgagatatct ttaaccctga tcctggcaat ttcggctata 8640
   cgtaacaggg tgttataagc aatccccaga aatgccagat tacgtatatc ctggcagcga 8700
   tcgctatttt ccatgagtga acgaacctgg tcgaaatcag tgcgttcgaa cgctagagcc 8760
   tgttttgcac gttcaccggc atcaacgttt tcttttcgga tccgccgcat aaccagtgaa 8820
   acagcattgc tgtcacttgg tcgtggcagc ccggaccgac gatgaagcat gtttagctgg 8880
   cccaaatgtt gctggatagt ttttactgcc agaccgcgcg cctgaagata tagaagataa 8940
   tcgcgaacat cttcaggttc tgcgggaaac catttccggt tattcaactt gcaccatgcc 9000
   gcccacgacc ggcaaacgga cagaagcatt ttccaggtat gctcagaaaa cgcctggcga 9060
   tccctgaaca tgtccatcag gttcttgcga acctcatcac tcgttgcatc gaccggtaat 9120
   gcaggcaaat tttggtgtac ggtcagtaaa ttggacacct tcctcttctt cttgggcatg 9180
   gccgcaggaa agcagagccc tgaagctccc atcaccggcc aataagagcc aagcctgcag 9240
   tgtgacctca tagagcaatg tgccagccag cctgacccca agggccctca ggcttgggca 9300
   cactgtctct aggaccctga gagaaagaca tacccatttc tgcttagggc cctgaggatg 9360
   agcccagggg tggcttggca ctgaagcaaa ggacactggg gctcagctgg cagcaaagtg 9420
   accaggatgc tgaggctttg acccagaagc cagaggccag aggccaggac ttctcttggt 9480
   cccagtccac cctcactcag agctttacca atgccctctg gatagttgtc gggtaacggt 9540
   ggacgccact gattctctgg ccagcctagg acttcgccat tccgctgatt ctgctcttcc 9600
   agccactggc tgaccggttg gaagtactcc agcagtgcct tggcatccag ggcatctgag 9660
   cctaccaggt ccttcagtac ctcctgccag ggcctggagc agccagcctg caacacctgc 9720
   ctgccaagca gagtgaccac tgtgggcaca ggggacacag ggtggggccc acaacagcac 9780
   cattgtccac ttgtccctca ctagtaaaag aactctaggg ttgcgggggg tgggggaggt 9840
   ctctgtgagg ctggtaaggg atatttgcct ggcccatgga gatccataac ttcgtataat 9900
   gtatgctata cgaagttata agctttcgcg agctcgagat ctagatatcg ataccgtcga 9960
   cctcgagggg gggcccggta cccagctttt gttcccttta gtgagggtta atttcgagct 10020
   tggcgtaatc atggtcatag ctgtttcctg tgtgaaattg ttatccgctc acaattccac 10080
   acaacatacg agccggaagc ataaagtgta aagcctgggg tgcctaatga gtgagctaac 10140
   tcacattaat tgcgttgcgc tcactgcccg ctttccagtc gggaaacctg tcgtgccagc 10200
   tgcattaatg aatcggccaa cgcgcgggga gaggcggttt gcgtattggg cgctcttccg 10260
   cttcctcgct cactgactcg ctgcgctcgg tcgttcggct gcggcgagcg gtatcagctc 10320
   actcaaaggc ggtaatacgg ttatccacag aatcagggga taacgcagga aagaacatgt 10380
   gagcaaaagg ccagcaaaag gccaggaacc gtaaaaaggc cgcgttgctg gcgtttttcc 10440
   ataggctccg cccccctgac gagcatcaca aaaatcgacg ctcaagtcag aggtggcgaa 10500
   acccgacagg actataaaga taccaggcgt ttccccctgg aagctccctc gtgcgctctc 10560
   ctgttccgac cctgccgctt accggatacc tgtccgcctt tctcccttcg ggaagcgtgg 10620
   cgctttctca tagctcacgc tgtaggtatc tcagttcggt gtaggtcgtt cgctccaagc 10680
   tgggctgtgt gcacgaaccc cccgttcagc ccgaccgctg cgccttatcc ggtaactatc 10740
   gtcttgagtc caacccggta agacacgact tatcgccact ggcagcagcc actggtaaca 10800
   ggattagcag agcgaggtat gtaggcggtg ctacagagtt cttgaagtgg tggcctaact 10860
   acggctacac tagaaggaca gtatttggta tctgcgctct gctgaagcca gttaccttcg 10920
   gaaaaagagt tggtagctct tgatccggca aacaaaccac cgctggtagc ggtggttttt 10980
   ttgtttgcaa gcagcagatt acgcgcagaa aaaaaggatc tcaagaagat cctttgatct 11040
   tttctacggg gtctgacgct cagtggaacg aaaactcacg ttaagggatt ttggtcatga 11100
   gattatcaaa aaggatcttc acctagatcc ttttaaatta aaaatgaagt tttaaatcaa 11160
   tctaaagtat atatgagtaa acttggtctg acagttacca atgcttaatc agtgaggcac 11220
   ctatctcagc gatctgtcta tttcgttcat ccatagttgc ctgactcccc gtcgtgtaga 11280
   taactacgat acgggagggc ttaccatctg gccccagtgc tgcaatgata ccgcgagacc 11340
   cacgctcacc ggctccagat ttatcagcaa taaaccagcc agccggaagg gccgagcgca 11400
   gaagtggtcc tgcaacttta tccgcctcca tccagtctat taattgttgc cgggaagcta 11460
   gagtaagtag ttcgccagtt aatagtttgc gcaacgttgt tgccattgct acaggcatcg 11520
   tggtgtcacg ctcgtcgttt ggtatggctt cattcagctc cggttcccaa cgatcaaggc 11580
   gagttacatg atcccccatg ttgtgcaaaa aagcggttag ctccttcggt cctccgatcg 11640
   ttgtcagaag taagttggcc gcagtgttat cactcatggt tatggcagca ctgcataatt 11700
   ctcttactgt catgccatcc gtaagatgct tttctgtgac tggtgagtac tcaaccaagt 11760
   cattctgaga atagtgtatg cggcgaccga gttgctcttg cccggcgtca atacgggata 11820
   ataccgcgcc acatagcaga actttaaaag tgctcatcat tggaaaacgt tcttcggggc 11880
   gaaaactctc aaggatctta ccgctgttga gatccagttc gatgtaaccc actcgtgcac 11940
   ccaactgatc ttcagcatct tttactttca ccagcgtttc tgggtgagca aaaacaggaa 12000
   ggcaaaatgc cgcaaaaaag ggaataaggg cgacacggaa atgttgaata ctcatactct 12060
   tcctttttca atattattga agcatttatc agggttattg tctcatgagc ggatacatat 12120
   ttgaatgtat ttagaaaaat aaacaaatag gggttccgcg cacatttccc cgaaaagtgc 12180
   ctcgagcatc tggctcctta gttctctggg gtcacacaca cagcactgga tcccacccca 12240
   agcctgccca ttccctgctt tccctcacgt acttgtgtct ccgctctccc agctctggca 12300
   actttgaagg aggacctgac ccggcgacgg tacctgatga gtggagtggt ggcacctgtg 12360
   aaaaggagga acgtcatccc tcatgacatt ggggatccgg gtgagattcc gtctgtcagt 12420
   ctctatacct gctatcctgt ccactgacac actgaaccag gtcccctccc ctccaacaga 12480
   tgatgagcca tggctccggg tcaacgcata tttgattcat gatactgctg actggaagga 12540
   cctgaacctg aagtttgtat tgcaaattta tcgggactat tacctgacgg gtgatcaagg 12600
   cttcctggag gacatgtggc ctgtgtgtct ggtaaggaac gagaactgtg tccagtatac 12660
   cagggatgta gctggagtct tgggagaacc cagactttct ttcttagaac ctaggtcttc 12720
   agtgtcttga tccctctcta ggctgtgatg gagtccgaaa tgaagtttga caaggaccaa 12780
   gatggactca ttgagaatgg aggctacgca gaccagacct atgatgcatg ggtcaccaca 12840
   ggccccaggt ttgggggtag ggatttccag gtctgaggtg aagacccggg catgtgagga 12900
   tgccgtgtag agcggccggt ctcactccag ccactgttcc cacccagtgc ttactgcgga 12960
   gggctgtggc tggcggcagt ggctgtaatg gttcagatgg ctgttctgtg tggggcccaa 13020
   gatgtccagg agaggtttgc ttccattctc tgccgaggcc gagaagctta tgagagactg 13080
   ctgtggaacg gtgagtaaaa gcaacctggg gcagcttaaa acccaactac gggatacaag 13140
   caacttgact ttgctctttc tctattccag gacgctatta caactacgac agcagctccc 13200
   atcctcagtc tcggagcatc atgtctgacc agtgtgctgg gcagtggttc ctgagggcct 13260
   gcggcctggg agaaggagac actgaagtga gaggctggag aagccagaag tctgggtttc 13320
   cctggaggtg ggaggcaagt ctgagactcc acctttgctc tccctccagg tatttcctac 13380
   cctgcatgtg gtccgtgctc tccaaaccat ctttgaactc aacgtccaag cctttgcagg 13440
   aggagccatg ggggccgtga acgggatgca ccctcatggt gtccctgata gatccagtgt 13500
   gcagtctgat gaagtctggg tgggtgtggt ctatgggctg gcagccacca tgatccaaga 13560
   ggtaatgaat tctcttccca gcccttcacc atttgtactg aggtccagaa aacttcctca 13620
   gccactaact ttattccaag aacaccctac tgcctgccat cttatccgtc cacagagcgc 13680
   ccttctccct aataagtgtt ccacctcttg cctttaagtt cttgccttta gggcgaacgg 13740
   cctcctgtct tctctgctca gctaaggtaa ttgtgccaag agtcagttat tatgtgtgac 13800
   ttctacgtct ccccacaggg cctgacttgg gaaggtttcc ggacagctga aggctgttac 13860
   cgcactgtat gggaacgcct gggcctggct ttccagaccc cagaggcata ctgccagcaa 13920
   caagtgttcc gctccctggc ctacatgcgg ccactgagca tctgggccat gcagctggcc 13980
   ctgcaacagc agcagcataa aaagagccgc aggccatcag tcacacaagg cacgggacta 14040
   agcacacagc ctgaatgtgg accaaagaga tcgctggcaa acctcaattc agagtgatcc 14100
   ctctgaactg tggaggccca gcctccacac tggacctcct ccttcctccc acaagtcctg 14160
   cagccctgag ccaataggac aatcgcgtcg accagctgct tcatccccgt ggcccgttgc 14220
   tcgcgtttgc tggcggtgtc cccggaagaa atatatttgc atgtctttag ttctatgatg 14280
   acacaaaccc cgcccagcgt cttgtcattg gcgaattcga acacgcagat gcagtcgggg 14340
   cggcgcggtc cgaggtccac ttcgcatatt aaggtgacgc gtgtggcctc gaacaccgag 14400
   cgaccctgca gcgacccgct taacagcgtc aacagcgtgc cgcagatctt ggtggcgtga 14460
   aactcccgca cctcttcggc cagcgccttg tagaagcgcg tatggcttcg taccccggcc 14520
   atcaacacgc gtctgcgttc gaccaggctg cgcgttctcg cggccatagc aaccgacgta 14580
   cggcgttgcg ccctcgccgg cagcaagaag ccacggaagt ccgcccggag cagaaaatgc 14640
   ccacgctact gcgggtttat atagacggtc cccacgggat ggggaaaacc accaccacgc 14700
   aactgctggt ggccctgggt tcgcgcgacg atatcgtcta cgtacccgag ccgatgactt 14760
   actggcgggt gctgggggct tccgagacaa tcgcgaacat ctacaccaca caacaccgcc 14820
   tcgaccaggg tgagatatcg gccggggacg cggcggtggt aatgacaagc gcccagataa 14880
   caatgggcat gccttatgcc gtgaccgacg ccgttctggc tcctcatatc gggggggagg 14940
   ctgggagctc acatgccccg cccccggccc tcaccctcat cttcgaccgc catcccatcg 15000
   ccgccctcct gtgctacccg gccgcgcggt accttatggg cagcatgacc ccccaggccg 15060
   tgctggcgtt cgtggccctc atcccgccga ccttgcccgg caccaacatc gtgcttgggg 15120
   cccttccgga ggacagacac atcgaccgcc tggccaaacg ccagcgcccc ggcgagcggc 15180
   tggacctggc tatgctggct gcgattcgcc gcgtttacgg gctacttgcc aatacggtgc 15240
   ggtatctgca gtgcggcggg tcgtggcggg aggactgggg acagctttcg gggacggccg 15300
   tgccgcccca gggtgccgag ccccagagca acgcgggccc acgaccccat atcggggaca 15360
   cgttatttac cctgtttcgg gcccccgagt tgctggcccc caacggcgac ctgtataacg 15420
   tgtttgcctg ggccttggac gtcttggcca aacgcctccg ttccatgcac gtctttatcc 15480
   tggattacga ccaatcgccc gccggctgcc gggacgccct gctgcaactt acctccggga 15540
   tggtccagac ccacgtcacc acccccggct ccataccgac gatatgcgac ctggcgcgca 15600
   cgtttgcccg ggagatgggg gaggctaact gaaacacgga aggagacaat accggaagga 15660
   acccgcgcta tgacggcaat aaaaagacag aataaaacgc acgggtgttg ggtcgtttgt 15720
   tcataaacgc ggggttcggt cccagggctg gcactctgtc gataccccac cgagacccca 15780
   ttggggccaa tacgcccgcg tttcttcctt ttccccaccc caccccccaa gttcgggtga 15840
   aggcccaggg ctcgcagcca acgtcggggc ggcaggccct gccatagcca ctggccccgt 15900
   gggttaggga cggggtcccc catggggaat ggtttatggt tcgtgggggt tattattttg 15960
   ggcgttgcgt ggggtctggt ggacgaccca gcagctgctt catccccgtg gcccgttgct 16020
   cgcgtttgct ggcggtgtcc ccggaagaaa tatatttgca tgtctttagt tctatgatga 16080
   cacaaacccc gcccagcgtc ttgtcattgg cgaattcgaa cacgcagatg cagtcggggc 16140
   ggcgcggtcc gaggtccact tcgcatatta aggtgacgcg tgtggcctcg aacaccgagc 16200
   gaccctgcag cgacccgctt aacagcgtca acagcgtgcc gcagatcttg gtggcgtgaa 16260
   actcccgcac ctcttcggcc agcgccttgt agaagcgcgt atggcttcgt accccggcca 16320
   tcaacacgcg tctgcgttcg accaggctgc gcgttctcgc ggccatagca accgacgtac 16380
   ggcgttgcgc cctcgccggc agcaagaagc cacggaagtc cgcccggagc agaaaatgcc 16440
   cacgctactg cgggtttata tagacggtcc ccacgggatg gggaaaacca ccaccacgca 16500
   actgctggtg gccctgggtt cgcgcgacga tatcgtctac gtacccgagc cgatgactta 16560
   ctggcgggtg ctgggggctt ccgagacaat cgcgaacatc tacaccacac aacaccgcct 16620
   cgaccagggt gagatatcgg ccggggacgc ggcggtggta atgacaagcg cccagataac 16680
   aatgggcatg ccttatgccg tgaccgacgc cgttctggct cctcatatcg ggggggaggc 16740
   tgggagctca catgccccgc ccccggccct caccctcatc ttcgaccgcc atcccatcgc 16800
   cgccctcctg tgctacccgg ccgcgcggta ccttatgggc agcatgaccc cccaggccgt 16860
   gctggcgttc gtggccctca tcccgccgac cttgcccggc accaacatcg tgcttggggc 16920
   ccttccggag gacagacaca tcgaccgcct ggccaaacgc cagcgccccg gcgagcggct 16980
   ggacctggct atgctggctg cgattcgccg cgtttacggg ctacttgcca atacggtgcg 17040
   gtatctgcag tgcggcgggt cgtggcggga ggactgggga cagctttcgg ggacggccgt 17100
   gccgccccag ggtgccgagc cccagagcaa cgcgggccca cgaccccata tcggggacac 17160
   gttatttacc ctgtttcggg cccccgagtt gctggccccc aacggcgacc tgtataacgt 17220
   gtttgcctgg gccttggacg tcttggccaa acgcctccgt tccatgcacg tctttatcct 17280
   ggattacgac caatcgcccg ccggctgccg ggacgccctg ctgcaactta cctccgggat 17340
   ggtccagacc cacgtcacca cccccggctc cataccgacg atatgcgacc tggcgcgcac 17400
   gtttgcccgg gagatggggg aggctaactg aaacacggaa ggagacaata ccggaaggaa 17460
   cccgcgctat gacggcaata aaaagacaga ataaaacgca cgggtgttgg gtcgtttgtt 17520
   cataaacgcg gggttcggtc ccagggctgg cactctgtcg ataccccacc gagaccccat  17580
   tggggccaat acgcccgcgt ttcttccttt tccccacccc accccccaag ttcgggtgaa  17640
   ggcccagggc tcgcagccaa cgtcggggcg gcaggccctg ccatagccac tggccccgtg  17700
   ggttagggac ggggtccccc atggggaatg gtttatggtt cgtgggggtt attattttgg  17760
   gcgttgcgtg gggtctggtg gacgacccag                                   17790